# EUROPEAN PATENT SPECIFICATION

(11) **EP 2 094 841 B1**
(45) Date of publication and mention of the grant of the patent: **14.03.2012**
(21) Application number: 07848031.6
(22) Date of filing: 11.12.2007
(51) Int. Cl.: C12N 9/02, C12P 7/62, C12P 17/06

(54) **PROCESS FOR PREPARING PRAVASTATIN**
VERFAHREN ZUR HERSTELLUNG VON PRAVASTATIN
PROCÉDÉ DE PRÉPARATION DE LA PRAVASTATINE

(30) Priority: 13.12.2006 EP 06126046
(43) Date of publication of application: 02.09.2009
(73) Proprietor: DSM Sinochem Pharmaceuticals Netherlands B.V., 2613 AX Delft (NL)
(72) Inventor: KLAASSEN, Paul, 3315 TN Dordrecht (NL); VOLLEBREGT, Adrianus Wilhelmus Hermanus, 2673 BB Naaldwijk (NL); VAN DEN BERG, Marco Alexander, 2685 EH Poeldijk (NL); HANS, Marcus, 2582 BN Den Haag (NL); VAN DER LAAN, Jan Metske, 4839 AP Breda (NL)
(74) Representative: De Vroom, Erik
(86) International application number: PCT/EP2007/063638
(87) International publication number: WO 2008/071673

(56) References cited:
- WO-A-96/40863
- CN-A- 1 584 016
- US-A1- 5 372 816
- DATABASE Geneseq [Online] 30 September 2003 (2003-09-30), "Bacterial P450 enzyme SEQ ID NO:38." XP002425141 retrieved from EBI accession no. GSP:ABR82137 Database accession no. ABR82137 -& DATABASE Geneseq [Online] 30 September 2003 (2003-09-30), "Bacterial P450 enzyme encoding DNA SEQ ID NO:37." XP002425142 retrieved from EBI accession no. GSN:ACF06127 Database accession no. ACF06127 -& WO 03/052050 A (DIVERSA CORP [US]; WEINER DAVID [US]; BURKE MARK [US]; HITCHMAN TIM [U) 26 June 2003 (2003-06-26)
- DATABASE UniProt [Online] 26 April 2005 (2005-04-26), "Cytochrome P450." XP002425146 retrieved from EBI accession no. UNIPROT:Q59HJ0 Database accession no. Q59HJ0
- DATABASE UniProt [Online] 26 April 2005 (2005-04-26), "Cytochrome P450 monooxygenase CYP105S2." XP002425145 retrieved from EBI accession no. UNIPROT:Q595S0 Database accession no. Q595S0 cited in the application -& JUNGMANN VOLKER ET AL: "Biocatalytic conversion of avermectin to 4 ''-oxo-avermectin: Characterization of biocatalytically active bacterial strains and of cytochrome P450 monooxygenase enzymes and their genes" APPLIED AND ENVIRONMENTAL MICROBIOLOGY, vol. 71, no. 11, November 2005 (2005-11), pages 6968-6976, XP002425267 ISSN: 0099-2240 -& MOLNAR ISTVAN ET AL: "Biocatalytic conversion of avermectin to 4 ''-oxo-avermectin: Heterologous expression of the ema1 cytochrome P450 monooxygenase" APPLIED AND ENVIRONMENTAL MICROBIOLOGY, vol. 71, no. 11, November 2005 (2005-11), pages 6977-6985, XP002425268 ISSN: 0099-2240
- DATABASE UniProt [Online] 1 October 2002 (2002-10-01), "Cytochrome P450 RhF." XP002425147 retrieved from EBI accession no. UNIPROT:Q8KU27 Database accession no. Q8KU27
- PENG Y DEMAIN A L: "Bioconversion of compactin to pravastatin by actinomadura sp. ATCC 55678" JOURNAL OF MOLECULAR CATALYSIS. B, ENZYMATIC, ELSEVIER, AMSTERDAM,, NL, vol. 10, 2000, pages 151-156, XP002956560 ISSN: 1381-1177
- CHEN CHAO-HSIEN ET AL: "Screening of compactin-resistant microorganisms capable of converting compactin to pravastatin" CURRENT MICROBIOLOGY, vol. 53, no. 2, August 2006 (2006-08), pages 108-112, XP002425251 ISSN: 0343-8651
- DATABASE JPO Proteins [Online] 17 November 2006 (2006-11-17), "METHOD FOR ISOLATION OF P450 GENES." XP002425148 retrieved from EBI accession no. JPOP:BD906462 Database accession no. BD906462 -& DATABASE EMBL [Online] 18 November 2006 (2006-11-18), "METHOD FOR ISOLATION OF P450 GENES." XP002425149 retrieved from EBI accession no. EMBL:DD355772 Database accession no. DD355772 & WO 2006/051729 A (MARINE BIOTECH INST CO LTD [JP]; KUBOTA MITSUTOSHI [JP]; NODATE MIHO [) 18 May 2006 (2006-05-18)
- DATABASE UniProt [Online] 21 August 2007 (2007-08-21), "Cytochrome P450 monooxygenase 105L." XP002472886 retrieved from EBI accession no. UNIPROT:A6YRR4 Database accession no. A6YRR4
- DATABASE UniProt [Online] 1 November 1998 (1998-11-01), "Cytochrome P450 hydroxylase (Fragment)." XP002472887 retrieved from EBI accession no. UNIPROT:O85653 Database accession no. O85653
- DATABASE Geneseq [Online] 24 November 1994 (1994-11-24), "Protein containing Cytochrome P450 SCA-2 activity." XP002472888 retrieved from EBI accession no. GSP:AAR51368 Database accession no. AAR51368
- DATABASE UniProt [Online] 21 March 2006 (2006-03-21), "Response regulator/GGDEF domain protein." XP002472889 retrieved from EBI accession no. UNIPROT:Q2GKF8 Database accession no. Q2GKF8

### SEQUENCE LISTING

<110> DSM IP Assets B.V.
<120> PROCESS FOR PREPARING PRAVASTATIN
<130> 25590WO
<150> EP06126046.9
   <151> 2006-12-13
<160> 59
<170> PatentIn version 3.2
<210> 1
   <211> 2545
   <212> DNA
   <213> Amycolatopsis orientalis
<400> 1
   gatctctacc tcgcgctggc gaacgacacg gactgactag ccccgcggcg ggttgaagat 60
   catcgattcc gggttgatct gcttggcctt ctccatcagg ccgtactgcg tcatcaggtc 120
   gatcacccgc tgcatgcgca ccgggctcat cgcggtcggc cacgtgccga ggcgcatcag 180
   gctgacggtg tccttgtcca ctttggcgta gctggtcacg gtctgctcga ccaggctgcg 240
   gttggccgcg tcccgctgtc ccttgacgat cgcccgctgg aacgccgccg tggtcttcgg 300
   gttctcctgt gcgtacttgg cgctggtcgc ccacaccgcg atcggcacgt ccaaagtggc 360
   cccggtcgcc gcgtccagca ccggcagcat cccggccttg cgctgcgcct gggtgatgta 420
   cggctccacc atgaacgcgg cgtcgacgtt cttgcgctcg atggccgcct gcatgtccgg 480
   gaacgggatc tcggtgaagg tcaccgtctt gatgtccaca ccgttggcct cgagcgcgga 540
   ccgtgcggtc agttcgacga tgttcgcctt ggtgttgatc gcgatcttct tgccggccag 600
   gtcggctggc ttggtgatcg cgttgtcctt gccggtcagg atcaggaaca tgccctgagc 660
   ggcctggtag gcgtccgcga ccagcttgat gtccagcacg ttcttgtact gcgcggtgaa 720
   gaacgagacg tagttgccga atgcgaactg cagttcgccg ttggcaaggc cgggcaccgc 780
   cgccgcgccg cccggcagcg acttcagttc gacatcaagg ccttcctggg tgaagtagcc 840
   tttctgctgt gcgatggcca gcggtacggt gtccacaatg ggcaacgtgc cgacgaccac 900
   tttggtctgc tccaaaccgc ctgtctggtt gggcttttcg gtatccccac ccagtgccga 960
   acagctggcg gcggcgaggg cgaggacgca ggacagggct atgcgccacg gacgggcgag 1020
   tgacatgcgg ggttctcctg gcaggcaaga cacgatgatc tgggccggat cagaccacat 1080
   cgtccctgtt caacgccagt cgacggaccc tactttcgac tgaaatatgc cagagctcac   1140
   tcgttaagtg gcacgaatgt gctatgcatc ccatgcaacg ggcagcgccc aaaccccgta   1200
   cgccgcggac ttctcgcgca gcttgatctc ctcggccggc accgcgagcc gcagcgacgg   1260
   gaacctcgcg aacagccggg tgaagccgat gcgcatctcc actctggcca gttgctggcc   1320
   gaggcattgg tgtataccac cgccgaacgc ggcgtgcttg cgtgcgtcca ctctgtccag   1380
   ttgaaggata tcgggttcgt cgaacacctt cgggtcccta ttgaccgcgg gcagtccgat   1440
   cgcgacagtg tcgcccttcc tgatcatctg gccttcgagc tccacgtcct ccagcgccgc   1500
   ccggttgggc gttccaaggt ggacgatcga gaggtagcgc agcagttcct ccaccgcgtc   1560
   cgggctgtcc agggcagcga tctgctccgg atgctgaagg agcgcgaaag tgcctaatcc   1620
   caacatgttc gcggtggtct cgtgcccggc gacgagcaaa agcaacgcga tgttcgtcag   1680
   ctcttcatcg gtcagatcgg tgtccgtgat caagctgcca agcaggtcgt ccttggggct   1740
   caaccgcttc gtggcgacca gttcagcgat gtagcgggtg agtttgccaa gcgccgtcgt   1800
   cacctcatcc tgtgtcttgt ccacactggc catgatcgtg gtctgctctt ggaagaacgc   1860
   gtgatcggca tacgagacgc ccagcagctc gcagatcacc agcgaaggca ctggcaacgc   1920
   gaacgcctgc accagatcga ccggcggtcc tgctttggcc atcgcgtcga ggtggtcctc   1980
   ggtgatctgg acgatccgcg gttcgagttc cttgatccgt cgcacggtga actggctgat   2040
   cagcatccgg cggtaacgcg tgtgctcggg tgcgtccatg ttgatgaacc agccgggcgc   2100
   cggggctttc gtggctccgc ctggtcgtgg gatgacgctg aacaccgggt gcttgtgctc   2160
   ggggcggttg ctgaagcgcg gatcgatcat gacagtccgc gctgcggcat ggctggtcac   2220
   cagccagccg atgtggccgt cagggaatcg catcgggctc actggcggaa gtttcaccag   2280
   gtcaggcggt gggtcgaagg ggtagcccac ggctcggccg gtcggtagtg tcactggctc   2340
   gttcatattt tcggagtcta ctctcatttg atggtggact gtcaaagaag agagttctcc   2400
   ggtgtgcagt tatcctgctg cggtggatca accagcgact gggttgcggg aacgcaagaa   2460
   ggccagaacg aagaccgcca tccagcagca cgcgctgcgg ctgttcaagg agcacggcta   2520
   ccaggccacc acggtcgagc agatc                                         2545
<210> 2
   <211> 1206
   <212> DNA
   <213> Amycolatopsis orientalis
<220>
   <221> CDS
   <222> (1) .. (1206)
<400> 2
   atg aga gta gac tcc gaa aat atg aac gag cca gtg aca cta ccg acc      48
   Met Arg Val Asp Ser Glu Asn Met Asn Glu Pro Val Thr Leu Pro Thr
   1               5                   10                  15
   ggc cga gcc gtg ggc tac ccc ttc gac cca ccg cct gac ctg gtg aaa      96
   Gly Arg Ala Val Gly Tyr Pro Phe Asp Pro Pro Pro Asp Leu Val Lys
               20                  25                  30
   ctt ccg cca gtg agc ccg atg cga ttc cct gac ggc cac atc ggc tgg     144
   Leu Pro Pro Val Ser Pro Met Arg Phe Pro Asp Gly His Ile Gly Trp
           35                  40                  45
   ctg gtg acc agc cat gcc gca gcg cgg act gtc atg atc gat ccg cgc     192
   Leu Val Thr Ser His Ala Ala Ala Arg Thr Val Met Ile Asp Pro Arg
       50                  55                  60
   ttc agc aac cgc ccc gag cac aag cac ccg gtg ttc agc gtc atc cca     240
   Phe Ser Asn Arg Pro Glu His Lys His Pro Val Phe Ser Val Ile Pro
   65                  70                  75                  80
   cga cca ggc gga gcc acg aaa gcc ccg gcg ccc ggc tgg ttc atc aac     288
   Arg Pro Gly Gly Ala Thr Lys Ala Pro Ala Pro Gly Trp Phe Ile Asn
                   85                  90                  95
   atg gac gca ccc gag cac acg cgt tac cgc cgg atg ctg atc agc cag     336
   Met Asp Ala Pro Glu His Thr Arg Tyr Arg Arg Met Leu Ile Ser Gln
               100                 105                 110
   ttc acc gtg cga cgg atc aag gaa ctc gaa ccg cgg atc gtc cag atc     384
   Phe Thr Val Arg Arg Ile Lys Glu Leu Glu Pro Arg Ile Val Gln Ile
           115                 120                 125
   acc gag gac cac ctc gac gcg atg gcc aaa gca gga ccg ccg gtc gat     432
   Thr Glu Asp His Leu Asp Ala Met Ala Lys Ala Gly Pro Pro Val Asp
       130                 135                 140
   ctg gtg cag gcg ttc gcg ttg cca gtg cct tcg ctg gtg atc tgc gag     480
   Leu Val Gln Ala Phe Ala Leu Pro Val Pro Ser Leu Val Ile Cys Glu
   145                 150                 155                 160
   ctg ctg ggc gtc tcg tat gcc gat cac gcg ttc ttc caa gag cag acc     528
   Leu Leu Gly Val Ser Tyr Ala Asp His Ala Phe Phe Gln Glu Gln Thr
                   165                 170                 175
   acg atc atg gcc agt gtg gac aag aca cag gat gag gtg acg acg gcg     576
   Thr Ile Met Ala Ser Val Asp Lys Thr Gln Asp Glu Val Thr Thr Ala
               180                 185                 190
   ctt ggc aaa ctc acc cgc tac atc gct gaa ctg gtc gcc acg aag cgg     624
   Leu Gly Lys Leu Thr Arg Tyr Ile Ala Glu Leu Val Ala Thr Lys Arg
           195                 200                 205
   ttg agc ccc aag gac gac ctg ctt ggc agc ttg atc acg gac acc gat      672
   Leu Ser Pro Lys Asp Asp Leu Leu Gly Ser Leu Ile Thr Asp Thr Asp
       210                 215                 220
   ctg acc gat gaa gag ctg acg aac atc gcg ttg ctt ttg ctc gtc gcc      720
   Leu Thr Asp Glu Glu Leu Thr Asn Ile Ala Leu Leu Leu Leu Val Ala
   225                 230                 235                 240
   ggg cac gag acc acc gcg aac atg ttg gga tta ggc act ttc gcg ctc      768
   Gly His Glu Thr Thr Ala Asn Met Leu Gly Leu Gly Thr Phe Ala Leu
                   245                 250                 255
   ctt cag cat ccg gag cag atc gct gcc ctg gac agc ccg gac gcg gtg      816
   Leu Gln His Pro Glu Gln Ile Ala Ala Leu Asp Ser Pro Asp Ala Val
               260                 265                 270
   gag gaa ctg ctg cgc tac ctc tcg atc gtc cac ctt gga acg ccc aac      864
   Glu Glu Leu Leu Arg Tyr Leu Ser Ile Val His Leu Gly Thr Pro Asn
           275                 280                 285
   cgg gcg gcg ctg gag gac gtg gag ctc gaa ggc cag atg atc agg aag      912
   Arg Ala Ala Leu Glu Asp Val Glu Leu Glu Gly Gln Met Ile Arg Lys
       290                 295                 300
   ggc gac act gtc gcg atc gga ctg ccc gcg gtc aat agg gac ccg aag      960
   Gly Asp Thr Val Ala Ile Gly Leu Pro Ala Val Asn Arg Asp Pro Lys
   305                 310                 315                 320
   gtg ttc gac gaa ccc gat atc ctt caa ctg gac aga gtg gac gca cgc     1008
   Val Phe Asp Glu Pro Asp Ile Leu Gln Leu Asp Arg Val Asp Ala Arg
                   325                 330                 335
   aag cac gcc gcg ttc ggc ggt ggt ata cac caa tgc ctc ggc cag caa     1056
   Lys His Ala Ala Phe Gly Gly Gly Ile His Gln Cys Leu Gly Gln Gln
               340                 345                 350
   ctg gcc aga gtg gag atg cgc atc ggc ttc acc cgg ctg ttc gcg agg     1104
   Leu Ala Arg Val Glu Met Arg Ile Gly Phe Thr Arg Leu Phe Ala Arg
           355                 360                 365
   ttc ccg tcg ctg cgg ctc gcg gtg ccg gcc gag gag atc aag ctg cgc     1152
   Phe Pro Ser Leu Arg Leu Ala Val Pro Ala Glu Glu Ile Lys Leu Arg
       370                 375                 380
   gag aag tcc gcg gcg tac ggg gtt tgg gcg ctg ccc gtt gca tgg gat     1200
   Glu Lys Ser Ala Ala Tyr Gly Val Trp Ala Leu Pro Val Ala Trp Asp
   385                 390                 395                 400
   gca tag                                                             1206
   Ala
<210> 3
   <211> 401
   <212> PRT
   <213> Amycolatopsis orientalis
<400> 3
   Met Arg Val Asp Ser Glu Asn Met Asn Glu Pro Val Thr Leu Pro Thr
   1               5                   10                  15
   Gly Arg Ala Val Gly Tyr Pro Phe Asp Pro Pro Pro Asp Leu Val Lys
               20                  25                  30
   Leu Pro Pro Val Ser Pro Met Arg Phe Pro Asp Gly His Ile Gly Trp
           35                  40                  45
   Leu Val Thr Ser His Ala Ala Ala Arg Thr Val Met Ile Asp Pro Arg
       50                  55                  60
   Phe Ser Asn Arg Pro Glu His Lys His Pro Val Phe Ser Val Ile Pro
   65                  70                  75                  80
   Arg Pro Gly Gly Ala Thr Lys Ala Pro Ala Pro Gly Trp Phe Ile Asn
                   85                  90                  95
   Met Asp Ala Pro Glu His Thr Arg Tyr Arg Arg Met Leu Ile Ser Gln
               100                 105                 110
   Phe Thr Val Arg Arg Ile Lys Glu Leu Glu Pro Arg Ile Val Gln Ile
           115                 120                 125
   Thr Glu Asp His Leu Asp Ala Met Ala Lys Ala Gly Pro Pro Val Asp
       130                 135                 140
   Leu Val Gln Ala Phe Ala Leu Pro Val Pro Ser Leu Val Ile Cys Glu
   145                 150                 155                 160
   Leu Leu Gly Val Ser Tyr Ala Asp His Ala Phe Phe Gln Glu Gln Thr
                   165                 170                 175
   Thr Ile Met Ala Ser Val Asp Lys Thr Gln Asp Glu Val Thr Thr Ala
               180                 185                 190
   Leu Gly Lys Leu Thr Arg Tyr Ile Ala Glu Leu Val Ala Thr Lys Arg
           195                 200                 205
   Leu Ser Pro Lys Asp Asp Leu Leu Gly Ser Leu Ile Thr Asp Thr Asp
       210                 215                 220
   Leu Thr Asp Glu Glu Leu Thr Asn Ile Ala Leu Leu Leu Leu Val Ala
   225                 230                 235                 240
   Gly His Glu Thr Thr Ala Asn Met Leu Gly Leu Gly Thr Phe Ala Leu
                   245                 250                 255
   Leu Gln His Pro Glu Gln Ile Ala Ala Leu Asp Ser Pro Asp Ala Val
               260                 265                 270
   Glu Glu Leu Leu Arg Tyr Leu Ser Ile Val His Leu Gly Thr Pro Asn
           275                 280                 285
   Arg Ala Ala Leu Glu Asp Val Glu Leu Glu Gly Gln Met Ile Arg Lys
       290                 295                 300
   Gly Asp Thr Val Ala Ile Gly Leu Pro Ala Val Asn Arg Asp Pro Lys
   305                 310                 315                 320
   Val Phe Asp Glu Pro Asp Ile Leu Gln Leu Asp Arg Val Asp Ala Arg
                   325                 330                 335
   Lys His Ala Ala Phe Gly Gly Gly Ile His Gln Cys Leu Gly Gln Gln
               340                 345                 350
   Leu Ala Arg Val Glu Met Arg Ile Gly Phe Thr Arg Leu Phe Ala Arg
           355                 360                 365
   Phe Pro Ser Leu Arg Leu Ala Val Pro Ala Glu Glu Ile Lys Leu Arg
       370                 375                 380
   Glu Lys Ser Ala Ala Tyr Gly Val Trp Ala Leu Pro Val Ala Trp Asp
   385                 390                 395                 400
   Ala
<210> 4
   <211> 2199
   <212> DNA
   <213> Artificial
<220>
   <223> Synthetic DNA
   <400> 4
   atgcgtgtcg actccgaaaa catgaacgag cctgtgaccc tccccaccgg ccgtgccgtg      60
   ggctacccct tcgaccctcc tcctgacctg gtgaagcttc ctcccgtgag ccccatgcgc     120
   ttccctgacg gccacatcgg ctggctggtg accagccacg ccgctgcgcg tactgtcatg     180
   atcgatcccc gcttcagcaa ccgccccgag cacaagcacc ctgtgttcag cgtcatcccc     240
   cgccccggcg gagccactaa ggcccccgcg cccggctggt tcatcaacat ggacgccccc     300
   gagcacaccc gttaccgccg catgctgatc agccagttca ccgtgcgccg tatcaaggaa     360
   ctcgaacctc gtatcgtcca gatcaccgag gaccacctcg acgcgatggc caaggctgga     420
   cctcctgtcg atctggtgca ggcgttcgcg ttgcctgtgc cttcgctggt gatctgcgag     480
   ctgctgggcg tctcgtacgc cgatcacgcg ttcttccagg agcagaccac catcatggcc     540
   tccgtggaca agactcagga tgaggtgacc accgcgcttg gcaagctcac ccgctacatc     600
   gctgaactgg tcgccactaa gcgtttgagc cccaaggacg acctgcttgg cagcttgatc     660
   actgacaccg atctgaccga tgaagagctg accaacatcg cgttgctttt gctcgtcgcc     720
   ggtcacgaga ccaccgcgaa catgttggga ctcggcactt tcgcgctcct tcagcacccc     780
   gagcagatcg ctgccctgga cagccccgac gcggtggagg aactgctgcg ctacctctcg     840
   atcgtccacc ttggaacccc caaccgtgcg gcgctggagg acgtggagct cgaaggccag     900
   atgatccgca agggcgacac tgtcgcgatc ggactgcccg cggtcaaccg tgaccccaag     960
   gtgttcgacg aacccgatat ccttcagctg gaccgtgtgg acgctcgcaa gcacgccgcg    1020
   ttcggcggtg gtattcacca gtgcctcggc cagcagctgg cccgtgtgga gatgcgcatc    1080
   ggcttcaccc gtctgttcgc gcgcttcccc tcgctgcgtc tcgcggtgcc cgccgaggag    1140
   atcaagctgc gcgagaagtc cgcggcgtac ggtgtttggg cgctgcccgt tgcttgggat    1200
   gcctctagtg tgctgcaccg tcaccagcct gtcaccatcg gagaacccgc cgcccgtgcg    1260
   gtgtcccgca ccgtcaccgt cgagcgcctg gaccgtatcg ccgacgacgt gctgcgcctc    1320
   gtcctgcgcg acgccggcgg aaagactctc cccacttgga ctcccggcgc ccacatcgac    1380
   ctcgacctcg gcgcgctgtc gcgccagtac tccctgtgcg gcgcgcccga tgcgcctagc    1440
   tacgagattg ccgtgcacct ggatcccgag agccgcggcg gttcgcgcta catccacgaa    1500
   cagctcgagg tgggaagccc tctccgtatg cgcggccctc gtaaccactt cgcgctcgac    1560
   cccggcgccg agcactacgt gttcgtcgcc ggcggcatcg gcatcacccc tgtcctggcc    1620
   atggccgacc acgcccgcgc ccgtggatgg agctacgaac tgcactactg cggccgtaac    1680
   cgttccggca tggcctacct cgagcgtgtc gccggtcacg gtgaccgtgc cgccctgcac   1740
   gtgtccgagg aaggcacccg tatcgacctc gccgccctcc tcgccgagcc cgcccccggc   1800
   gtccagatct acgcgtgcgg tgccggtcgt ctgctcgccg gactcgagga cgcgagccgt   1860
   aactggcccg acggtgcgct gcacgtcgag cacttcacct cgtccctcgc ggcgctcgat   1920
   cctgacgtcg agcacgcctt cgacctcgaa ctgcgtgact cgggtctgac cgtgcgtgtc   1980
   gaacccaccc agaccgtcct cgacgcgttg cgcgccaaca acatcgacgt gcccagcgac   2040
   tgcgaggaag gcctctgcgg ctcgtgcgag gtcgccgtcc tcgacggcga ggtcgaccac   2100
   cgcgacactg tgctgaccaa ggccgagcgt gcggcgaacc gtcagatgat gacctgctgc   2160
   tcgcgtgcct gcggcgaccg tctggccctg cgtctctaa                           2199
<210> 5
   <211> 2199
   <212> DNA
   <213> Artificial
<220>
   <223> synthetic DNA
<220>
   <221> CDS
   <222> (1)..(2199)
<400> 5
   atg cgt gtc gac tcc gaa aac atg aac gag cct gtg acc ctc ccc acc       48
   Met Arg Val Asp Ser Glu Asn Met Asn Glu Pro Val Thr Leu Pro Thr
   1               5                   10                  15
   ggc cgt gcc gtg ggc tac ccc ttc gac cct cct cct gac ctg gtg aag       96
   Gly Arg Ala Val Gly Tyr Pro Phe Asp Pro Pro Pro Asp Leu Val Lys
               20                  25                  30
   ctt cct ccc gtg agc ccc atg cgc ttc cct gac ggc cac atc ggc tgg      144
   Leu Pro Pro Val Ser Pro Met Arg Phe Pro Asp Gly His Ile Gly Trp
           35                  40                  45
   ctg gtg acc agc cac gcc gct gcg cgt act gtc atg atc gat ccc cgc      192
   Leu Val Thr Ser His Ala Ala Ala Arg Thr Val Met Ile Asp Pro Arg
       50                  55                  60
   ttc agc aac cgc ccc gag cac aag cac cct gtg ttc agc gtc atc ccc      240
   Phe Ser Asn Arg Pro Glu His Lys His Pro Val Phe Ser Val Ile Pro
   65                  70                  75                  80
   cgc ccc ggc gga gcc act aag gcc ccc gcg ccc ggc tgg ttc atc aac      288
   Arg Pro Gly Gly Ala Thr Lys Ala Pro Ala Pro Gly Trp Phe Ile Asn
                   85                  90                  95
   atg gac gcc ccc gag cac acc cgt tac cgc cgc atg ctg atc agc cag        336
   Met Asp Ala Pro Glu His Thr Arg Tyr Arg Arg Met Leu Ile Ser Gln
               100                 105                 110
   ttc acc gtg cgc cgt atc aag gaa ctc gaa cct cgt atc gtc cag atc        384
   Phe Thr Val Arg Arg Ile Lys Glu Leu Glu Pro Arg Ile Val Gln Ile
           115                 120                 125
   acc gag gac cac ctc gac gcg atg gcc aag gct gga cct cct gtc gat        432
   Thr Glu Asp His Leu Asp Ala Met Ala Lys Ala Gly Pro Pro Val Asp
       130                 135                 140
   ctg gtg cag gcg ttc gcg ttg cct gtg cct tcg ctg gtg atc tgc gag        480
   Leu Val Gln Ala Phe Ala Leu Pro Val Pro Ser Leu Val Ile Cys Glu
   145                 150                 155                 160
   ctg ctg ggc gtc tcg tac gcc gat cac gcg ttc ttc cag gag cag acc        528
   Leu Leu Gly Val Ser Tyr Ala Asp His Ala Phe Phe Gln Glu Gln Thr
                   165                 170                 175
   acc atc atg gcc tcc gtg gac aag act cag gat gag gtg acc acc gcg        576
   Thr Ile Met Ala Ser Val Asp Lys Thr Gln Asp Glu Val Thr Thr Ala
               180                 185                 190
   ctt ggc aag ctc acc cgc tac atc gct gaa ctg gtc gcc act aag cgt        624
   Leu Gly Lys Leu Thr Arg Tyr Ile Ala Glu Leu Val Ala Thr Lys Arg
           195                 200                 205
   ttg agc ccc aag gac gac ctg ctt ggc agc ttg atc act gac acc gat        672
   Leu Ser Pro Lys Asp Asp Leu Leu Gly Ser Leu Ile Thr Asp Thr Asp
       210                 215                 220
   ctg acc gat gaa gag ctg acc aac atc gcg ttg ctt ttg ctc gtc gcc        720
   Leu Thr Asp Glu Glu Leu Thr Asn Ile Ala Leu Leu Leu Leu Val Ala
   225                 230                 235                 240
   ggt cac gag acc acc gcg aac atg ttg gga ctc ggc act ttc gcg ctc        768
   Gly His Glu Thr Thr Ala Asn Met Leu Gly Leu Gly Thr Phe Ala Leu
                   245                 250                 255
   ctt cag cac ccc gag cag atc gct gcc ctg gac agc ccc gac gcg gtg        816
   Leu Gln His Pro Glu Gln Ile Ala Ala Leu Asp Ser Pro Asp Ala Val
               260                 265                 270
   gag gaa ctg ctg cgc tac ctc tcg atc gtc cac ctt gga acc ccc aac        864
   Glu Glu Leu Leu Arg Tyr Leu Ser Ile Val His Leu Gly Thr Pro Asn
           275                 280                 285
   cgt gcg gcg ctg gag gac gtg gag ctc gaa ggc cag atg atc cgc aag        912
   Arg Ala Ala Leu Glu Asp Val Glu Leu Glu Gly Gln Met Ile Arg Lys
       290                 295                 300
   ggc gac act gtc gcg atc gga ctg ccc gcg gtc aac cgt gac ccc aag        960
   Gly Asp Thr Val Ala Ile Gly Leu Pro Ala Val Asn Arg Asp Pro Lys
   305                 310                 315                 320
   gtg ttc gac gaa ccc gat atc ctt cag ctg gac cgt gtg gac gct cgc      1008
   Val Phe Asp Glu Pro Asp Ile Leu Gln Leu Asp Arg Val Asp Ala Arg
                   325                 330                 335
   aag cac gcc gcg ttc ggc ggt ggt att cac cag tgc ctc ggc cag cag      1056
   Lys His Ala Ala Phe Gly Gly Gly Ile His Gln Cys Leu Gly Gln Gln
               340                 345                 350
   ctg gcc cgt gtg gag atg cgc atc ggc ttc acc cgt ctg ttc gcg cgc      1104
   Leu Ala Arg Val Glu Met Arg Ile Gly Phe Thr Arg Leu Phe Ala Arg
           355                 360                 365
   ttc ccc tcg ctg cgt ctc gcg gtg ccc gcc gag gag atc aag ctg cgc      1152
   Phe Pro Ser Leu Arg Leu Ala Val Pro Ala Glu Glu Ile Lys Leu Arg
       370                 375                 380
   gag aag tcc gcg gcg tac ggt gtt tgg gcg ctg ccc gtt gct tgg gat      1200
   Glu Lys Ser Ala Ala Tyr Gly Val Trp Ala Leu Pro Val Ala Trp Asp
   385                 390                 395                 400
   gcc tct agt gtg ctg cac cgt cac cag cct gtc acc atc gga gaa ccc      1248
   Ala Ser Ser Val Leu His Arg His Gln Pro Val Thr Ile Gly Glu Pro
                   405                 410                 415
   gcc gcc cgt gcg gtg tcc cgc acc gtc acc gtc gag cgc ctg gac cgt      1296
   Ala Ala Arg Ala Val Ser Arg Thr Val Thr Val Glu Arg Leu Asp Arg
               420                 425                 430
   atc gcc gac gac gtg ctg cgc ctc gtc ctg cgc gac gcc ggc gga aag      1344
   Ile Ala Asp Asp Val Leu Arg Leu Val Leu Arg Asp Ala Gly Gly Lys
           435                 440                 445
   act ctc ccc act tgg act ccc ggc gcc cac atc gac ctc gac ctc ggc      1392
   Thr Leu Pro Thr Trp Thr Pro Gly Ala His Ile Asp Leu Asp Leu Gly
       450                 455                 460
   gcg ctg tcg cgc cag tac tcc ctg tgc ggc gcg ccc gat gcg cct agc      1440
   Ala Leu Ser Arg Gln Tyr Ser Leu Cys Gly Ala Pro Asp Ala Pro Ser
   465                 470                 475                 480
   tac gag att gcc gtg cac ctg gat ccc gag agc cgc ggc ggt tcg cgc      1488
   Tyr Glu Ile Ala Val His Leu Asp Pro Glu Ser Arg Gly Gly Ser Arg
                   485                 490                 495
   tac atc cac gaa cag ctc gag gtg gga agc cct ctc cgt atg cgc ggc      1536
   Tyr Ile His Glu Gln Leu Glu Val Gly Ser Pro Leu Arg Met Arg Gly
               500                 505                 510
   cct cgt aac cac ttc gcg ctc gac ccc ggc gcc gag cac tac gtg ttc      1584
   Pro Arg Asn His Phe Ala Leu Asp Pro Gly Ala Glu His Tyr Val Phe
           515                 520                 525
   gtc gcc ggc ggc atc ggc atc acc cct gtc ctg gcc atg gcc gac cac      1632
   Val Ala Gly Gly Ile Gly Ile Thr Pro Val Leu Ala Met Ala Asp His
       530                 535                 540
   gcc cgc gcc cgt gga tgg agc tac gaa ctg cac tac tgc ggc cgt aac      1680
   Ala Arg Ala Arg Gly Trp Ser Tyr Glu Leu His Tyr Cys Gly Arg Asn
   545                 550                 555                 560
   cgt tcc ggc atg gcc tac ctc gag cgt gtc gcc ggt cac ggt gac cgt      1728
   Arg Ser Gly Met Ala Tyr Leu Glu Arg Val Ala Gly His Gly Asp Arg
                   565                 570                 575
   gcc gcc ctg cac gtg tcc gag gaa ggc acc cgt atc gac ctc gcc gcc      1776
   Ala Ala Leu His Val Ser Glu Glu Gly Thr Arg Ile Asp Leu Ala Ala
               580                 585                 590
   ctc ctc gcc gag ccc gcc ccc ggc gtc cag atc tac gcg tgc ggt gcc      1824
   Leu Leu Ala Glu Pro Ala Pro Gly Val Gln Ile Tyr Ala Cys Gly Ala
           595                 600                 605
   ggt cgt ctg ctc gcc gga ctc gag gac gcg agc cgt aac tgg ccc gac      1872
   Gly Arg Leu Leu Ala Gly Leu Glu Asp Ala Ser Arg Asn Trp Pro Asp
       610                 615                 620
   ggt gcg ctg cac gtc gag cac ttc acc tcg tcc ctc gcg gcg ctc gat      1920
   Gly Ala Leu His Val Glu His Phe Thr Ser Ser Leu Ala Ala Leu Asp
   625                 630                 635                 640
   cct gac gtc gag cac gcc ttc gac ctc gaa ctg cgt gac tcg ggt ctg      1968
   Pro Asp Val Glu His Ala Phe Asp Leu Glu Leu Arg Asp Ser Gly Leu
                   645                 650                 655
   acc gtg cgt gtc gaa ccc acc cag acc gtc ctc gac gcg ttg cgc gcc      2016
   Thr Val Arg Val Glu Pro Thr Gln Thr Val Leu Asp Ala Leu Arg Ala
               660                 665                 670
   aac aac atc gac gtg ccc agc gac tgc gag gaa ggc ctc tgc ggc tcg      2064
   Asn Asn Ile Asp Val Pro Ser Asp Cys Glu Glu Gly Leu Cys Gly Ser
           675                 680                 685
   tgc gag gtc gcc gtc ctc gac ggc gag gtc gac cac cgc gac act gtg      2112
   Cys Glu Val Ala Val Leu Asp Gly Glu Val Asp His Arg Asp Thr Val
       690                 695                 700
   ctg acc aag gcc gag cgt gcg gcg aac cgt cag atg atg acc tgc tgc      2160
   Leu Thr Lys Ala Glu Arg Ala Ala Asn Arg Gln Met Met Thr Cys Cys
   705                 710                 715                 720
   tcg cgt gcc tgc ggc gac cgt ctg gcc ctg cgt ctc taa                  2199
   Ser Arg Ala Cys Gly Asp Arg Leu Ala Leu Arg Leu
                   725                 730
<210> 6
   <211> 732
   <212> PRT
   <213> Artificial
<220>
   <223> synthetic DNA
<400> 6
   Met Arg Val Asp Ser Glu Asn Met Asn Glu Pro Val Thr Leu Pro Thr
   1               5                   10                  15
   Gly Arg Ala Val Gly Tyr Pro Phe Asp Pro Pro Pro Asp Leu Val Lys
               20                  25                  30
   Leu Pro Pro Val Ser Pro Met Arg Phe Pro Asp Gly His Ile Gly Trp
           35                  40                  45
   Leu Val Thr Ser His Ala Ala Ala Arg Thr Val Met Ile Asp Pro Arg
       50                  55                  60
   Phe Ser Asn Arg Pro Glu His Lys His Pro Val Phe Ser Val Ile Pro
   65                  70                  75                  80
   Arg Pro Gly Gly Ala Thr Lys Ala Pro Ala Pro Gly Trp Phe Ile Asn
                   85                  90                  95
   Met Asp Ala Pro Glu His Thr Arg Tyr Arg Arg Met Leu Ile Ser Gln
               100                 105                 110
   Phe Thr Val Arg Arg Ile Lys Glu Leu Glu Pro Arg Ile Val Gln Ile
           115                 120                 125
   Thr Glu Asp His Leu Asp Ala Met Ala Lys Ala Gly Pro Pro Val Asp
       130                 135                 140
   Leu Val Gln Ala Phe Ala Leu Pro Val Pro Ser Leu Val Ile Cys Glu
   145                 150                 155                 160
   Leu Leu Gly Val Ser Tyr Ala Asp His Ala Phe Phe Gln Glu Gln Thr
                   165                 170                 175
   Thr Ile Met Ala Ser Val Asp Lys Thr Gln Asp Glu Val Thr Thr Ala
               180                 185                 190
   Leu Gly Lys Leu Thr Arg Tyr Ile Ala Glu Leu Val Ala Thr Lys Arg
           195                 200                 205
   Leu Ser Pro Lys Asp Asp Leu Leu Gly Ser Leu Ile Thr Asp Thr Asp
       210                 215                 220
   Leu Thr Asp Glu Glu Leu Thr Asn Ile Ala Leu Leu Leu Leu Val Ala
   225                 230                 235                 240
   Gly His Glu Thr Thr Ala Asn Met Leu Gly Leu Gly Thr Phe Ala Leu
                   245                 250                 255
   Leu Gln His Pro Glu Gln Ile Ala Ala Leu Asp Ser Pro Asp Ala Val
               260                 265                 270
   Glu Glu Leu Leu Arg Tyr Leu Ser Ile Val His Leu Gly Thr Pro Asn
           275                 280                 285
   Arg Ala Ala Leu Glu Asp Val Glu Leu Glu Gly Gln Met Ile Arg Lys
       290                 295                 300
   Gly Asp Thr Val Ala Ile Gly Leu Pro Ala Val Asn Arg Asp Pro Lys
   305                 310                 315                 320
   Val Phe Asp Glu Pro Asp Ile Leu Gln Leu Asp Arg Val Asp Ala Arg
                   325                 330                 335
   Lys His Ala Ala Phe Gly Gly Gly Ile His Gln Cys Leu Gly Gln Gln
               340                 345                 350
   Leu Ala Arg Val Glu Met Arg Ile Gly Phe Thr Arg Leu Phe Ala Arg
           355                 360                 365
   Phe Pro Ser Leu Arg Leu Ala Val Pro Ala Glu Glu Ile Lys Leu Arg
       370                 375                 380
   Glu Lys Ser Ala Ala Tyr Gly Val Trp Ala Leu Pro Val Ala Trp Asp
   385                 390                 395                 400
   Ala Ser Ser Val Leu His Arg His Gln Pro Val Thr Ile Gly Glu Pro
                   405                 410                 415
   Ala Ala Arg Ala Val Ser Arg Thr Val Thr Val Glu Arg Leu Asp Arg
               420                 425                 430
   Ile Ala Asp Asp Val Leu Arg Leu Val Leu Arg Asp Ala Gly Gly Lys
           435                 440                 445
   Thr Leu Pro Thr Trp Thr Pro Gly Ala His Ile Asp Leu Asp Leu Gly
   450 455 460
   Ala Leu Ser Arg Gln Tyr Ser Leu Cys Gly Ala Pro Asp Ala Pro Ser
   465 470 475 480
   Tyr Glu Ile Ala Val His Leu Asp Pro Glu Ser Arg Gly Gly Ser Arg
   485 490 495
   Tyr Ile His Glu Gln Leu Glu Val Gly Ser Pro Leu Arg Met Arg Gly
   500 505 510
   Pro Arg Asn His Phe Ala Leu Asp Pro Gly Ala Glu His Tyr Val Phe
   515 520 525
   Val Ala Gly Gly Ile Gly Ile Thr Pro Val Leu Ala Met Ala Asp His
   530 535 540
   Ala Arg Ala Arg Gly Trp Ser Tyr Glu Leu His Tyr Cys Gly Arg Asn
   545 550 555 560
   Arg Ser Gly Met Ala Tyr Leu Glu Arg Val Ala Gly His Gly Asp Arg
   565 570 575
   Ala Ala Leu His Val Ser Glu Glu Gly Thr Arg Ile Asp Leu Ala Ala
   580 585 590
   Leu Leu Ala Glu Pro Ala Pro Gly Val Gln Ile Tyr Ala Cys Gly Ala
   595 600 605
   Gly Arg Leu Leu Ala Gly Leu Glu Asp Ala Ser Arg Asn Trp Pro Asp
   610 615 620
   Gly Ala Leu His Val Glu His Phe Thr Ser Ser Leu Ala Ala Leu Asp
   625 630 635 640
   Pro Asp Val Glu His Ala Phe Asp Leu Glu Leu Arg Asp Ser Gly Leu
   645 650 655
   Thr Val Arg Val Glu Pro Thr Gln Thr Val Leu Asp Ala Leu Arg Ala
   660 665 670
   Asn Asn Ile Asp Val Pro Ser Asp Cys Glu Glu Gly Leu Cys Gly Ser
   675 680 685
   Cys Glu Val Ala Val Leu Asp Gly Glu Val Asp His Arg Asp Thr Val
       690                 695                 700
   Leu Thr Lys Ala Glu Arg Ala Ala Asn Arg Gln Met Met Thr Cys Cys
   705                 710                 715                 720
   Ser Arg Ala Cys Gly Asp Arg Leu Ala Leu Arg Leu
               725                 730
<210> 7
   <211> 33
   <212> DNA
   <213> Artificial
<220>
   <223> synthetic primer
<400> 7
   gggggtacca tggccgagat gacagagaaa gcc 33
<210> 8
   <211> 30
   <212> DNA
   <213> Artificial
<220>
   <223> synthetic primer
<400> 8
   gggggtacct caccaggtga ccgggagttc 30
<210> 9
   <211> 40
   <212> DNA
   <213> Artificial
<220>
<223> synthetic primer
<400> 9
   gggggtacca tgagagtaga ctccgaaaat atgaacgagc 40
<210> 10
   <211> 30
   <212> DNA
   <213> Artificial
<220>
   <223> synthetic primer
<400> 10
   gggggtaccc tatgcatccc atgcaacggg 30
<210> 11
   <211> 1206
   <212> DNA
   <213> Artificial
<220>
   <223> Synthetic DNA
<400> 11
   atgagagtag actccgaaaa tatgaacgag ccagtgacac taccgaccgg ccgagccgtg      60
   ggctacccct tcgacccacc gcctgacctg gtgaaacttc cgccagtgag cccgatgcga     120
   ttccctgacg gccacatcgg ctggctggtg accagccatg ccgcagcgcg gactgtcatg     180
   atcgatccgc gcttcagcaa ccgctccgag cacaagcacc cggtgttcag cgtcatccca     240
   cgaccaggcg gagccacgaa agccccggcg cccggctggt tcatcaacat ggacgcaccc     300
   gagcacacgc gttaccgccg gatgctgatc agccagttca ccgtgcgacg gatcaaggaa     360
   ctcgaaccgc ggatcgtcca gatcaccgag gaccacctcg acgcgatggc caaagcagga     420
   ccgccggtcg atctggtgca ggcgttcgcg ttgccagtgc cttcgctggt gatctgcgag     480
   ctgctgggcg tctcgtatgc cgatcacgcg ttcttccaag agcagaccac gatcatgtta     540
   agtgtggaca agacacagga tgaggtgacg acagcgcttg gcaaactcac ccgctacatc     600
   gctgaactgg tcgccacgaa gcggttgagc cccaaggacg acctgcttgg cagcttgatc     660
   acggacaccg atctgaccga tgaagagctg acgaacatcg cgttgatttt gctcgtcgcc     720
   gggcacgaga ccaccgcgaa catgctggga ttaggcactt tcgcgctcct tcagcatccg     780
   gagcagatcg ctgtcctgga cagcccggac gcggtggagg aactgctgcg ctacctctcg     840
   atcgtccacc ttggaacgcc caaccgggcg gcgctggagg acgtggagct cgaaggccag     900
   atgatcagga agggcgacac tgtcgcgatc ggactgcccg cggtcaatag ggacccgaag     960
   gtgttcgacg aacccgatat ccttcaactg gacagagtgg acgcacgcaa gcacgccgcg    1020
   ttcggcggtg gtatacacca atgcctcggc cagcaactgg ccagagtgga gatgcgcatc    1080
   ggcttcaccc ggctgttcgc gaggttcccg tcgctgcggc tcgcggtgcc ggccgaggag    1140
   atcaagctgc gcgagaagtc cgcggcgtac ggggtttggg cgctgcccgt tgcatgggat    1200
   gcatag                                                               1206
<210> 12
   <211> 1206
   <212> DNA
   <213> Artificial
<220>
   <223> Synthetic DNA
<400> 12
   atgagagtag actccgaaaa tatgaacgag ccagtgacac taccgaccgg ccgagccgtg 60
   ggctacccct tcgacccacc gcctgacctg gtgaaacttc cgccagtgag cccgatgcga 120
   ttccctgacg gccacatcgg ctggctggtg accagccatg ccgcagcgcg gaccgtcatg 180
   atcgatccgc gcttcagcaa ccgccccgag cacaagcacc cggtgttcag cgtcatccca 240
   cgaccaggcg gagccacgaa agccccggcg cccggctggt tctttaacat ggacgcaccc 300
   gagcacacgc gttaccgccg gatgctgatc agccagttca ccgtgcgacg gatcaaggaa 360
   ctcgaaccgc ggatcgtcca gatcaccgag gaccacctcg acgcgatggc caaagcagga 420
   ccgccggtcg atctggtgca ggcgttcgcg ttgccagtgc cttcgctggt gatctgcgag 480
   ctgctgggcg tctcgtatgc cgatcacgcg ttcttccaag agcagaccac gatcatggtc 540
   agtgtggaca agacacagga tgaggtgacg acggcgcttg gcaaactcac ccgctacatc 600
   gctgaactgg tcgccacgaa gcggttgagc cccaaggacg acctgcttgg cagcttgatc 660
   acggacaccg atctgaccga tgaagagctg acgaacatcg cgttgctttt gctcgtcgcc 720
   gggcacgaga ccaccgcgaa catgttggga ttaggcactt tcgcgctcct tcagcatccg 780
   gagcagatcg ctgccctgga cagcccggac gcggtggagg aactgctgcg ctacctctcg 840
   atcgtccacc ttggaacgcc caaccgggcg gcgctggagg acgtggagct cgaaggccag 900
   atgatcagga agggcgacac tgtcgcgatc ggactgcccg cggtcaatag ggacccgaag 960
   gtgttcgacg aacccgatat ccttcaactg gacagagtgg acgcacgcaa gcacgccgcg 1020
   ttcggcggtg gtatacacca atgcctcggc cagcaactgg ccagagtgga gatgcgcatc 1080
   ggcttcaccc ggctgttcgc gaggttcccg tcgctgcggc tcgcggtgcc ggccgaggag 1140
   atcaagctgc gcgagaagtc cgcggcgtac ggggtttggg cgctgcccgt tgcatgggat 1200
   gcatag 1206
<210> 13
   <211> 1206
   <212> DNA
   <213> Artificial
<220>
   <223> Synthetic DNA
<400> 13
   atgagagtag actccgaaaa tatgaacgag ccagtgacac taccgaccgg ccgagccgtg 60
   ggctacccct tcgacccacc gcctgacctg gtgaaacttc cgccagtgag cccgatgcga 120
   ttccctgacg gccacatcgg ctggctggtg accagccatg ccgcagcgcg gactgtcatg 180
   atcgatccgc gcttcagcaa ccgccccgag cacaagcacc cggtgttcag cgtcatccca 240
   cgaccaggcg gagccacgaa agccccggcg cccggctggt tcaccaacat ggacgcaccc 300
   gagcacacgc gttaccgccg gatgctgatc agccagttca ccgtgcgacg gatcaaggaa 360
   ctcgaaccgc ggatcgtcca gatcaccgag gaccacctcg acgcgatggc caaagcagga 420
   ccgccggtcg atctggtgca ggcgttcgcg ttgccagtgc cttcgctggt gatctgcgag 480
   ctgctgggcg tctcgtatgc cgatcacgcg ttcttccaag agcagaccac gatcatggtc 540
   agtgtggaca agacacagga tgaggtgacg acggcgcttg gcaaactcac ccgctacatc 600
   gctgaactgg tcgccacgaa gcggttgagc cccaaggacg acctgcttgg cagcttgatc 660
   acggacaccg atctgaccga tgaagagctg acgaacatcg cgttgatttt gctcgtcgcc 720
   gggcacgaga ccaccgcgaa catgttggga ttaggcactt tcgtgctcct tcagcacccg 780
   gagcagatcg ctcttctgga cagcccggac gcggtggagg aactgctgcg ctacctctcg 840
   atcgtccacc ttggaacgcc caaccgggcg gcgctggagg acgtggagct cgaaggccag 900
   atgatcagga agggcgacac tgtcgcgatc ggactgcccg cggtcaatag ggacccgaag 960
   gtgttcgacg aacccgatat ccttcaactg gacagagtgg acgcacgcaa gcacgccgcg 1020
   ttcggcggtg gtatacacca atgcctcggc cagcaactgg ccagagtgga gatgcgcatc 1080
   ggcttcaccc ggctgttcgc gaggttcccg tcgctgcggc tcgcggtgcc ggccgaggag 1140
   atcaagctgc gcgagaagtc cgcggcgtac ggggtttggg cgctgcccgt tgcatgggat 1200
   gcatag 1206
<210> 14
   <211> 1206
   <212> DNA
   <213> Artificial
<220>
   <223> Synthetic DNA
<400> 14
   atgagagtag actccgaaaa tatgaacgag ccagtgacac taccgaccgg ccgagccgtg 60
   ggctacccct tcgacccacc gcctgacctg gtgaaacttc cgccagtgag cccgatgcga 120
   tttcctgacg gccacatcgg ctggctggtg accagccatg ccgcagcgcg gactgtcatg 180
   atcgatccgc gcttcagcaa ccgccccgag cacaagcacc cggtgttcag cgtcatccca 240
   cgaccaggcg gagccacgaa agccccggcg cccggctggt tcgccaacat ggacgcaccc 300
   gagcacacgc gttaccgccg gatgctgatc agccagttca ccgtgcgacg gatcaaggaa 360
   ctcgaaccgc ggatcgtcca gatcaccgag gaccacctcg acgcgatggc caaagcagga 420
   ccgccggtcg atctggtgca ggcgttcgcg ttgccagtgc cttcgctggt gatctgcgag 480
   ctgctgggcg tctcgtatgc tgatcacgcg ttcttccaag aacagaccac gatcatgttt 540
   agtgtggaca agacacagga tgaggtgacg acggcgcttg gcaaactcac ccgctacatc 600
   gctgaactgg tcgccacgaa gcggttgagc cctaaggacg acctgcttgg cagcttgatc 660
   acggacaccg atctgaccga tgaagagctg acgaacaccg cgttgctttt gctcgtcgcc 720
   gggcacgaga ccaccgcgaa catgttggga ttaggcactt tcgcgctcct tcagcatccg 780
   gagcagatcg ctgccctgga cagcccggac gcggtggagg aactgctgcg ctacctctcg 840
   atcgtccacc ttggagcgcc caaccgggcg gcgctggagg acgtggagct cgaaggccag 900
   atgatcagga agggcgacac tgtcgcgatc ggactgcccg cggtcaatag ggacccgaag 960
   gtgttcgacg aacccgatat ccttcaactg gacagagtgg acgcacgcaa gcacgccgcg 1020
   ttcggcggtg gtatacacca atgcctcggc cagcaactgg ccagagtgga gatgcgcatc 1080
   ggcttcaccc ggctgttcgc gaggttcccg tcgctgcggc tcgcggtgcc ggccgaggag 1140
   atcaagctgc gcgagaagtc cgcggcgtac ggggtttggg cgctgcccgt tgcatgggat 1200
   gcatag 1206
<210> 15
   <211> 1206
   <212> DNA
   <213> Artificial
<220>
   <223> Synthetic DNA
<400> 15
   atgagagtag actccgaaaa tatgaacgag ccagtgacac taccgaccgg ccgagccgtg 60
   ggctacccct tcgacccacc gcctgacctg gtgaaacttc cgccagtgag cccgatgcga 120
   ttccctgacg gccacatcgg ctggctggtg accagccatg ccgcagcgcg gactgtcatg 180
   atcgatccgc gcttcagcaa ccgccccgag cacaagcacc cggtgttcag cgtcatccca 240
   cgaccaggcg gagccacgaa agccccggcg cccggctggt tcaccaacat ggacgcaccc 300
   gagcacacgc gttaccgccg gatgctgatc agccagttca ccgtgcgacg gatcaaggaa 360
   ctcgaaccgc ggatcgtccg gatcaccgag gaccacctcg acgcgatggc caaagcagga 420
   ccgccggtcg atctggtgca ggcgttcgcg ttgccagtgc cttcgctggt gatctgcgag 480
   ctgctgggcg tctcgtatgc cgatcacgcg ttcttccaag agcagaccac gatcatggtc 540
   agtgtggaca agacacagga tgaggtgacg acggcgcttg gcaaactcac ccgctacatc 600
   gctgaactgg tcgccacgaa gcggttgagc cccaaggacg acctgcttgg cagcttgatc 660
   acggacaccg atctgaccga tgaagagctg acgaacatcg cgttgatttt gctcgtcgcc 720
   gggcacgaga ccaccgcgaa catgttggga ttaggcactt tcgcgctcct tcagcatccg 780
   gagcagatcg ctaatctgga cagcccggac gcggtggagg aactgctgcg ctacctctcg 840
   atcgtccacc ttggaacgcc caaccgggcg gcgctggagg acgtggagct cgaaggccag 900
   atgatcagga agggcgacac tgtcgcgatc ggactgcccg cggtcaatag ggacccgaag 960
   gtgttcgacg aacccgatat ccttcaactg gacagagtgg acgcacgcaa gcacgccgcg 1020
   ttcggcggtg gtatacacca atgcctcggc cagcaactgg ccagagtgga gatgcgcatc 1080
   ggcttcaccc ggctgttcgc gaggttcccg tcgctgcggc tcgcggtgcc ggccgaggag 1140
   atcaagctgc gcgagaagtc cgcggcgtac ggggtttggg cgctgcccgt tgcatgggat 1200
   gcatag 1206
<210> 16
   <211> 1206
   <212> DNA
   <213> Artificial
<220>
   <223> Synthetic DNA
<400> 16
   atgagagtag actccgaaaa tatgaacgag ccagtgacac taccgaccgg ccgagccgtg 60
   ggctacccct tcgacccacc gcctgacctg gtgaaacttc cgccagtgag cccgatgcga 120
   ttccctgacg gccacatcgg ctggctggtg accagccatg ccgcagcgcg gactgtcatg 180
   atcgatccgc gcttcagcaa ccgccccgag cacaagcacc cggtgttcag cgtcatccca 240
   cgaccaggcg gagccacgaa agccccggcg cccggctggt tcatcaacat ggacgcaccc 300
   gagcacacgc gttaccgccg gatgctgatc agccagttca ccgtgcgacg gatcaaggaa 360
   ctcgaaccgc ggatcgtcca gatcaccgag gaccacctcg acgcgatggc caaagcagga 420
   ccgccggtcg atctggtgca ggcgttcgcg ttgccagtgc cttcgctggt gatctgcgag 480
   ctgctgggcg tctcgtatgc tgatcacgcg ttcttccaag agcagaccac gatcatgctg 540
   agtgtggaca agacacagga taaggtgacg acggcgcttg gcaaactcac ccgctacatc 600
   gctgaactgg tcgccacgaa gcggttgagc cccaaggacg acctgcttgg cagcttgatc 660
   acggacaccg atctgaccga tgaagagctg acgaacatcg cgttgatttt gctcgtcgcc 720
   gggcacgaga ccaccgcgaa catgttggga ttaggcactt tcgcgctcct tcagcatccg 780
   gagcagatcg ctgtcctgga cagcccggac gcggtggagg aactgctgcg ctacctctcg 840
   atcgtccacc ttggaacgcc caaccgggcg gcgctggagg acgtggagct cgaaggccag 900
   atgatcagga agggcgacac tgtcgcgatc ggactgcccg cggtcaatag ggacccgaag 960
   gtgttcgacg aacccgatat ccttcaactg gacagagtgg acgcacgcaa gcacgccgcg 1020
   ttcggcggtg gtatacacca atgcctcggc cagcaactgg ccagagtgga gatgcgcatc 1080
   ggcttcaccc ggctgttcgc gaggttcccg tcgctgcggc tcgcggtgcc ggccgaggag 1140
   atcaagctgc gcgagaagtc cgcggcgtac ggggtttggg cgctgcccgt tgcatgggat 1200
   gcatag 1206
<210> 17
   <211> 1206
   <212> DNA
   <213> Artificial
<220>
   <223> Synthetic DNA
<400> 17
   atgagagtag actccgaaaa tatgaacgag ccagtgacac taccgaccgg ccgagccgtg 60
   ggctacccct tcgacccacc gcctgacctg gtgaaacttc cgccagtgag cccgatgcga 120
   ttccctgacg gccacatcgg ctggctggtg accagccatg ccgcagcgcg gactgtcatg 180
   atcgatccgc gcttcagcaa ccgccccgag cacaagcacc cggtgttcag cgtcatccca 240
   cgaccaggcg gagccacgaa agccccggcg cccggctggt tcatcaacat ggacgcaccc 300
   gagcacacgc gttaccgccg gatgctgatc agccagttca ccgtgcgacg gatcaaggaa 360
   ctcgaaccgc ggatcgtcca gatcaccgag gaccgcctcg acgcgatggc caaagcagga 420
   ccgccggtcg atctggtgca ggcgttcgcg ttgccagtgc cttcgctggt gatctgcgag 480
   ctgctgggcg tctcgtatgc cgatcacgcg ttcttccaag agcagaccac gatcatgctt 540
   agtgtggaca agacacagga tgaggtgacg acggcgcttg gcaaactcac ccgctacatc 600
   gctgaactgg tcgccacgaa gcggttgagc cccaaggacg acctgcttgg cagcttgatc 660
   acggacaccg atctgaccga tgaagagctg acgaacatcg cgttgatttt gctcgtcgcc 720
   gggcacgaga ccaccgcgaa catgttggga ttaggcactt tcgcgctcct tcagcatccg 780
   gagcagatcg ctgtcctgga cagcccggac gcggtggagg aactgctgcg ctacctctcg 840
   atcgtccacc ttggaacgcc caaccgggcg gcgctggagg acatggagct cgaaggccag 900
   atgatcagga agggcgacac tgtcgcgatc ggactgcccg cggtcaatag ggacccgaag 960
   gtgttcgacg aacccgatat ccttcaactg gacagagtgg acgcacgcaa gcacgccgcg 1020
   ttcggcggtg gtatacacca atgcctcggc cagcaactgg ccagagtgga gatgcgcatc 1080
   ggcctcaccc ggctgttcgc gaggttcccg tcgctgcggc tcgcggtgcc ggccgaggag 1140
   atcaagctgc gcgagaagtc cgcggcgtac ggggtttggg cgctgcccgt tgcatgggat 1200
   gcatag 1206
<210> 18
   <211> 1206
   <212> DNA
   <213> Artificial
<220>
   <223> Synthetic DNA
<400> 18
   atgagagtag actccgaaaa tatgaacgag ccagtgacac taccgaccgg ccgagccgtg 60
   ggctaccccc tcgacccacc gcctgacctg gtgaaacttc cgccagtgag cccgatgcga 120
   ttccctgacg gccacatcgg ctggctggtg accagccatg ccgcagcgcg gactgtcatg 180
   atcgatccgc gcttcagcaa ccgccccgag cacaagcacc cggtgttcag cgtcatccca 240
   cgaccaggcg gagccacgaa agccccggcg cccggctggt tcatcaacat ggacgcaccc 300
   gagcacacgc gttaccgccg gatgctgatc agccagttca ccgtgcgacg gatcaaggaa 360
   ctcgaaccgc ggatcgtcca gatcaccgag gaccacctcg acgcgatggc caaagcagga 420
   ccgccggtcg atctggtgca ggcgttcgcg ttgccagtgc cttcgctggt gatctgcgag 480
   ctgctgggcg tctcgtatgc cgatcacgcg ttcttccaag agcagaccac gatcatgttg 540
   agtgtggaca agacacagga tgaggtgacg acggcgcttg gcaaactcac ccgctacatc 600
   gctgaactgg tcgccacgaa gcggttgagc cccaaggacg acctgcttgg cagcttgatc 660
   acggacaccg atctgaccga tgaagagctg acgaacatcg cgttgatttt gctcgtcgcc     720
   gggcacgaga ccaccgcgaa catgttggga ttaggcactt tcgcgctcct tcagcatccg     780
   gagcagatcg cttgcctgga cagcccggac gcggtggagg aactgctgcg ctacctctcg     840
   atcgtccacc ttggaacgcc caaccgggcg gcgctggagg acgtggagct cgaaggccag     900
   atgatcagga agggcgacac tgtcgcgatc ggactgcccg cggtcaatag ggacccgaag     960
   gtgttcgacg aacccgatat ccttcaactg gacagagtgg acgcacgcaa gcacgccgcg    1020
   ttcggcggtg gtatacacca atgcctcggc cagcaactgg ccagagtgga gatgcgcatc    1080
   ggcttcaccc ggctgttcgc gaggttcccg tcgctgcggc tcgcggtgcc ggccgaggag    1140
   atcaagctgc gcgagaagtc cgcggcgtac ggggtttggg cgctgcccgt tgcatgggat    1200
   gcatag                                                               1206
<210> 19
   <211> 401
   <212> PRT
   <213> Artificial
<220>
   <223> Synthetic protein
<400> 19
   Met Arg Val Asp Ser Glu Asn Met Asn Glu Pro Val Thr Leu Pro Thr
   1               5                   10                  15
   Gly Arg Ala Val Gly Tyr Pro Phe Asp Pro Pro Pro Asp Leu Val Lys
               20                  25                  30
   Leu Pro Pro Val Ser Pro Met Arg Phe Pro Asp Gly His Ile Gly Trp
           35                  40                  45
   Leu Val Thr Ser His Ala Ala Ala Arg Thr Val Met Ile Asp Pro Arg
       50                  55                  60
   Phe Ser Asn Arg Ser Glu His Lys His Pro Val Phe Ser Val Ile Pro
   65                  70                  75                  80
   Arg Pro Gly Gly Ala Thr Lys Ala Pro Ala Pro Gly Trp Phe Ile Asn
                   85                  90                  95
   Met Asp Ala Pro Glu His Thr Arg Tyr Arg Arg Met Leu Ile Ser Gln
   100 105 110
   Phe Thr Val Arg Arg Ile Lys Glu Leu Glu Pro Arg Ile Val Gln Ile
   115 120 125
   Thr Glu Asp His Leu Asp Ala Met Ala Lys Ala Gly Pro Pro Val Asp
   130 135 140
   Leu Val Gln Ala Phe Ala Leu Pro Val Pro Ser Leu Val Ile Cys Glu
   145 150 155 160
   Leu Leu Gly Val Ser Tyr Ala Asp His Ala Phe Phe Gln Glu Gln Thr
   165 170 175
   Thr Ile Met Leu Ser Val Asp Lys Thr Gln Asp Glu Val Thr Thr Ala
   180 185 190
   Leu Gly Lys Leu Thr Arg Tyr Ile Ala Glu Leu Val Ala Thr Lys Arg
   195 200 205
   Leu Ser Pro Lys Asp Asp Leu Leu Gly Ser Leu Ile Thr Asp Thr Asp
   210 215 220
   Leu Thr Asp Glu Glu Leu Thr Asn Ile Ala Leu Ile Leu Leu Val Ala
   225 230 235 240
   Gly His Glu Thr Thr Ala Asn Met Leu Gly Leu Gly Thr Phe Ala Leu
   245 250 255
   Leu Gln His Pro Glu Gln Ile Ala Val Leu Asp Ser Pro Asp Ala Val
   260 265 270
   Glu Glu Leu Leu Arg Tyr Leu Ser Ile Val His Leu Gly Thr Pro Asn
   275 280 285
   Arg Ala Ala Leu Glu Asp Val Glu Leu Glu Gly Gln Met Ile Arg Lys
   290 295 300
   Gly Asp Thr Val Ala Ile Gly Leu Pro Ala Val Asn Arg Asp Pro Lys
   305 310 315 320
   Val Phe Asp Glu Pro Asp Ile Leu Gln Leu Asp Arg Val Asp Ala Arg
   325 330 335
   Lys His Ala Ala Phe Gly Gly Gly Ile His Gln Cys Leu Gly Gln Gln
   340 345 350
   Leu Ala Arg Val Glu Met Arg Ile Gly Phe Thr Arg Leu Phe Ala Arg
   355 360 365
   Phe Pro Ser Leu Arg Leu Ala Val Pro Ala Glu Glu Ile Lys Leu Arg
   370 375 380
   Glu Lys Ser Ala Ala Tyr Gly Val Trp Ala Leu Pro Val Ala Trp Asp
   385 390 395 400
   Ala
<210> 20
   <211> 401
   <212> PRT
   <213> Artificial
<220>
   <223> Synthetic protein
<400> 20
   Met Arg Val Asp Ser Glu Asn Met Asn Glu Pro Val Thr Leu Pro Thr
   1 5 10 15
   Gly Arg Ala Val Gly Tyr Pro Phe Asp Pro Pro Pro Asp Leu Val Lys
   20 25 30
   Leu Pro Pro Val Ser Pro Met Arg Phe Pro Asp Gly His Ile Gly Trp
   35 40 45
   Leu Val Thr Ser His Ala Ala Ala Arg Thr Val Met Ile Asp Pro Arg
   50 55 60
   Phe Ser Asn Arg Pro Glu His Lys His Pro Val Phe Ser Val Ile Pro
   65 70 75 80
   Arg Pro Gly Gly Ala Thr Lys Ala Pro Ala Pro Gly Trp Phe Phe Asn
   85 90 95
   Met Asp Ala Pro Glu His Thr Arg Tyr Arg Arg Met Leu Ile Ser Gln
                   100                 105                 110
   Phe Thr Val Arg Arg Ile Lys Glu Leu Glu Pro Arg Ile Val Gln Ile
           115                 120                 125
   Thr Glu Asp His Leu Asp Ala Met Ala Lys Ala Gly Pro Pro Val Asp
       130                 135                 140
   Leu Val Gln Ala Phe Ala Leu Pro Val Pro Ser Leu Val Ile Cys Glu
   145                 150                 155                 160
   Leu Leu Gly Val Ser Tyr Ala Asp His Ala Phe Phe Gln Glu Gln Thr
                   165                 170                 175
   Thr Ile Met Val Ser Val Asp Lys Thr Gln Asp Glu Val Thr Thr Ala
               180                 185                 190
   Leu Gly Lys Leu Thr Arg Tyr Ile Ala Glu Leu Val Ala Thr Lys Arg
           195                 200                 205
   Leu Ser Pro Lys Asp Asp Leu Leu Gly Ser Leu Ile Thr Asp Thr Asp
       210                 215                 220
   Leu Thr Asp Glu Glu Leu Thr Asn Ile Ala Leu Leu Leu Leu Val Ala
   225                 230                 235                 240
   Gly His Glu Thr Thr Ala Asn Met Leu Gly Leu Gly Thr Phe Ala Leu
                   245                 250                 255
   Leu Gln His Pro Glu Gln Ile Ala Ala Leu Asp Ser Pro Asp Ala Val
               260                 265                 270
   Glu Glu Leu Leu Arg Tyr Leu Ser Ile Val His Leu Gly Thr Pro Asn
           275                 280                 285
   Arg Ala Ala Leu Glu Asp Val Glu Leu Glu Gly Gln Met Ile Arg Lys
       290                 295                 300
   Gly Asp Thr Val Ala Ile Gly Leu Pro Ala Val Asn Arg Asp Pro Lys
   305                 310                 315                 320
   Val Phe Asp Glu Pro Asp Ile Leu Gln Leu Asp Arg Val Asp Ala Arg
                   325                 330                 335
   Lys His Ala Ala Phe Gly Gly Gly Ile His Gln Cys Leu Gly Gln Gln
               340                 345                 350
   Leu Ala Arg Val Glu Met Arg Ile Gly Phe Thr Arg Leu Phe Ala Arg
           355                 360                 365
   Phe Pro Ser Leu Arg Leu Ala Val Pro Ala Glu Glu Ile Lys Leu Arg
       370                 375                 380
   Glu Lys Ser Ala Ala Tyr Gly Val Trp Ala Leu Pro Val Ala Trp Asp
   385                 390                 395                 400
   Ala
<210> 21
   <211> 401
   <212> PRT
   <213> Artificial
<220>
   <223> Synthetic protein
<400> 21
   Met Arg Val Asp Ser Glu Asn Met Asn Glu Pro Val Thr Leu Pro Thr
   1               5                   10                  15
   Gly Arg Ala Val Gly Tyr Pro Phe Asp Pro Pro Pro Asp Leu Val Lys
               20                  25                  30
   Leu Pro Pro Val Ser Pro Met Arg Phe Pro Asp Gly His Ile Gly Trp
           35                  40                  45
   Leu Val Thr Ser His Ala Ala Ala Arg Thr Val Met Ile Asp Pro Arg
       50                  55                  60
   Phe Ser Asn Arg Pro Glu His Lys His Pro Val Phe Ser Val Ile Pro
   65                  70                  75                  80
   Arg Pro Gly Gly Ala Thr Lys Ala Pro Ala Pro Gly Trp Phe Thr Asn
                   85                  90                  95
   Met Asp Ala Pro Glu His Thr Arg Tyr Arg Arg Met Leu Ile Ser Gln
   100 105 110
   Phe Thr Val Arg Arg Ile Lys Glu Leu Glu Pro Arg Ile Val Gln Ile
   115 120 125
   Thr Glu Asp His Leu Asp Ala Met Ala Lys Ala Gly Pro Pro Val Asp
   130 135 140
   Leu Val Gln Ala Phe Ala Leu Pro Val Pro Ser Leu Val Ile Cys Glu
   145 150 155 160
   Leu Leu Gly Val Ser Tyr Ala Asp His Ala Phe Phe Gln Glu Gln Thr
   165 170 175
   Thr Ile Met Val Ser Val Asp Lys Thr Gln Asp Glu Val Thr Thr Ala
   180 185 190
   Leu Gly Lys Leu Thr Arg Tyr Ile Ala Glu Leu Val Ala Thr Lys Arg
   195 200 205
   Leu Ser Pro Lys Asp Asp Leu Leu Gly Ser Leu Ile Thr Asp Thr Asp
   210 215 220
   Leu Thr Asp Glu Glu Leu Thr Asn Ile Ala Leu Ile Leu Leu Val Ala
   225 230 235 240
   Gly His Glu Thr Thr Ala Asn Met Leu Gly Leu Gly Thr Phe Val Leu
   245 250 255
   Leu Gln His Pro Glu Gln Ile Ala Leu Leu Asp Ser Pro Asp Ala Val
   260 265 270
   Glu Glu Leu Leu Arg Tyr Leu Ser Ile Val His Leu Gly Thr Pro Asn
   275 280 285
   Arg Ala Ala Leu Glu Asp Val Glu Leu Glu Gly Gln Met Ile Arg Lys
   290 295 300
   Gly Asp Thr Val Ala Ile Gly Leu Pro Ala Val Asn Arg Asp Pro Lys
   305 310 315 320
   Val Phe Asp Glu Pro Asp Ile Leu Gln Leu Asp Arg Val Asp Ala Arg
   325 330 335
   Lys His Ala Ala Phe Gly Gly Gly Ile His Gln Cys Leu Gly Gln Gln
   340 345 350
   Leu Ala Arg Val Glu Met Arg Ile Gly Phe Thr Arg Leu Phe Ala Arg
   355 360 365
   Phe Pro Ser Leu Arg Leu Ala Val Pro Ala Glu Glu Ile Lys Leu Arg
   370 375 380
   Glu Lys Ser Ala Ala Tyr Gly Val Trp Ala Leu Pro Val Ala Trp Asp
   385 390 395 400
   Ala
<210> 22
   <211> 401
   <212> PRT
   <213> Artificial
<220>
   <223> Synthetic protein
<400> 22
   Met Arg Val Asp Ser Glu Asn Met Asn Glu Pro Val Thr Leu Pro Thr
   1 5 10 15
   Gly Arg Ala Val Gly Tyr Pro Phe Asp Pro Pro Pro Asp Leu Val Lys
   20 25 30
   Leu Pro Pro Val Ser Pro Met Arg Phe Pro Asp Gly His Ile Gly Trp
   35 40 45
   Leu Val Thr Ser His Ala Ala Ala Arg Thr Val Met Ile Asp Pro Arg
   50 55 60
   Phe Ser Asn Arg Pro Glu His Lys His Pro Val Phe Ser Val Ile Pro
   65 70 75 80
   Arg Pro Gly Gly Ala Thr Lys Ala Pro Ala Pro Gly Trp Phe Ala Asn
   85 90 95
   Met Asp Ala Pro Glu His Thr Arg Tyr Arg Arg Met Leu Ile Ser Gln
   100 105 110
   Phe Thr Val Arg Arg Ile Lys Glu Leu Glu Pro Arg Ile Val Gln Ile
   115 120 125
   Thr Glu Asp His Leu Asp Ala Met Ala Lys Ala Gly Pro Pro Val Asp
   130 135 140
   Leu Val Gln Ala Phe Ala Leu Pro Val Pro Ser Leu Val Ile Cys Glu
   145 150 155 160
   Leu Leu Gly Val Ser Tyr Ala Asp His Ala Phe Phe Gln Glu Gln Thr
   165 170 175
   Thr Ile Met Phe Ser Val Asp Lys Thr Gln Asp Glu Val Thr Thr Ala
   180 185 190
   Leu Gly Lys Leu Thr Arg Tyr Ile Ala Glu Leu Val Ala Thr Lys Arg
   195 200 205
   Leu Ser Pro Lys Asp Asp Leu Leu Gly Ser Leu Ile Thr Asp Thr Asp
   210 215 220
   Leu Thr Asp Glu Glu Leu Thr Asn Thr Ala Leu Leu Leu Leu Val Ala
   225 230 235 240
   Gly His Glu Thr Thr Ala Asn Met Leu Gly Leu Gly Thr Phe Ala Leu
   245 250 255
   Leu Gln His Pro Glu Gln Ile Ala Ala Leu Asp Ser Pro Asp Ala Val
   260 265 270
   Glu Glu Leu Leu Arg Tyr Leu Ser Ile Val His Leu Gly Ala Pro Asn
   275 280 285
   Arg Ala Ala Leu Glu Asp Val Glu Leu Glu Gly Gln Met Ile Arg Lys
   290 295 300
   Gly Asp Thr Val Ala Ile Gly Leu Pro Ala Val Asn Arg Asp Pro Lys
   305 310 315 320
   Val Phe Asp Glu Pro Asp Ile Leu Gln Leu Asp Arg Val Asp Ala Arg
   325 330 335
   Lys His Ala Ala Phe Gly Gly Gly Ile His Gln Cys Leu Gly Gln Gln
   340 345 350
   Leu Ala Arg Val Glu Met Arg Ile Gly Phe Thr Arg Leu Phe Ala Arg
   355 360 365
   Phe Pro Ser Leu Arg Leu Ala Val Pro Ala Glu Glu Ile Lys Leu Arg
   370 375 380
   Glu Lys Ser Ala Ala Tyr Gly Val Trp Ala Leu Pro Val Ala Trp Asp
   385 390 395 400
   Ala
<210> 23
   <211> 401
   <212> PRT
   <213> Artificial
<220>
   <223> Synthetic protein
<400> 23
   Met Arg Val Asp Ser Glu Asn Met Asn Glu Pro Val Thr Leu Pro Thr
   1 5 10 15
   Gly Arg Ala Val Gly Tyr Pro Phe Asp Pro Pro Pro Asp Leu Val Lys
   20 25 30
   Leu Pro Pro Val Ser Pro Met Arg Phe Pro Asp Gly His Ile Gly Trp
   35 40 45
   Leu Val Thr Ser His Ala Ala Ala Arg Thr Val Met Ile Asp Pro Arg
   50 55 60
   Phe Ser Asn Arg Pro Glu His Lys His Pro Val Phe Ser Val Ile Pro
   65 70 75 80
   Arg Pro Gly Gly Ala Thr Lys Ala Pro Ala Pro Gly Trp Phe Thr Asn
   85 90 95
   Met Asp Ala Pro Glu His Thr Arg Tyr Arg Arg Met Leu Ile Ser Gln
   100 105 110
   Phe Thr Val Arg Arg Ile Lys Glu Leu Glu Pro Arg Ile Val Arg Ile
   115 120 125
   Thr Glu Asp His Leu Asp Ala Met Ala Lys Ala Gly Pro Pro Val Asp
   130 135 140
   Leu Val Gln Ala Phe Ala Leu Pro Val Pro Ser Leu Val Ile Cys Glu
   145 150 155 160
   Leu Leu Gly Val Ser Tyr Ala Asp His Ala Phe Phe Gln Glu Gln Thr
   165 170 175
   Thr Ile Met Val Ser Val Asp Lys Thr Gln Asp Glu Val Thr Thr Ala
   180 185 190
   Leu Gly Lys Leu Thr Arg Tyr Ile Ala Glu Leu Val Ala Thr Lys Arg
   195 200 205
   Leu Ser Pro Lys Asp Asp Leu Leu Gly Ser Leu Ile Thr Asp Thr Asp
   210 215 220
   Leu Thr Asp Glu Glu Leu Thr Asn Ile Ala Leu Ile Leu Leu Val Ala
   225 230 235 240
   Gly His Glu Thr Thr Ala Asn Met Leu Gly Leu Gly Thr Phe Ala Leu
   245 250 255
   Leu Gln His Pro Glu Gln Ile Ala Asn Leu Asp Ser Pro Asp Ala Val
   260 265 270
   Glu Glu Leu Leu Arg Tyr Leu Ser Ile Val His Leu Gly Thr Pro Asn
   275 280 285
   Arg Ala Ala Leu Glu Asp Val Glu Leu Glu Gly Gln Met Ile Arg Lys
   290 295 300
   Gly Asp Thr Val Ala Ile Gly Leu Pro Ala Val Asn Arg Asp Pro Lys
   305 310 315 320
   Val Phe Asp Glu Pro Asp Ile Leu Gln Leu Asp Arg Val Asp Ala Arg
   325 330 335
   Lys His Ala Ala Phe Gly Gly Gly Ile His Gln Cys Leu Gly Gln Gln
               340                 345                 350
   Leu Ala Arg Val Glu Met Arg Ile Gly Phe Thr Arg Leu Phe Ala Arg
           355                 360                 365
   Phe Pro Ser Leu Arg Leu Ala Val Pro Ala Glu Glu Ile Lys Leu Arg
       370                 375                 380
   Glu Lys Ser Ala Ala Tyr Gly Val Trp Ala Leu Pro Val Ala Trp Asp
   385                 390                 395                 400
   Ala
<210> 24
   <211> 401
   <212> PRT
   <213> Artificial
<220>
   <223> Synthetic protein
<400> 24
   Met Arg Val Asp Ser Glu Asn Met Asn Glu Pro Val Thr Leu Pro Thr
   1               5                   10                  15
   Gly Arg Ala Val Gly Tyr Pro Phe Asp Pro Pro Pro Asp Leu Val Lys
               20                  25                  30
   Leu Pro Pro Val Ser Pro Met Arg Phe Pro Asp Gly His Ile Gly Trp
           35                  40                  45
   Leu Val Thr Ser His Ala Ala Ala Arg Thr Val Met Ile Asp Pro Arg
       50                  55                  60
   Phe Ser Asn Arg Pro Glu His Lys His Pro Val Phe Ser Val Ile Pro
   65                  70                  75                  80
   Arg Pro Gly Gly Ala Thr Lys Ala Pro Ala Pro Gly Trp Phe Ile Asn
                   85                  90                  95
   Met Asp Ala Pro Glu His Thr Arg Tyr Arg Arg Met Leu Ile Ser Gln
   100 105 110
   Phe Thr Val Arg Arg Ile Lys Glu Leu Glu Pro Arg Ile Val Gln Ile
   115 120 125
   Thr Glu Asp His Leu Asp Ala Met Ala Lys Ala Gly Pro Pro Val Asp
   130 135 140
   Leu Val Gln Ala Phe Ala Leu Pro Val Pro Ser Leu Val Ile Cys Glu
   145 150 155 160
   Leu Leu Gly Val Ser Tyr Ala Asp His Ala Phe Phe Gln Glu Gln Thr
   165 170 175
   Thr Ile Met Leu Ser Val Asp Lys Thr Gln Asp Lys Val Thr Thr Ala
   180 185 190
   Leu Gly Lys Leu Thr Arg Tyr Ile Ala Glu Leu Val Ala Thr Lys Arg
   195 200 205
   Leu Ser Pro Lys Asp Asp Leu Leu Gly Ser Leu Ile Thr Asp Thr Asp
   210 215 220
   Leu Thr Asp Glu Glu Leu Thr Asn Ile Ala Leu Ile Leu Leu Val Ala
   225 230 235 240
   Gly His Glu Thr Thr Ala Asn Met Leu Gly Leu Gly Thr Phe Ala Leu
   245 250 255
   Leu Gln His Pro Glu Gln Ile Ala Val Leu Asp Ser Pro Asp Ala Val
   260 265 270
   Glu Glu Leu Leu Arg Tyr Leu Ser Ile Val His Leu Gly Thr Pro Asn
   275 280 285
   Arg Ala Ala Leu Glu Asp Val Glu Leu Glu Gly Gln Met Ile Arg Lys
   290 295 300
   Gly Asp Thr Val Ala Ile Gly Leu Pro Ala Val Asn Arg Asp Pro Lys
   305 310 315 320
   Val Phe Asp Glu Pro Asp Ile Leu Gln Leu Asp Arg Val Asp Ala Arg
   325 330 335
   Lys His Ala Ala Phe Gly Gly Gly Ile His Gln Cys Leu Gly Gln Gln
               340                 345                 350
   Leu Ala Arg Val Glu Met Arg Ile Gly Phe Thr Arg Leu Phe Ala Arg
           355                 360                 365
   Phe Pro Ser Leu Arg Leu Ala Val Pro Ala Glu Glu Ile Lys Leu Arg
       370                 375                 380
   Glu Lys Ser Ala Ala Tyr Gly Val Trp Ala Leu Pro Val Ala Trp Asp
   385                 390                 395                 400
   Ala
<210> 25
   <211> 401
   <212> PRT
   <213> Artificial
<220>
   <223> Synthetic protein
<400> 25
   Met Arg Val Asp Ser Glu Asn Met Asn Glu Pro Val Thr Leu Pro Thr
   1               5                   10                  15
   Gly Arg Ala Val Gly Tyr Pro Phe Asp Pro Pro Pro Asp Leu Val Lys
               20                  25                  30
   Leu Pro Pro Val Ser Pro Met Arg Phe Pro Asp Gly His Ile Gly Trp
           35                  40                  45
   Leu Val Thr Ser His Ala Ala Ala Arg Thr Val Met Ile Asp Pro Arg
       50                  55                  60
   Phe Ser Asn Arg Pro Glu His Lys His Pro Val Phe Ser Val Ile Pro
   65                  70                  75                  80
   Arg Pro Gly Gly Ala Thr Lys Ala Pro Ala Pro Gly Trp Phe Ile Asn
                   85                  90                  95
   Met Asp Ala Pro Glu His Thr Arg Tyr Arg Arg Met Leu Ile Ser Gln
   100 105 110
   Phe Thr Val Arg Arg Ile Lys Glu Leu Glu Pro Arg Ile Val Gln Ile
   115 120 125
   Thr Glu Asp Arg Leu Asp Ala Met Ala Lys Ala Gly Pro Pro Val Asp
   130 135 140
   Leu Val Gln Ala Phe Ala Leu Pro Val Pro Ser Leu Val Ile Cys Glu
   145 150 155 160
   Leu Leu Gly Val Ser Tyr Ala Asp His Ala Phe Phe Gln Glu Gln Thr
   165 170 175
   Thr Ile Met Leu Ser Val Asp Lys Thr Gln Asp Glu Val Thr Thr Ala
   180 185 190
   Leu Gly Lys Leu Thr Arg Tyr Ile Ala Glu Leu Val Ala Thr Lys Arg
   195 200 205
   Leu Ser Pro Lys Asp Asp Leu Leu Gly Ser Leu Ile Thr Asp Thr Asp
   210 215 220
   Leu Thr Asp Glu Glu Leu Thr Asn Ile Ala Leu Ile Leu Leu Val Ala
   225 230 235 240
   Gly His Glu Thr Thr Ala Asn Met Leu Gly Leu Gly Thr Phe Ala Leu
   245 250 255
   Leu Gln His Pro Glu Gln Ile Ala Val Leu Asp Ser Pro Asp Ala Val
   260 265 270
   Glu Glu Leu Leu Arg Tyr Leu Ser Ile Val His Leu Gly Thr Pro Asn
   275 280 285
   Arg Ala Ala Leu Glu Asp Met Glu Leu Glu Gly Gln Met Ile Arg Lys
   290 295 300
   Gly Asp Thr Val Ala Ile Gly Leu Pro Ala Val Asn Arg Asp Pro Lys
   305 310 315 320
   Val Phe Asp Glu Pro Asp Ile Leu Gln Leu Asp Arg Val Asp Ala Arg
   325 330 335
   Lys His Ala Ala Phe Gly Gly Gly Ile His Gln Cys Leu Gly Gln Gln
               340                 345                 350
   Leu Ala Arg Val Glu Met Arg Ile Gly Leu Thr Arg Leu Phe Ala Arg
           355                 360                 365
   Phe Pro Ser Leu Arg Leu Ala Val Pro Ala Glu Glu Ile Lys Leu Arg
       370                 375                 380
   Glu Lys Ser Ala Ala Tyr Gly Val Trp Ala Leu Pro Val Ala Trp Asp
   385                 390                 395                 400
   Ala
<210> 26
   <211> 401
   <212> PRT
   <213> Artificial
<220>
   <223> Synthetic protein
<400> 26
   Met Arg Val Asp Ser Glu Asn Met Asn Glu Pro Val Thr Leu Pro Thr
   1               5                   10                  15
   Gly Arg Ala Val Gly Tyr Pro Leu Asp Pro Pro Pro Asp Leu Val Lys
               20                  25                  30
   Leu Pro Pro Val Ser Pro Met Arg Phe Pro Asp Gly His Ile Gly Trp
           35                  40                  45
   Leu Val Thr Ser His Ala Ala Ala Arg Thr Val Met Ile Asp Pro Arg
       50                  55                  60
   Phe Ser Asn Arg Pro Glu His Lys His Pro Val Phe Ser Val Ile Pro
   65                  70                  75                  80
   Arg Pro Gly Gly Ala Thr Lys Ala Pro Ala Pro Gly Trp Phe Ile Asn
                   85                  90                  95
   Met Asp Ala Pro Glu His Thr Arg Tyr Arg Arg Met Leu Ile Ser Gln
   100 105 110
   Phe Thr Val Arg Arg Ile Lys Glu Leu Glu Pro Arg Ile Val Gln Ile
   115 120 125
   Thr Glu Asp His Leu Asp Ala Met Ala Lys Ala Gly Pro Pro Val Asp
   130 135 140
   Leu Val Gln Ala Phe Ala Leu Pro Val Pro Ser Leu Val Ile Cys Glu
   145 150 155 160
   Leu Leu Gly Val Ser Tyr Ala Asp His Ala Phe Phe Gln Glu Gln Thr
   165 170 175
   Thr Ile Met Leu Ser Val Asp Lys Thr Gln Asp Glu Val Thr Thr Ala
   180 185 190
   Leu Gly Lys Leu Thr Arg Tyr Ile Ala Glu Leu Val Ala Thr Lys Arg
   195 200 205
   Leu Ser Pro Lys Asp Asp Leu Leu Gly Ser Leu Ile Thr Asp Thr Asp
   210 215 220
   Leu Thr Asp Glu Glu Leu Thr Asn Ile Ala Leu Ile Leu Leu Val Ala
   225 230 235 240
   Gly His Glu Thr Thr Ala Asn Met Leu Gly Leu Gly Thr Phe Ala Leu
   245 250 255
   Leu Gln His Pro Glu Gln Ile Ala Cys Leu Asp Ser Pro Asp Ala Val
   260 265 270
   Glu Glu Leu Leu Arg Tyr Leu Ser Ile Val His Leu Gly Thr Pro Asn
   275 280 285
   Arg Ala Ala Leu Glu Asp Val Glu Leu Glu Gly Gln Met Ile Arg Lys
   290 295 300
   Gly Asp Thr Val Ala Ile Gly Leu Pro Ala Val Asn Arg Asp Pro Lys
   305 310 315 320
   Val Phe Asp Glu Pro Asp Ile Leu Gln Leu Asp Arg Val Asp Ala Arg
   325 330 335
   Lys His Ala Ala Phe Gly Gly Gly Ile His Gln Cys Leu Gly Gln Gln
   340 345 350
   Leu Ala Arg Val Glu Met Arg Ile Gly Phe Thr Arg Leu Phe Ala Arg
   355 360 365
   Phe Pro Ser Leu Arg Leu Ala Val Pro Ala Glu Glu Ile Lys Leu Arg
   370 375 380
   Glu Lys Ser Ala Ala Tyr Gly Val Trp Ala Leu Pro Val Ala Trp Asp
   385 390 395 400
   Ala
<210> 27
   <211> 2199
   <212> DNA
   <213> Artificial
<220>
   <223> Synthetic DNA
<400> 27
   atgagagtag actccgaaaa tatgaacgag ccagtgacac taccgaccgg ccgagccgtg 60
   ggctacccct tcgacccacc gcctgacctg gtgaaacttc cgccagtgag cccgatgcga 120
   ttccctgacg gccacatcgg ctggctggtg accagccatg ccgcagcgcg gactgtcatg 180
   atcgatccgc gcttcagcaa ccgctccgag cacaagcacc cggtgttcag cgtcatccca 240
   cgaccaggcg gagccacgaa agccccggcg cccggctggt tcatcaacat ggacgcaccc 300
   gagcacacgc gttaccgccg gatgctgatc agccagttca ccgtgcgacg gatcaaggaa 360
   ctcgaaccgc ggatcgtcca gatcaccgag gaccacctcg acgcgatggc caaagcagga 420
   ccgccggtcg atctggtgca ggcgttcgcg ttgccagtgc cttcgctggt gatctgcgag 480
   ctgctgggcg tctcgtatgc cgatcacgcg ttcttccaag agcagaccac gatcatgtta 540
   agtgtggaca agacacagga tgaggtgacg acagcgcttg gcaaactcac ccgctacatc 600
   gctgaactgg tcgccacgaa gcggttgagc cccaaggacg acctgcttgg cagcttgatc 660
   acggacaccg atctgaccga tgaagagctg acgaacatcg cgttgatttt gctcgtcgcc 720
   gggcacgaga ccaccgcgaa catgctggga ttaggcactt tcgcgctcct tcagcatccg 780
   gagcagatcg ctgtcctgga cagcccggac gcggtggagg aactgctgcg ctacctctcg     840
   atcgtccacc ttggaacgcc caaccgggcg gcgctggagg acgtggagct cgaaggccag     900
   atgatcagga agggcgacac tgtcgcgatc ggactgcccg cggtcaatag ggacccgaag     960
   gtgttcgacg aacccgatat ccttcaactg gacagagtgg acgcacgcaa gcacgccgcg    1020
   ttcggcggtg gtatacacca atgcctcggc cagcaactgg ccagagtgga gatgcgcatc    1080
   ggcttcaccc ggctgttcgc gaggttcccg tcgctgcggc tcgcggtgcc ggccgaggag    1140
   atcaagctgc gcgagaagtc cgcggcgtac ggggtttggg cgctgcccgt tgcttgggat    1200
   gcctctagtg tgctgcaccg tcaccagcct gtcaccatcg gagaacccgc cgcccgtgcg    1260
   gtgtcccgca ccgtcaccgt cgagcgcctg gaccgtatcg ccgacgacgt gctgcgcctc    1320
   gtcctgcgcg acgccggcgg aaagactctc cccacttgga ctcccggcgc ccacatcgac    1380
   ctcgacctcg gcgcgctgtc gcgccagtac tccctgtgcg gcgcgcccga tgcgcctagc    1440
   tacgagattg ccgtgcacct ggatcccgag agccgcggcg gttcgcgcta catccacgaa    1500
   cagctcgagg tgggaagccc tctccgtatg cgcggccctc gtaaccactt cgcgctcgac    1560
   cccggcgccg agcactacgt gttcgtcgcc ggcggcatcg gcatcacccc tgtcctggcc    1620
   atggccgacc acgcccgcgc ccgtggatgg agctacgaac tgcactactg cggccgtaac    1680
   cgttccggca tggcctacct cgagcgtgtc gccggtcacg gtgaccgtgc cgccctgcac    1740
   gtgtccgagg aaggcacccg tatcgacctc gccgccctcc tcgccgagcc cgcccccggc    1800
   gtccagatct acgcgtgcgg tgccggtcgt ctgctcgccg gactcgagga cgcgagccgt    1860
   aactggcccg acggtgcgct gcacgtcgag cacttcacct cgtccctcgc ggcgctcgat    1920
   cctgacgtcg agcacgcctt cgacctcgaa ctgcgtgact cgggtctgac cgtgcgtgtc    1980
   gaacccaccc agaccgtcct cgacgcgttg cgcgccaaca acatcgacgt gcccagcgac    2040
   tgcgaggaag gcctctgcgg ctcgtgcgag gtcgccgtcc tcgacggcga ggtcgaccac    2100
   cgcgacactg tgctgaccaa ggccgagcgt gcggcgaacc gtcagatgat gacctgctgc    2160
   tcgcgtgcct gcggcgaccg tctggccctg cgtctctaa                           2199
<210> 28
   <211> 2199
   <212> DNA
   <213> Artificial
<220>
   <223> Synthetic DNA
<400> 28
   atgagagtag actccgaaaa tatgaacgag ccagtgacac taccgaccgg ccgagccgtg     60
   ggctacccct tcgacccacc gcctgacctg gtgaaacttc cgccagtgag cccgatgcga    120
   ttccctgacg gccacatcgg ctggctggtg accagccatg ccgcagcgcg gaccgtcatg    180
   atcgatccgc gcttcagcaa ccgccccgag cacaagcacc cggtgttcag cgtcatccca    240
   cgaccaggcg gagccacgaa agccccggcg cccggctggt tctttaacat ggacgcaccc    300
   gagcacacgc gttaccgccg gatgctgatc agccagttca ccgtgcgacg gatcaaggaa    360
   ctcgaaccgc ggatcgtcca gatcaccgag gaccacctcg acgcgatggc caaagcagga    420
   ccgccggtcg atctggtgca ggcgttcgcg ttgccagtgc cttcgctggt gatctgcgag    480
   ctgctgggcg tctcgtatgc cgatcacgcg ttcttccaag agcagaccac gatcatggtc    540
   agtgtggaca agacacagga tgaggtgacg acggcgcttg gcaaactcac ccgctacatc    600
   gctgaactgg tcgccacgaa gcggttgagc cccaaggacg acctgcttgg cagcttgatc    660
   acggacaccg atctgaccga tgaagagctg acgaacatcg cgttgctttt gctcgtcgcc    720
   gggcacgaga ccaccgcgaa catgttggga ttaggcactt tcgcgctcct tcagcatccg    780
   gagcagatcg ctgccctgga cagcccggac gcggtggagg aactgctgcg ctacctctcg    840
   atcgtccacc ttggaacgcc caaccgggcg gcgctggagg acgtggagct cgaaggccag    900
   atgatcagga agggcgacac tgtcgcgatc ggactgcccg cggtcaatag ggacccgaag    960
   gtgttcgacg aacccgatat ccttcaactg gacagagtgg acgcacgcaa gcacgccgcg   1020
   ttcggcggtg gtatacacca atgcctcggc cagcaactgg ccagagtgga gatgcgcatc   1080
   ggcttcaccc ggctgttcgc gaggttcccg tcgctgcggc tcgcggtgcc ggccgaggag   1140
   atcaagctgc gcgagaagtc cgcggcgtac ggggtttggg cgctgcccgt tgcttgggat   1200
   gcctctagtg tgctgcaccg tcaccagcct gtcaccatcg gagaacccgc cgcccgtgcg   1260
   gtgtcccgca ccgtcaccgt cgagcgcctg gaccgtatcg ccgacgacgt gctgcgcctc   1320
   gtcctgcgcg acgccggcgg aaagactctc cccacttgga ctcccggcgc ccacatcgac   1380
   ctcgacctcg gcgcgctgtc gcgccagtac tccctgtgcg gcgcgcccga tgcgcctagc   1440
   tacgagattg ccgtgcacct ggatcccgag agccgcggcg gttcgcgcta catccacgaa   1500
   cagctcgagg tgggaagccc tctccgtatg cgcggccctc gtaaccactt cgcgctcgac   1560
   cccggcgccg agcactacgt gttcgtcgcc ggcggcatcg gcatcacccc tgtcctggcc   1620
   atggccgacc acgcccgcgc ccgtggatgg agctacgaac tgcactactg cggccgtaac   1680
   cgttccggca tggcctacct cgagcgtgtc gccggtcacg gtgaccgtgc cgccctgcac 1740
   gtgtccgagg aaggcacccg tatcgacctc gccgccctcc tcgccgagcc cgcccccggc 1800
   gtccagatct acgcgtgcgg tgccggtcgt ctgctcgccg gactcgagga cgcgagccgt 1860
   aactggcccg acggtgcgct gcacgtcgag cacttcacct cgtccctcgc ggcgctcgat 1920
   cctgacgtcg agcacgcctt cgacctcgaa ctgcgtgact cgggtctgac cgtgcgtgtc 1980
   gaacccaccc agaccgtcct cgacgcgttg cgcgccaaca acatcgacgt gcccagcgac 2040
   tgcgaggaag gcctctgcgg ctcgtgcgag gtcgccgtcc tcgacggcga ggtcgaccac 2100
   cgcgacactg tgctgaccaa ggccgagcgt gcggcgaacc gtcagatgat gacctgctgc 2160
   tcgcgtgcct gcggcgaccg tctggccctg cgtctctaa 2199
<210> 29
   <211> 2199
   <212> DNA
   <213> Artificial
<220>
   <223> Synthetic DNA
<400> 29
   atgagagtag actccgaaaa tatgaacgag ccagtgacac taccgaccgg ccgagccgtg 60
   ggctacccct tcgacccacc gcctgacctg gtgaaacttc cgccagtgag cccgatgcga 120
   ttccctgacg gccacatcgg ctggctggtg accagccatg ccgcagcgcg gactgtcatg 180
   atcgatccgc gcttcagcaa ccgccccgag cacaagcacc cggtgttcag cgtcatccca 240
   cgaccaggcg gagccacgaa agccccggcg cccggctggt tcaccaacat ggacgcaccc 300
   gagcacacgc gttaccgccg gatgctgatc agccagttca ccgtgcgacg gatcaaggaa 360
   ctcgaaccgc ggatcgtcca gatcaccgag gaccacctcg acgcgatggc caaagcagga 420
   ccgccggtcg atctggtgca ggcgttcgcg ttgccagtgc cttcgctggt gatctgcgag 480
   ctgctgggcg tctcgtatgc cgatcacgcg ttcttccaag agcagaccac gatcatggtc 540
   agtgtggaca agacacagga tgaggtgacg acggcgcttg gcaaactcac ccgctacatc 600
   gctgaactgg tcgccacgaa gcggttgagc cccaaggacg acctgcttgg cagcttgatc 660
   acggacaccg atctgaccga tgaagagctg acgaacatcg cgttgatttt gctcgtcgcc 720
   gggcacgaga ccaccgcgaa catgttggga ttaggcactt tcgtgctcct tcagcacccg 780
   gagcagatcg ctcttctgga cagcccggac gcggtggagg aactgctgcg ctacctctcg 840
   atcgtccacc ttggaacgcc caaccgggcg gcgctggagg acgtggagct cgaaggccag 900
   atgatcagga agggcgacac tgtcgcgatc ggactgcccg cggtcaatag ggacccgaag     960
   gtgttcgacg aacccgatat ccttcaactg gacagagtgg acgcacgcaa gcacgccgcg    1020
   ttcggcggtg gtatacacca atgcctcggc cagcaactgg ccagagtgga gatgcgcatc    1080
   ggcttcaccc ggctgttcgc gaggttcccg tcgctgcggc tcgcggtgcc ggccgaggag    1140
   atcaagctgc gcgagaagtc cgcggcgtac ggggtttggg cgctgcccgt tgcttgggat    1200
   gcctctagtg tgctgcaccg tcaccagcct gtcaccatcg gagaacccgc cgcccgtgcg    1260
   gtgtcccgca ccgtcaccgt cgagcgcctg gaccgtatcg ccgacgacgt gctgcgcctc    1320
   gtcctgcgcg acgccggcgg aaagactctc cccacttgga ctcccggcgc ccacatcgac    1380
   ctcgacctcg gcgcgctgtc gcgccagtac tccctgtgcg gcgcgcccga tgcgcctagc    1440
   tacgagattg ccgtgcacct ggatcccgag agccgcggcg gttcgcgcta catccacgaa    1500
   cagctcgagg tgggaagccc tctccgtatg cgcggccctc gtaaccactt cgcgctcgac    1560
   cccggcgccg agcactacgt gttcgtcgcc ggcggcatcg gcatcacccc tgtcctggcc    1620
   atggccgacc acgcccgcgc ccgtggatgg agctacgaac tgcactactg cggccgtaac    1680
   cgttccggca tggcctacct cgagcgtgtc gccggtcacg gtgaccgtgc cgccctgcac    1740
   gtgtccgagg aaggcacccg tatcgacctc gccgccctcc tcgccgagcc cgcccccggc    1800
   gtccagatct acgcgtgcgg tgccggtcgt ctgctcgccg gactcgagga cgcgagccgt    1860
   aactggcccg acggtgcgct gcacgtcgag cacttcacct cgtccctcgc ggcgctcgat    1920
   cctgacgtcg agcacgcctt cgacctcgaa ctgcgtgact cgggtctgac cgtgcgtgtc    1980
   gaacccaccc agaccgtcct cgacgcgttg cgcgccaaca acatcgacgt gcccagcgac    2040
   tgcgaggaag gcctctgcgg ctcgtgcgag gtcgccgtcc tcgacggcga ggtcgaccac    2100
   cgcgacactg tgctgaccaa ggccgagcgt gcggcgaacc gtcagatgat gacctgctgc    2160
   tcgcgtgcct gcggcgaccg tctggccctg cgtctctaa                           2199
<210> 30
   <211> 2199
   <212> DNA
   <213> Artificial
<220>
   <223> Synthetic DNA
<400> 30
   atgagagtag actccgaaaa tatgaacgag ccagtgacac taccgaccgg ccgagccgtg      60
   ggctacccct tcgacccacc gcctgacctg gtgaaacttc cgccagtgag cccgatgcga 120
   tttcctgacg gccacatcgg ctggctggtg accagccatg ccgcagcgcg gactgtcatg 180
   atcgatccgc gcttcagcaa ccgccccgag cacaagcacc cggtgttcag cgtcatccca 240
   cgaccaggcg gagccacgaa agccccggcg cccggctggt tcgccaacat ggacgcaccc 300
   gagcacacgc gttaccgccg gatgctgatc agccagttca ccgtgcgacg gatcaaggaa 360
   ctcgaaccgc ggatcgtcca gatcaccgag gaccacctcg acgcgatggc caaagcagga 420
   ccgccggtcg atctggtgca ggcgttcgcg ttgccagtgc cttcgctggt gatctgcgag 480
   ctgctgggcg tctcgtatgc tgatcacgcg ttcttccaag aacagaccac gatcatgttt 540
   agtgtggaca agacacagga tgaggtgacg acggcgcttg gcaaactcac ccgctacatc 600
   gctgaactgg tcgccacgaa gcggttgagc cctaaggacg acctgcttgg cagcttgatc 660
   acggacaccg atctgaccga tgaagagctg acgaacaccg cgttgctttt gctcgtcgcc 720
   gggcacgaga ccaccgcgaa catgttggga ttaggcactt tcgcgctcct tcagcatccg 780
   gagcagatcg ctgccctgga cagcccggac gcggtggagg aactgctgcg ctacctctcg 840
   atcgtccacc ttggagcgcc caaccgggcg gcgctggagg acgtggagct cgaaggccag 900
   atgatcagga agggcgacac tgtcgcgatc ggactgcccg cggtcaatag ggacccgaag 960
   gtgttcgacg aacccgatat ccttcaactg gacagagtgg acgcacgcaa gcacgccgcg 1020
   ttcggcggtg gtatacacca atgcctcggc cagcaactgg ccagagtgga gatgcgcatc 1080
   ggcttcaccc ggctgttcgc gaggttcccg tcgctgcggc tcgcggtgcc ggccgaggag 1140
   atcaagctgc gcgagaagtc cgcggcgtac ggggtttggg cgctgcccgt tgcttgggat 1200
   gcctctagtg tgctgcaccg tcaccagcct gtcaccatcg gagaacccgc cgcccgtgcg 1260
   gtgtcccgca ccgtcaccgt cgagcgcctg gaccgtatcg ccgacgacgt gctgcgcctc 1320
   gtcctgcgcg acgccggcgg aaagactctc cccacttgga ctcccggcgc ccacatcgac 1380
   ctcgacctcg gcgcgctgtc gcgccagtac tccctgtgcg gcgcgcccga tgcgcctagc 1440
   tacgagattg ccgtgcacct ggatcccgag agccgcggcg gttcgcgcta catccacgaa 1500
   cagctcgagg tgggaagccc tctccgtatg cgcggccctc gtaaccactt cgcgctcgac 1560
   cccggcgccg agcactacgt gttcgtcgcc ggcggcatcg gcatcacccc tgtcctggcc 1620
   atggccgacc acgcccgcgc ccgtggatgg agctacgaac tgcactactg cggccgtaac 1680
   cgttccggca tggcctacct cgagcgtgtc gccggtcacg gtgaccgtgc cgccctgcac 1740
   gtgtccgagg aaggcacccg tatcgacctc gccgccctcc tcgccgagcc cgcccccggc 1800
   gtccagatct acgcgtgcgg tgccggtcgt ctgctcgccg gactcgagga cgcgagccgt 1860
   aactggcccg acggtgcgct gcacgtcgag cacttcacct cgtccctcgc ggcgctcgat 1920
   cctgacgtcg agcacgcctt cgacctcgaa ctgcgtgact cgggtctgac cgtgcgtgtc 1980
   gaacccaccc agaccgtcct cgacgcgttg cgcgccaaca acatcgacgt gcccagcgac 2040
   tgcgaggaag gcctctgcgg ctcgtgcgag gtcgccgtcc tcgacggcga ggtcgaccac 2100
   cgcgacactg tgctgaccaa ggccgagcgt gcggcgaacc gtcagatgat gacctgctgc 2160
   tcgcgtgcct gcggcgaccg tctggccctg cgtctctaa 2199
<210> 31
   <211> 2199
   <212> DNA
   <213> Artificial
<220>
   <223> Synthetic DNA
<400> 31
   atgagagtag actccgaaaa tatgaacgag ccagtgacac taccgaccgg ccgagccgtg 60
   ggctacccct tcgacccacc gcctgacctg gtgaaacttc cgccagtgag cccgatgcga 120
   ttccctgacg gccacatcgg ctggctggtg accagccatg ccgcagcgcg gactgtcatg 180
   atcgatccgc gcttcagcaa ccgccccgag cacaagcacc cggtgttcag cgtcatccca 240
   cgaccaggcg gagccacgaa agccccggcg cccggctggt tcaccaacat ggacgcaccc 300
   gagcacacgc gttaccgccg gatgctgatc agccagttca ccgtgcgacg gatcaaggaa 360
   ctcgaaccgc ggatcgtccg gatcaccgag gaccacctcg acgcgatggc caaagcagga 420
   ccgccggtcg atctggtgca ggcgttcgcg ttgccagtgc cttcgctggt gatctgcgag 480
   ctgctgggcg tctcgtatgc cgatcacgcg ttcttccaag agcagaccac gatcatggtc 540
   agtgtggaca agacacagga tgaggtgacg acggcgcttg gcaaactcac ccgctacatc 600
   gctgaactgg tcgccacgaa gcggttgagc cccaaggacg acctgcttgg cagcttgatc 660
   acggacaccg atctgaccga tgaagagctg acgaacatcg cgttgatttt gctcgtcgcc 720
   gggcacgaga ccaccgcgaa catgttggga ttaggcactt tcgcgctcct tcagcatccg 780
   gagcagatcg ctaatctgga cagcccggac gcggtggagg aactgctgcg ctacctctcg 840
   atcgtccacc ttggaacgcc caaccgggcg gcgctggagg acgtggagct cgaaggccag 900
   atgatcagga agggcgacac tgtcgcgatc ggactgcccg cggtcaatag ggacccgaag 960
   gtgttcgacg aacccgatat ccttcaactg gacagagtgg acgcacgcaa gcacgccgcg 1020
   ttcggcggtg gtatacacca atgcctcggc cagcaactgg ccagagtgga gatgcgcatc 1080
   ggcttcaccc ggctgttcgc gaggttcccg tcgctgcggc tcgcggtgcc ggccgaggag 1140
   atcaagctgc gcgagaagtc cgcggcgtac ggggtttggg cgctgcccgt tgcttgggat 1200
   gcctctagtg tgctgcaccg tcaccagcct gtcaccatcg gagaacccgc cgcccgtgcg 1260
   gtgtcccgca ccgtcaccgt cgagcgcctg gaccgtatcg ccgacgacgt gctgcgcctc 1320
   gtcctgcgcg acgccggcgg aaagactctc cccacttgga ctcccggcgc ccacatcgac 1380
   ctcgacctcg gcgcgctgtc gcgccagtac tccctgtgcg gcgcgcccga tgcgcctagc 1440
   tacgagattg ccgtgcacct ggatcccgag agccgcggcg gttcgcgcta catccacgaa 1500
   cagctcgagg tgggaagccc tctccgtatg cgcggccctc gtaaccactt cgcgctcgac 1560
   cccggcgccg agcactacgt gttcgtcgcc ggcggcatcg gcatcacccc tgtcctggcc 1620
   atggccgacc acgcccgcgc ccgtggatgg agctacgaac tgcactactg cggccgtaac 1680
   cgttccggca tggcctacct cgagcgtgtc gccggtcacg gtgaccgtgc cgccctgcac 1740
   gtgtccgagg aaggcacccg tatcgacctc gccgccctcc tcgccgagcc cgcccccggc 1800
   gtccagatct acgcgtgcgg tgccggtcgt ctgctcgccg gactcgagga cgcgagccgt 1860
   aactggcccg acggtgcgct gcacgtcgag cacttcacct cgtccctcgc ggcgctcgat 1920
   cctgacgtcg agcacgcctt cgacctcgaa ctgcgtgact cgggtctgac cgtgcgtgtc 1980
   gaacccaccc agaccgtcct cgacgcgttg cgcgccaaca acatcgacgt gcccagcgac 2040
   tgcgaggaag gcctctgcgg ctcgtgcgag gtcgccgtcc tcgacggcga ggtcgaccac 2100
   cgcgacactg tgctgaccaa ggccgagcgt gcggcgaacc gtcagatgat gacctgctgc 2160
   tcgcgtgcct gcggcgaccg tctggccctg cgtctctaa 2199
<210> 32
   <211> 2199
   <212> DNA
   <213> Artificial
<220>
   <223> Synthetic DNA
<400> 32 <210> 33
   <211> 2199
   <212> DNA
   <213> Artificial
<220>
   <223> Synthetic DNA
<400> 33
<210> 34
   <211> 2199
   <212> DNA
   <213> Artificial
<220>
   <223> Synthetic DNA
<400> 34
   atgagagtag actccgaaaa tatgaacgag ccagtgacac taccgaccgg ccgagccgtg 60
   ggctaccccc tcgacccacc gcctgacctg gtgaaacttc cgccagtgag cccgatgcga 120
   ttccctgacg gccacatcgg ctggctggtg accagccatg ccgcagcgcg gactgtcatg 180
   atcgatccgc gcttcagcaa ccgccccgag cacaagcacc cggtgttcag cgtcatccca 240
   cgaccaggcg gagccacgaa agccccggcg cccggctggt tcatcaacat ggacgcaccc     300
   gagcacacgc gttaccgccg gatgctgatc agccagttca ccgtgcgacg gatcaaggaa     360
   ctcgaaccgc ggatcgtcca gatcaccgag gaccacctcg acgcgatggc caaagcagga     420
   ccgccggtcg atctggtgca ggcgttcgcg ttgccagtgc cttcgctggt gatctgcgag     480
   ctgctgggcg tctcgtatgc cgatcacgcg ttcttccaag agcagaccac gatcatgttg     540
   agtgtggaca agacacagga tgaggtgacg acggcgcttg gcaaactcac ccgctacatc     600
   gctgaactgg tcgccacgaa gcggttgagc cccaaggacg acctgcttgg cagcttgatc     660
   acggacaccg atctgaccga tgaagagctg acgaacatcg cgttgatttt gctcgtcgcc     720
   gggcacgaga ccaccgcgaa catgttggga ttaggcactt tcgcgctcct tcagcatccg     780
   gagcagatcg cttgcctgga cagcccggac gcggtggagg aactgctgcg ctacctctcg     840
   atcgtccacc ttggaacgcc caaccgggcg gcgctggagg acgtggagct cgaaggccag     900
   atgatcagga agggcgacac tgtcgcgatc ggactgcccg cggtcaatag ggacccgaag     960
   gtgttcgacg aacccgatat ccttcaactg gacagagtgg acgcacgcaa gcacgccgcg    1020
   ttcggcggtg gtatacacca atgcctcggc cagcaactgg ccagagtgga gatgcgcatc    1080
   ggcttcaccc ggctgttcgc gaggttcccg tcgctgcggc tcgcggtgcc ggccgaggag    1140
   atcaagctgc gcgagaagtc cgcggcgtac ggggtttggg cgctgcccgt tgcttgggat    1200
   gcctctagtg tgctgcaccg tcaccagcct gtcaccatcg gagaacccgc cgcccgtgcg    1260
   gtgtcccgca ccgtcaccgt cgagcgcctg gaccgtatcg ccgacgacgt gctgcgcctc    1320
   gtcctgcgcg acgccggcgg aaagactctc cccacttgga ctcccggcgc ccacatcgac    1380
   ctcgacctcg gcgcgctgtc gcgccagtac tccctgtgcg gcgcgcccga tgcgcctagc    1440
   tacgagattg ccgtgcacct ggatcccgag agccgcggcg gttcgcgcta catccacgaa    1500
   cagctcgagg tgggaagccc tctccgtatg cgcggccctc gtaaccactt cgcgctcgac    1560
   cccggcgccg agcactacgt gttcgtcgcc ggcggcatcg gcatcacccc tgtcctggcc    1620
   atggccgacc acgcccgcgc ccgtggatgg agctacgaac tgcactactg cggccgtaac    1680
   cgttccggca tggcctacct cgagcgtgtc gccggtcacg gtgaccgtgc cgccctgcac    1740
   gtgtccgagg aaggcacccg tatcgacctc gccgccctcc tcgccgagcc cgcccccggc    1800
   gtccagatct acgcgtgcgg tgccggtcgt ctgctcgccg gactcgagga cgcgagccgt    1860
   aactggcccg acggtgcgct gcacgtcgag cacttcacct cgtccctcgc ggcgctcgat    1920
   cctgacgtcg agcacgcctt cgacctcgaa ctgcgtgact cgggtctgac cgtgcgtgtc    1980
   gaacccaccc agaccgtcct cgacgcgttg cgcgccaaca acatcgacgt gcccagcgac 2040
   tgcgaggaag gcctctgcgg ctcgtgcgag gtcgccgtcc tcgacggcga ggtcgaccac 2100
   cgcgacactg tgctgaccaa ggccgagcgt gcggcgaacc gtcagatgat gacctgctgc 2160
   tcgcgtgcct gcggcgaccg tctggccctg cgtctctaa 2199
<210> 35
   <211> 732
   <212> PRT
   <213> Artificial
<220>
   <223> Synthetic protein
<400> 35
   Met Arg Val Asp Ser Glu Asn Met Asn Glu Pro Val Thr Leu Pro Thr
   1 5 10 15
   Gly Arg Ala Val Gly Tyr Pro Phe Asp Pro Pro Pro Asp Leu Val Lys
   20 25 30
   Leu Pro Pro Val Ser Pro Met Arg Phe Pro Asp Gly His Ile Gly Trp
   35 40 45
   Leu Val Thr Ser His Ala Ala Ala Arg Thr Val Met Ile Asp Pro Arg
   50 55 60
   Phe Ser Asn Arg Ser Glu His Lys His Pro Val Phe Ser Val Ile Pro
   65 70 75 80
   Arg Pro Gly Gly Ala Thr Lys Ala Pro Ala Pro Gly Trp Phe Ile Asn
   85 90 95
   Met Asp Ala Pro Glu His Thr Arg Tyr Arg Arg Met Leu Ile Ser Gln
   100 105 110
   Phe Thr Val Arg Arg Ile Lys Glu Leu Glu Pro Arg Ile Val Gln Ile
   115 120 125
   Thr Glu Asp His Leu Asp Ala Met Ala Lys Ala Gly Pro Pro Val Asp
   130 135 140
   Leu Val Gln Ala Phe Ala Leu Pro Val Pro Ser Leu Val Ile Cys Glu
   145 150 155 160
   Leu Leu Gly Val Ser Tyr Ala Asp His Ala Phe Phe Gln Glu Gln Thr
   165 170 175
   Thr Ile Met Leu Ser Val Asp Lys Thr Gln Asp Glu Val Thr Thr Ala
   180 185 190
   Leu Gly Lys Leu Thr Arg Tyr Ile Ala Glu Leu Val Ala Thr Lys Arg
   195 200 205
   Leu Ser Pro Lys Asp Asp Leu Leu Gly Ser Leu Ile Thr Asp Thr Asp
   210 215 220
   Leu Thr Asp Glu Glu Leu Thr Asn Ile Ala Leu Ile Leu Leu Val Ala
   225 230 235 240
   Gly His Glu Thr Thr Ala Asn Met Leu Gly Leu Gly Thr Phe Ala Leu
   245 250 255
   Leu Gln His Pro Glu Gln Ile Ala Val Leu Asp Ser Pro Asp Ala Val
   260 265 270
   Glu Glu Leu Leu Arg Tyr Leu Ser Ile Val His Leu Gly Thr Pro Asn
   275 280 285
   Arg Ala Ala Leu Glu Asp Val Glu Leu Glu Gly Gln Met Ile Arg Lys
   290 295 300
   Gly Asp Thr Val Ala Ile Gly Leu Pro Ala Val Asn Arg Asp Pro Lys
   305 310 315 320
   Val Phe Asp Glu Pro Asp Ile Leu Gln Leu Asp Arg Val Asp Ala Arg
   325 330 335
   Lys His Ala Ala Phe Gly Gly Gly Ile His Gln Cys Leu Gly Gln Gln
   340 345 350
   Leu Ala Arg Val Glu Met Arg Ile Gly Phe Thr Arg Leu Phe Ala Arg
   355 360 365
   Phe Pro Ser Leu Arg Leu Ala Val Pro Ala Glu Glu Ile Lys Leu Arg
   370 375 380
   Glu Lys Ser Ala Ala Tyr Gly Val Trp Ala Leu Pro Val Ala Trp Asp
   385 390 395 400
   Ala Ser Ser Val Leu His Arg His Gln Pro Val Thr Ile Gly Glu Pro
   405 410 415
   Ala Ala Arg Ala Val Ser Arg Thr Val Thr Val Glu Arg Leu Asp Arg
   420 425 430
   Ile Ala Asp Asp Val Leu Arg Leu Val Leu Arg Asp Ala Gly Gly Lys
   435 440 445
   Thr Leu Pro Thr Trp Thr Pro Gly Ala His Ile Asp Leu Asp Leu Gly
   450 455 460
   Ala Leu Ser Arg Gln Tyr Ser Leu Cys Gly Ala Pro Asp Ala Pro Ser
   465 470 475 480
   Tyr Glu Ile Ala Val His Leu Asp Pro Glu Ser Arg Gly Gly Ser Arg
   485 490 495
   Tyr Ile His Glu Gln Leu Glu Val Gly Ser Pro Leu Arg Met Arg Gly
   500 505 510
   Pro Arg Asn His Phe Ala Leu Asp Pro Gly Ala Glu His Tyr Val Phe
   515 520 525
   Val Ala Gly Gly Ile Gly Ile Thr Pro Val Leu Ala Met Ala Asp His
   530 535 540
   Ala Arg Ala Arg Gly Trp Ser Tyr Glu Leu His Tyr Cys Gly Arg Asn
   545 550 555 560
   Arg Ser Gly Met Ala Tyr Leu Glu Arg Val Ala Gly His Gly Asp Arg
   565 570 575
   Ala Ala Leu His Val Ser Glu Glu Gly Thr Arg Ile Asp Leu Ala Ala
   580 585 590
   Leu Leu Ala Glu Pro Ala Pro Gly Val Gln Ile Tyr Ala Cys Gly Ala
   595 600 605
   Gly Arg Leu Leu Ala Gly Leu Glu Asp Ala Ser Arg Asn Trp Pro Asp
   610 615 620
   Gly Ala Leu His Val Glu His Phe Thr Ser Ser Leu Ala Ala Leu Asp
   625 630 635 640
   Pro Asp Val Glu His Ala Phe Asp Leu Glu Leu Arg Asp Ser Gly Leu
   645 650 655
   Thr Val Arg Val Glu Pro Thr Gln Thr Val Leu Asp Ala Leu Arg Ala
   660 665 670
   Asn Asn Ile Asp Val Pro Ser Asp Cys Glu Glu Gly Leu Cys Gly Ser
   675 680 685
   Cys Glu Val Ala Val Leu Asp Gly Glu Val Asp His Arg Asp Thr Val
   690 695 700
   Leu Thr Lys Ala Glu Arg Ala Ala Asn Arg Gln Met Met Thr Cys Cys
   705 710 715 720
   Ser Arg Ala Cys Gly Asp Arg Leu Ala Leu Arg Leu
   725 730
<210> 36
   <211> 732
   <212> PRT
   <213> Artificial
<220>
   <223> Synthetic protein
<400> 36
   Met Arg Val Asp Ser Glu Asn Met Asn Glu Pro Val Thr Leu Pro Thr
   1 5 10 15
   Gly Arg Ala Val Gly Tyr Pro Phe Asp Pro Pro Pro Asp Leu Val Lys
   20 25 30
   Leu Pro Pro Val Ser Pro Met Arg Phe Pro Asp Gly His Ile Gly Trp
   35 40 45
   Leu Val Thr Ser His Ala Ala Ala Arg Thr Val Met Ile Asp Pro Arg
   50 55 60
   Phe Ser Asn Arg Pro Glu His Lys His Pro Val Phe Ser Val Ile Pro
   65                  70                  75                  80
   Arg Pro Gly Gly Ala Thr Lys Ala Pro Ala Pro Gly Trp Phe Phe Asn
                   85                  90                  95
   Met Asp Ala Pro Glu His Thr Arg Tyr Arg Arg Met Leu Ile Ser Gln
               100                 105                 110
   Phe Thr Val Arg Arg Ile Lys Glu Leu Glu Pro Arg Ile Val Gln Ile
           115                 120                 125
   Thr Glu Asp His Leu Asp Ala Met Ala Lys Ala Gly Pro Pro Val Asp
       130                 135                 140
   Leu Val Gln Ala Phe Ala Leu Pro Val Pro Ser Leu Val Ile Cys Glu
   145                 150                 155                 160
   Leu Leu Gly Val Ser Tyr Ala Asp His Ala Phe Phe Gln Glu Gln Thr
                   165                 170                 175
   Thr Ile Met Val Ser Val Asp Lys Thr Gln Asp Glu Val Thr Thr Ala
               180                 185                 190
   Leu Gly Lys Leu Thr Arg Tyr Ile Ala Glu Leu Val Ala Thr Lys Arg
           195                 200                 205
   Leu Ser Pro Lys Asp Asp Leu Leu Gly Ser Leu Ile Thr Asp Thr Asp
       210                 215                 220
   Leu Thr Asp Glu Glu Leu Thr Asn Ile Ala Leu Leu Leu Leu Val Ala
   225                 230                 235                 240
   Gly His Glu Thr Thr Ala Asn Met Leu Gly Leu Gly Thr Phe Ala Leu
                   245                 250                 255
   Leu Gln His Pro Glu Gln Ile Ala Ala Leu Asp Ser Pro Asp Ala Val
               260                 265                 270
   Glu Glu Leu Leu Arg Tyr Leu Ser Ile Val His Leu Gly Thr Pro Asn
           275                 280                 285
   Arg Ala Ala Leu Glu Asp Val Glu Leu Glu Gly Gln Met Ile Arg Lys
       290                 295                 300
   Gly Asp Thr Val Ala Ile Gly Leu Pro Ala Val Asn Arg Asp Pro Lys
   305                 310                 315                 320
   Val Phe Asp Glu Pro Asp Ile Leu Gln Leu Asp Arg Val Asp Ala Arg
                   325                 330                 335
   Lys His Ala Ala Phe Gly Gly Gly Ile His Gln Cys Leu Gly Gln Gln
               340                 345                 350
   Leu Ala Arg Val Glu Met Arg Ile Gly Phe Thr Arg Leu Phe Ala Arg
           355                 360                 365
   Phe Pro Ser Leu Arg Leu Ala Val Pro Ala Glu Glu Ile Lys Leu Arg
       370                 375                 380
   Glu Lys Ser Ala Ala Tyr Gly Val Trp Ala Leu Pro Val Ala Trp Asp
   385                 390                 395                 400
   Ala Ser Ser Val Leu His Arg His Gln Pro Val Thr Ile Gly Glu Pro
                   405                 410                 415
   Ala Ala Arg Ala Val Ser Arg Thr Val Thr Val Glu Arg Leu Asp Arg
               420                 425                 430
   Ile Ala Asp Asp Val Leu Arg Leu Val Leu Arg Asp Ala Gly Gly Lys
           435                 440                 445
   Thr Leu Pro Thr Trp Thr Pro Gly Ala His Ile Asp Leu Asp Leu Gly
       450                 455                 460
   Ala Leu Ser Arg Gln Tyr Ser Leu Cys Gly Ala Pro Asp Ala Pro Ser
   465                 470                 475                 480
   Tyr Glu Ile Ala Val His Leu Asp Pro Glu Ser Arg Gly Gly Ser Arg
                   485                 490                 495
   Tyr Ile His Glu Gln Leu Glu Val Gly Ser Pro Leu Arg Met Arg Gly
               500                 505                 510
   Pro Arg Asn His Phe Ala Leu Asp Pro Gly Ala Glu His Tyr Val Phe
           515                 520                 525
   Val Ala Gly Gly Ile Gly Ile Thr Pro Val Leu Ala Met Ala Asp His
       530                 535                 540
   Ala Arg Ala Arg Gly Trp Ser Tyr Glu Leu His Tyr Cys Gly Arg Asn
   545                 550                 555                 560
   Arg Ser Gly Met Ala Tyr Leu Glu Arg Val Ala Gly His Gly Asp Arg
                   565                 570                 575
   Ala Ala Leu His Val Ser Glu Glu Gly Thr Arg Ile Asp Leu Ala Ala
               580                 585                 590
   Leu Leu Ala Glu Pro Ala Pro Gly Val Gln Ile Tyr Ala Cys Gly Ala
           595                 600                 605
   Gly Arg Leu Leu Ala Gly Leu Glu Asp Ala Ser Arg Asn Trp Pro Asp
       610                 615                 620
   Gly Ala Leu His Val Glu His Phe Thr Ser Ser Leu Ala Ala Leu Asp
   625                 630                 635                 640
   Pro Asp Val Glu His Ala Phe Asp Leu Glu Leu Arg Asp Ser Gly Leu
                   645                 650                 655
   Thr Val Arg Val Glu Pro Thr Gln Thr Val Leu Asp Ala Leu Arg Ala
               660                 665                 670
   Asn Asn Ile Asp Val Pro Ser Asp Cys Glu Glu Gly Leu Cys Gly Ser
           675                 680                 685
   Cys Glu Val Ala Val Leu Asp Gly Glu Val Asp His Arg Asp Thr Val
       690                 695                 700
   Leu Thr Lys Ala Glu Arg Ala Ala Asn Arg Gln Met Met Thr Cys Cys
   705                 710                 715                 720
   Ser Arg Ala Cys Gly Asp Arg Leu Ala Leu Arg Leu
                   725                 730
<210> 37
   <211> 732
   <212> PRT
   <213> Artificial
<220>
   <223> Synthetic protein
<400> 37
   Met Arg Val Asp Ser Glu Asn Met Asn Glu Pro Val Thr Leu Pro Thr
   1               5                   10                  15
   Gly Arg Ala Val Gly Tyr Pro Phe Asp Pro Pro Pro Asp Leu Val Lys
               20                  25                  30
   Leu Pro Pro Val Ser Pro Met Arg Phe Pro Asp Gly His Ile Gly Trp
           35                  40                  45
   Leu Val Thr Ser His Ala Ala Ala Arg Thr Val Met Ile Asp Pro Arg
       50                  55                  60
   Phe Ser Asn Arg Pro Glu His Lys His Pro Val Phe Ser Val Ile Pro
   65                  70                  75                  80
   Arg Pro Gly Gly Ala Thr Lys Ala Pro Ala Pro Gly Trp Phe Thr Asn
                   85                  90                  95
   Met Asp Ala Pro Glu His Thr Arg Tyr Arg Arg Met Leu Ile Ser Gln
               100                 105                 110
   Phe Thr Val Arg Arg Ile Lys Glu Leu Glu Pro Arg Ile Val Gln Ile
           115                 120                 125
   Thr Glu Asp His Leu Asp Ala Met Ala Lys Ala Gly Pro Pro Val Asp
       130                 135                 140
   Leu Val Gln Ala Phe Ala Leu Pro Val Pro Ser Leu Val Ile Cys Glu
   145                 150                 155                 160
   Leu Leu Gly Val Ser Tyr Ala Asp His Ala Phe Phe Gln Glu Gln Thr
                   165                 170                 175
   Thr Ile Met Val Ser Val Asp Lys Thr Gln Asp Glu Val Thr Thr Ala
               180                 185                 190
   Leu Gly Lys Leu Thr Arg Tyr Ile Ala Glu Leu Val Ala Thr Lys Arg
           195                 200                 205
   Leu Ser Pro Lys Asp Asp Leu Leu Gly Ser Leu Ile Thr Asp Thr Asp
       210                 215                 220
   Leu Thr Asp Glu Glu Leu Thr Asn Ile Ala Leu Ile Leu Leu Val Ala
   225                 230                 235                 240
   Gly His Glu Thr Thr Ala Asn Met Leu Gly Leu Gly Thr Phe Val Leu
                   245                 250                 255
   Leu Gln His Pro Glu Gln Ile Ala Leu Leu Asp Ser Pro Asp Ala Val
               260                 265                 270
   Glu Glu Leu Leu Arg Tyr Leu Ser Ile Val His Leu Gly Thr Pro Asn
           275                 280                 285
   Arg Ala Ala Leu Glu Asp Val Glu Leu Glu Gly Gln Met Ile Arg Lys
       290                 295                 300
   Gly Asp Thr Val Ala Ile Gly Leu Pro Ala Val Asn Arg Asp Pro Lys
   305                 310                 315                 320
   Val Phe Asp Glu Pro Asp Ile Leu Gln Leu Asp Arg Val Asp Ala Arg
                   325                 330                 335
   Lys His Ala Ala Phe Gly Gly Gly Ile His Gln Cys Leu Gly Gln Gln
               340                 345                 350
   Leu Ala Arg Val Glu Met Arg Ile Gly Phe Thr Arg Leu Phe Ala Arg
           355                 360                 365
   Phe Pro Ser Leu Arg Leu Ala Val Pro Ala Glu Glu Ile Lys Leu Arg
       370                 375                 380
   Glu Lys Ser Ala Ala Tyr Gly Val Trp Ala Leu Pro Val Ala Trp Asp
   385                 390                 395                 400
   Ala Ser Ser Val Leu His Arg His Gln Pro Val Thr Ile Gly Glu Pro
                   405                 410                 415
   Ala Ala Arg Ala Val Ser Arg Thr Val Thr Val Glu Arg Leu Asp Arg
   420 425 430
   Ile Ala Asp Asp Val Leu Arg Leu Val Leu Arg Asp Ala Gly Gly Lys
   435 440 445
   Thr Leu Pro Thr Trp Thr Pro Gly Ala His Ile Asp Leu Asp Leu Gly
   450 455 460
   Ala Leu Ser Arg Gln Tyr Ser Leu Cys Gly Ala Pro Asp Ala Pro Ser
   465 470 475 480
   Tyr Glu Ile Ala Val His Leu Asp Pro Glu Ser Arg Gly Gly Ser Arg
   485 490 495
   Tyr Ile His Glu Gln Leu Glu Val Gly Ser Pro Leu Arg Met Arg Gly
   500 505 510
   Pro Arg Asn His Phe Ala Leu Asp Pro Gly Ala Glu His Tyr Val Phe
   515 520 525
   Val Ala Gly Gly Ile Gly Ile Thr Pro Val Leu Ala Met Ala Asp His
   530 535 540
   Ala Arg Ala Arg Gly Trp Ser Tyr Glu Leu His Tyr Cys Gly Arg Asn
   545 550 555 560
   Arg Ser Gly Met Ala Tyr Leu Glu Arg Val Ala Gly His Gly Asp Arg
   565 570 575
   Ala Ala Leu His Val Ser Glu Glu Gly Thr Arg Ile Asp Leu Ala Ala
   580 585 590
   Leu Leu Ala Glu Pro Ala Pro Gly Val Gln Ile Tyr Ala Cys Gly Ala
   595 600 605
   Gly Arg Leu Leu Ala Gly Leu Glu Asp Ala Ser Arg Asn Trp Pro Asp
   610 615 620
   Gly Ala Leu His Val Glu His Phe Thr Ser Ser Leu Ala Ala Leu Asp
   625 630 635 640
   Pro Asp Val Glu His Ala Phe Asp Leu Glu Leu Arg Asp Ser Gly Leu
   645 650 655
   Thr Val Arg Val Glu Pro Thr Gln Thr Val Leu Asp Ala Leu Arg Ala
   660 665 670
   Asn Asn Ile Asp Val Pro Ser Asp Cys Glu Glu Gly Leu Cys Gly Ser
   675 680 685
   Cys Glu Val Ala Val Leu Asp Gly Glu Val Asp His Arg Asp Thr Val
   690 695 700
   Leu Thr Lys Ala Glu Arg Ala Ala Asn Arg Gln Met Met Thr Cys Cys
   705 710 715 720
   Ser Arg Ala Cys Gly Asp Arg Leu Ala Leu Arg Leu
   725 730
<210> 38
   <211> 732
   <212> PRT
   <213> Artificial
<220>
   <223> Synthetic protein
<400> 38
   Met Arg Val Asp Ser Glu Asn Met Asn Glu Pro Val Thr Leu Pro Thr
   1 5 10 15
   Gly Arg Ala Val Gly Tyr Pro Phe Asp Pro Pro Pro Asp Leu Val Lys
   20 25 30
   Leu Pro Pro Val Ser Pro Met Arg Phe Pro Asp Gly His Ile Gly Trp
   35 40 45
   Leu Val Thr Ser His Ala Ala Ala Arg Thr Val Met Ile Asp Pro Arg
   50 55 60
   Phe Ser Asn Arg Pro Glu His Lys His Pro Val Phe Ser Val Ile Pro
   65 70 75 80
   Arg Pro Gly Gly Ala Thr Lys Ala Pro Ala Pro Gly Trp Phe Ala Asn
   85 90 95
   Met Asp Ala Pro Glu His Thr Arg Tyr Arg Arg Met Leu Ile Ser Gln
   100 105 110
   Phe Thr Val Arg Arg Ile Lys Glu Leu Glu Pro Arg Ile Val Gln Ile
   115 120 125
   Thr Glu Asp His Leu Asp Ala Met Ala Lys Ala Gly Pro Pro Val Asp
   130 135 140
   Leu Val Gln Ala Phe Ala Leu Pro Val Pro Ser Leu Val Ile Cys Glu
   145 150 155 160
   Leu Leu Gly Val Ser Tyr Ala Asp His Ala Phe Phe Gln Glu Gln Thr
   165 170 175
   Thr Ile Met Phe Ser Val Asp Lys Thr Gln Asp Glu Val Thr Thr Ala
   180 185 190
   Leu Gly Lys Leu Thr Arg Tyr Ile Ala Glu Leu Val Ala Thr Lys Arg
   195 200 205
   Leu Ser Pro Lys Asp Asp Leu Leu Gly Ser Leu Ile Thr Asp Thr Asp
   210 215 220
   Leu Thr Asp Glu Glu Leu Thr Asn Thr Ala Leu Leu Leu Leu Val Ala
   225 230 235 240
   Gly His Glu Thr Thr Ala Asn Met Leu Gly Leu Gly Thr Phe Ala Leu
   245 250 255
   Leu Gln His Pro Glu Gln Ile Ala Ala Leu Asp Ser Pro Asp Ala Val
   260 265 270
   Glu Glu Leu Leu Arg Tyr Leu Ser Ile Val His Leu Gly Ala Pro Asn
   275 280 285
   Arg Ala Ala Leu Glu Asp Val Glu Leu Glu Gly Gln Met Ile Arg Lys
   290 295 300
   Gly Asp Thr Val Ala Ile Gly Leu Pro Ala Val Asn Arg Asp Pro Lys
   305 310 315 320
   Val Phe Asp Glu Pro Asp Ile Leu Gln Leu Asp Arg Val Asp Ala Arg
   325 330 335
   Lys His Ala Ala Phe Gly Gly Gly Ile His Gln Cys Leu Gly Gln Gln
   340 345 350
   Leu Ala Arg Val Glu Met Arg Ile Gly Phe Thr Arg Leu Phe Ala Arg
   355 360 365
   Phe Pro Ser Leu Arg Leu Ala Val Pro Ala Glu Glu Ile Lys Leu Arg
   370 375 380
   Glu Lys Ser Ala Ala Tyr Gly Val Trp Ala Leu Pro Val Ala Trp Asp
   385 390 395 400
   Ala Ser Ser Val Leu His Arg His Gln Pro Val Thr Ile Gly Glu Pro
   405 410 415
   Ala Ala Arg Ala Val Ser Arg Thr Val Thr Val Glu Arg Leu Asp Arg
   420 425 430
   Ile Ala Asp Asp Val Leu Arg Leu Val Leu Arg Asp Ala Gly Gly Lys
   435 440 445
   Thr Leu Pro Thr Trp Thr Pro Gly Ala His Ile Asp Leu Asp Leu Gly
   450 455 460
   Ala Leu Ser Arg Gln Tyr Ser Leu Cys Gly Ala Pro Asp Ala Pro Ser
   465 470 475 480
   Tyr Glu Ile Ala Val His Leu Asp Pro Glu Ser Arg Gly Gly Ser Arg
   485 490 495
   Tyr Ile His Glu Gln Leu Glu Val Gly Ser Pro Leu Arg Met Arg Gly
   500 505 510
   Pro Arg Asn His Phe Ala Leu Asp Pro Gly Ala Glu His Tyr Val Phe
   515 520 525
   Val Ala Gly Gly Ile Gly Ile Thr Pro Val Leu Ala Met Ala Asp His
   530 535 540
   Ala Arg Ala Arg Gly Trp Ser Tyr Glu Leu His Tyr Cys Gly Arg Asn
   545 550 555 560
   Arg Ser Gly Met Ala Tyr Leu Glu Arg Val Ala Gly His Gly Asp Arg
                   565                 570                 575
   Ala Ala Leu His Val Ser Glu Glu Gly Thr Arg Ile Asp Leu Ala Ala
               580                 585                 590
   Leu Leu Ala Glu Pro Ala Pro Gly Val Gln Ile Tyr Ala Cys Gly Ala
           595                 600                 605
   Gly Arg Leu Leu Ala Gly Leu Glu Asp Ala Ser Arg Asn Trp Pro Asp
       610                 615                 620
   Gly Ala Leu His Val Glu His Phe Thr Ser Ser Leu Ala Ala Leu Asp
   625                 630                 635                 640
   Pro Asp Val Glu His Ala Phe Asp Leu Glu Leu Arg Asp Ser Gly Leu
                   645                 650                 655
   Thr Val Arg Val Glu Pro Thr Gln Thr Val Leu Asp Ala Leu Arg Ala
               660                 665                 670
   Asn Asn Ile Asp Val Pro Ser Asp Cys Glu Glu Gly Leu Cys Gly Ser
           675                 680                 685
   Cys Glu Val Ala Val Leu Asp Gly Glu Val Asp His Arg Asp Thr Val
       690                 695                 700
   Leu Thr Lys Ala Glu Arg Ala Ala Asn Arg Gln Met Met Thr Cys Cys
   705                 710                 715                 720
   Ser Arg Ala Cys Gly Asp Arg Leu Ala Leu Arg Leu
                   725                 730
<210> 39
   <211> 732
   <212> PRT
   <213> Artificial
<220>
   <223> Synthetic protein
<400> 39
   Met Arg Val Asp Ser Glu Asn Met Asn Glu Pro Val Thr Leu Pro Thr
   1               5                   10                  15
   Gly Arg Ala Val Gly Tyr Pro Phe Asp Pro Pro Pro Asp Leu Val Lys
               20                  25                  30
   Leu Pro Pro Val Ser Pro Met Arg Phe Pro Asp Gly His Ile Gly Trp
           35                  40                  45
   Leu Val Thr Ser His Ala Ala Ala Arg Thr Val Met Ile Asp Pro Arg
       50                  55                  60
   Phe Ser Asn Arg Pro Glu His Lys His Pro Val Phe Ser Val Ile Pro
   65                  70                  75                  80
   Arg Pro Gly Gly Ala Thr Lys Ala Pro Ala Pro Gly Trp Phe Thr Asn
                   85                  90                  95
   Met Asp Ala Pro Glu His Thr Arg Tyr Arg Arg Met Leu Ile Ser Gln
               100                 105                 110
   Phe Thr Val Arg Arg Ile Lys Glu Leu Glu Pro Arg Ile Val Arg Ile
           115                 120                 125
   Thr Glu Asp His Leu Asp Ala Met Ala Lys Ala Gly Pro Pro Val Asp
       130                 135                 140
   Leu Val Gln Ala Phe Ala Leu Pro Val Pro Ser Leu Val Ile Cys Glu
   145                 150                 155                 160
   Leu Leu Gly Val Ser Tyr Ala Asp His Ala Phe Phe Gln Glu Gln Thr
                   165                 170                 175
   Thr Ile Met Val Ser Val Asp Lys Thr Gln Asp Glu Val Thr Thr Ala
               180                 185                 190
   Leu Gly Lys Leu Thr Arg Tyr Ile Ala Glu Leu Val Ala Thr Lys Arg
           195                 200                 205
   Leu Ser Pro Lys Asp Asp Leu Leu Gly Ser Leu Ile Thr Asp Thr Asp
       210                 215                 220
   Leu Thr Asp Glu Glu Leu Thr Asn Ile Ala Leu Ile Leu Leu Val Ala
   225                 230                 235                 240
   Gly His Glu Thr Thr Ala Asn Met Leu Gly Leu Gly Thr Phe Ala Leu
                   245                 250                 255
   Leu Gln His Pro Glu Gln Ile Ala Asn Leu Asp Ser Pro Asp Ala Val
               260                 265                 270
   Glu Glu Leu Leu Arg Tyr Leu Ser Ile Val His Leu Gly Thr Pro Asn
           275                 280                 285
   Arg Ala Ala Leu Glu Asp Val Glu Leu Glu Gly Gln Met Ile Arg Lys
       290                 295                 300
   Gly Asp Thr Val Ala Ile Gly Leu Pro Ala Val Asn Arg Asp Pro Lys
   305                 310                 315                 320
   Val Phe Asp Glu Pro Asp Ile Leu Gln Leu Asp Arg Val Asp Ala Arg
                   325                 330                 335
   Lys His Ala Ala Phe Gly Gly Gly Ile His Gln Cys Leu Gly Gln Gln
               340                 345                 350
   Leu Ala Arg Val Glu Met Arg Ile Gly Phe Thr Arg Leu Phe Ala Arg
           355                 360                 365
   Phe Pro Ser Leu Arg Leu Ala Val Pro Ala Glu Glu Ile Lys Leu Arg
       370                 375                 380
   Glu Lys Ser Ala Ala Tyr Gly Val Trp Ala Leu Pro Val Ala Trp Asp
   385                 390                 395                 400
   Ala Ser Ser Val Leu His Arg His Gln Pro Val Thr Ile Gly Glu Pro
                   405                 410                 415
   Ala Ala Arg Ala Val Ser Arg Thr Val Thr Val Glu Arg Leu Asp Arg
               420                 425                 430
   Ile Ala Asp Asp Val Leu Arg Leu Val Leu Arg Asp Ala Gly Gly Lys
           435                 440                 445
   Thr Leu Pro Thr Trp Thr Pro Gly Ala His Ile Asp Leu Asp Leu Gly
       450                 455                 460
   Ala Leu Ser Arg Gln Tyr Ser Leu Cys Gly Ala Pro Asp Ala Pro Ser
   465 470 475 480
   Tyr Glu Ile Ala Val His Leu Asp Pro Glu Ser Arg Gly Gly Ser Arg
   485 490 495
   Tyr Ile His Glu Gln Leu Glu Val Gly Ser Pro Leu Arg Met Arg Gly
   500 505 510
   Pro Arg Asn His Phe Ala Leu Asp Pro Gly Ala Glu His Tyr Val Phe
   515 520 525
   Val Ala Gly Gly Ile Gly Ile Thr Pro Val Leu Ala Met Ala Asp His
   530 535 540
   Ala Arg Ala Arg Gly Trp Ser Tyr Glu Leu His Tyr Cys Gly Arg Asn
   545 550 555 560
   Arg Ser Gly Met Ala Tyr Leu Glu Arg Val Ala Gly His Gly Asp Arg
   565 570 575
   Ala Ala Leu His Val Ser Glu Glu Gly Thr Arg Ile Asp Leu Ala Ala
   580 585 590
   Leu Leu Ala Glu Pro Ala Pro Gly Val Gln Ile Tyr Ala Cys Gly Ala
   595 600 605
   Gly Arg Leu Leu Ala Gly Leu Glu Asp Ala Ser Arg Asn Trp Pro Asp
   610 615 620
   Gly Ala Leu His Val Glu His Phe Thr Ser Ser Leu Ala Ala Leu Asp
   625 630 635 640
   Pro Asp Val Glu His Ala Phe Asp Leu Glu Leu Arg Asp Ser Gly Leu
   645 650 655
   Thr Val Arg Val Glu Pro Thr Gln Thr Val Leu Asp Ala Leu Arg Ala
   660 665 670
   Asn Asn Ile Asp Val Pro Ser Asp Cys Glu Glu Gly Leu Cys Gly Ser
   675 680 685
   Cys Glu Val Ala Val Leu Asp Gly Glu Val Asp His Arg Asp Thr Val
   690 695 700
   Leu Thr Lys Ala Glu Arg Ala Ala Asn Arg Gln Met Met Thr Cys Cys
   705 710 715 720
   Ser Arg Ala Cys Gly Asp Arg Leu Ala Leu Arg Leu
   725 730
<210> 40
   <211> 732
   <212> PRT
   <213> Artificial
<220>
   <223> Synthetic protein
<400> 40
   Met Arg Val Asp Ser Glu Asn Met Asn Glu Pro Val Thr Leu Pro Thr
   1 5 10 15
   Gly Arg Ala Val Gly Tyr Pro Phe Asp Pro Pro Pro Asp Leu Val Lys
   20 25 30
   Leu Pro Pro Val Ser Pro Met Arg Phe Pro Asp Gly His Ile Gly Trp
   35 40 45
   Leu Val Thr Ser His Ala Ala Ala Arg Thr Val Met Ile Asp Pro Arg
   50 55 60
   Phe Ser Asn Arg Pro Glu His Lys His Pro Val Phe Ser Val Ile Pro
   65 70 75 80
   Arg Pro Gly Gly Ala Thr Lys Ala Pro Ala Pro Gly Trp Phe Ile Asn
   85 90 95
   Met Asp Ala Pro Glu His Thr Arg Tyr Arg Arg Met Leu Ile Ser Gln
   100 105 110
   Phe Thr Val Arg Arg Ile Lys Glu Leu Glu Pro Arg Ile Val Gln Ile
   115 120 125
   Thr Glu Asp His Leu Asp Ala Met Ala Lys Ala Gly Pro Pro Val Asp
   130 135 140
   Leu Val Gln Ala Phe Ala Leu Pro Val Pro Ser Leu Val Ile Cys Glu
   145                 150                 155                 160
   Leu Leu Gly Val Ser Tyr Ala Asp His Ala Phe Phe Gln Glu Gln Thr
                   165                 170                 175
   Thr Ile Met Leu Ser Val Asp Lys Thr Gln Asp Lys Val Thr Thr Ala
               180                 185                 190
   Leu Gly Lys Leu Thr Arg Tyr Ile Ala Glu Leu Val Ala Thr Lys Arg
           195                 200                 205
   Leu Ser Pro Lys Asp Asp Leu Leu Gly Ser Leu Ile Thr Asp Thr Asp
       210                 215                 220
   Leu Thr Asp Glu Glu Leu Thr Asn Ile Ala Leu Ile Leu Leu Val Ala
   225                 230                 235                 240
   Gly His Glu Thr Thr Ala Asn Met Leu Gly Leu Gly Thr Phe Ala Leu
                   245                 250                 255
   Leu Gln His Pro Glu Gln Ile Ala Val Leu Asp Ser Pro Asp Ala Val
               260                 265                 270
   Glu Glu Leu Leu Arg Tyr Leu Ser Ile Val His Leu Gly Thr Pro Asn
           275                 280                 285
   Arg Ala Ala Leu Glu Asp Val Glu Leu Glu Gly Gln Met Ile Arg Lys
       290                 295                 300
   Gly Asp Thr Val Ala Ile Gly Leu Pro Ala Val Asn Arg Asp Pro Lys
   305                 310                 315                 320
   Val Phe Asp Glu Pro Asp Ile Leu Gln Leu Asp Arg Val Asp Ala Arg
                   325                 330                 335
   Lys His Ala Ala Phe Gly Gly Gly Ile His Gln Cys Leu Gly Gln Gln
               340                 345                 350
   Leu Ala Arg Val Glu Met Arg Ile Gly Phe Thr Arg Leu Phe Ala Arg
           355                 360                 365
   Phe Pro Ser Leu Arg Leu Ala Val Pro Ala Glu Glu Ile Lys Leu Arg
   370 375 380
   Glu Lys Ser Ala Ala Tyr Gly Val Trp Ala Leu Pro Val Ala Trp Asp
   385 390 395 400
   Ala Ser Ser Val Leu His Arg His Gln Pro Val Thr Ile Gly Glu Pro
   405 410 415
   Ala Ala Arg Ala Val Ser Arg Thr Val Thr Val Glu Arg Leu Asp Arg
   420 425 430
   Ile Ala Asp Asp Val Leu Arg Leu Val Leu Arg Asp Ala Gly Gly Lys
   435 440 445
   Thr Leu Pro Thr Trp Thr Pro Gly Ala His Ile Asp Leu Asp Leu Gly
   450 455 460
   Ala Leu Ser Arg Gln Tyr Ser Leu Cys Gly Ala Pro Asp Ala Pro Ser
   465 470 475 480
   Tyr Glu Ile Ala Val His Leu Asp Pro Glu Ser Arg Gly Gly Ser Arg
   485 490 495
   Tyr Ile His Glu Gln Leu Glu Val Gly Ser Pro Leu Arg Met Arg Gly
   500 505 510
   Pro Arg Asn His Phe Ala Leu Asp Pro Gly Ala Glu His Tyr Val Phe
   515 520 525
   Val Ala Gly Gly Ile Gly Ile Thr Pro Val Leu Ala Met Ala Asp His
   530 535 540
   Ala Arg Ala Arg Gly Trp Ser Tyr Glu Leu His Tyr Cys Gly Arg Asn
   545 550 555 560
   Arg Ser Gly Met Ala Tyr Leu Glu Arg Val Ala Gly His Gly Asp Arg
   565 570 575
   Ala Ala Leu His Val Ser Glu Glu Gly Thr Arg Ile Asp Leu Ala Ala
   580 585 590
   Leu Leu Ala Glu Pro Ala Pro Gly Val Gln Ile Tyr Ala Cys Gly Ala
   595 600 605
   Gly Arg Leu Leu Ala Gly Leu Glu Asp Ala Ser Arg Asn Trp Pro Asp
       610                 615                 620
   Gly Ala Leu His Val Glu His Phe Thr Ser Ser Leu Ala Ala Leu Asp
   625                 630                 635                 640
   Pro Asp Val Glu His Ala Phe Asp Leu Glu Leu Arg Asp Ser Gly Leu
                   645                 650                 655
   Thr Val Arg Val Glu Pro Thr Gln Thr Val Leu Asp Ala Leu Arg Ala
               660                 665                 670
   Asn Asn Ile Asp Val Pro Ser Asp Cys Glu Glu Gly Leu Cys Gly Ser
           675                 680                 685
   Cys Glu Val Ala Val Leu Asp Gly Glu Val Asp His Arg Asp Thr Val
       690                 695                 700
   Leu Thr Lys Ala Glu Arg Ala Ala Asn Arg Gln Met Met Thr Cys Cys
   705                 710                 715                 720
   Ser Arg Ala Cys Gly Asp Arg Leu Ala Leu Arg Leu
                   725                 730
<210> 41
   <211> 732
   <212> PRT
   <213> Artificial
<220>
   <223> Synthetic protein
<400> 41
   Met Arg Val Asp Ser Glu Asn Met Asn Glu Pro Val Thr Leu Pro Thr
   1               5                   10                  15
   Gly Arg Ala Val Gly Tyr Pro Phe Asp Pro Pro Pro Asp Leu Val Lys
               20                  25                  30
   Leu Pro Pro Val Ser Pro Met Arg Phe Pro Asp Gly His Ile Gly Trp
           35                  40                  45
   Leu Val Thr Ser His Ala Ala Ala Arg Thr Val Met Ile Asp Pro Arg
   50 55 60
   Phe Ser Asn Arg Pro Glu His Lys His Pro Val Phe Ser Val Ile Pro
   65 70 75 80
   Arg Pro Gly Gly Ala Thr Lys Ala Pro Ala Pro Gly Trp Phe Ile Asn
   85 90 95
   Met Asp Ala Pro Glu His Thr Arg Tyr Arg Arg Met Leu Ile Ser Gln
   100 105 110
   Phe Thr Val Arg Arg Ile Lys Glu Leu Glu Pro Arg Ile Val Gln Ile
   115 120 125
   Thr Glu Asp Arg Leu Asp Ala Met Ala Lys Ala Gly Pro Pro Val Asp
   130 135 140
   Leu Val Gln Ala Phe Ala Leu Pro Val Pro Ser Leu Val Ile Cys Glu
   145 150 155 160
   Leu Leu Gly Val Ser Tyr Ala Asp His Ala Phe Phe Gln Glu Gln Thr
   165 170 175
   Thr Ile Met Leu Ser Val Asp Lys Thr Gln Asp Glu Val Thr Thr Ala
   180 185 190
   Leu Gly Lys Leu Thr Arg Tyr Ile Ala Glu Leu Val Ala Thr Lys Arg
   195 200 205
   Leu Ser Pro Lys Asp Asp Leu Leu Gly Ser Leu Ile Thr Asp Thr Asp
   210 215 220
   Leu Thr Asp Glu Glu Leu Thr Asn Ile Ala Leu Ile Leu Leu Val Ala
   225 230 235 240
   Gly His Glu Thr Thr Ala Asn Met Leu Gly Leu Gly Thr Phe Ala Leu
   245 250 255
   Leu Gln His Pro Glu Gln Ile Ala Val Leu Asp Ser Pro Asp Ala Val
   260 265 270
   Glu Glu Leu Leu Arg Tyr Leu Ser Ile Val His Leu Gly Thr Pro Asn
   275 280 285
   Arg Ala Ala Leu Glu Asp Met Glu Leu Glu Gly Gln Met Ile Arg Lys
       290                 295                 300
   Gly Asp Thr Val Ala Ile Gly Leu Pro Ala Val Asn Arg Asp Pro Lys
   305                 310                 315                 320
   Val Phe Asp Glu Pro Asp Ile Leu Gln Leu Asp Arg Val Asp Ala Arg
                   325                 330                 335
   Lys His Ala Ala Phe Gly Gly Gly Ile His Gln Cys Leu Gly Gln Gln
               340                 345                 350
   Leu Ala Arg Val Glu Met Arg Ile Gly Leu Thr Arg Leu Phe Ala Arg
           355                 360                 365
   Phe Pro Ser Leu Arg Leu Ala Val Pro Ala Glu Glu Ile Lys Leu Arg
       370                 375                 380
   Glu Lys Ser Ala Ala Tyr Gly Val Trp Ala Leu Pro Val Ala Trp Asp
   385                 390                 395                 400
   Ala Ser Ser Val Leu His Arg His Gln Pro Val Thr Ile Gly Glu Pro
                   405                 410                 415
   Ala Ala Arg Ala Val Ser Arg Thr Val Thr Val Glu Arg Leu Asp Arg
               420                 425                 430
   Ile Ala Asp Asp Val Leu Arg Leu Val Leu Arg Asp Ala Gly Gly Lys
           435                 440                 445
   Thr Leu Pro Thr Trp Thr Pro Gly Ala His Ile Asp Leu Asp Leu Gly
       450                 455                 460
   Ala Leu Ser Arg Gln Tyr Ser Leu Cys Gly Ala Pro Asp Ala Pro Ser
   465                 470                 475                 480
   Tyr Glu Ile Ala Val His Leu Asp Pro Glu Ser Arg Gly Gly Ser Arg
                   485                 490                 495
   Tyr Ile His Glu Gln Leu Glu Val Gly Ser Pro Leu Arg Met Arg Gly
               500                 505                 510
   Pro Arg Asn His Phe Ala Leu Asp Pro Gly Ala Glu His Tyr Val Phe
           515                 520                 525
   Val Ala Gly Gly Ile Gly Ile Thr Pro Val Leu Ala Met Ala Asp His
       530                 535                 540
   Ala Arg Ala Arg Gly Trp Ser Tyr Glu Leu His Tyr Cys Gly Arg Asn
   545                 550                 555                 560
   Arg Ser Gly Met Ala Tyr Leu Glu Arg Val Ala Gly His Gly Asp Arg
                   565                 570                 575
   Ala Ala Leu His Val Ser Glu Glu Gly Thr Arg Ile Asp Leu Ala Ala
               580                 585                 590
   Leu Leu Ala Glu Pro Ala Pro Gly Val Gln Ile Tyr Ala Cys Gly Ala
           595                 600                 605
   Gly Arg Leu Leu Ala Gly Leu Glu Asp Ala Ser Arg Asn Trp Pro Asp
       610                 615                 620
   Gly Ala Leu His Val Glu His Phe Thr Ser Ser Leu Ala Ala Leu Asp
   625                 630                 635                 640
   Pro Asp Val Glu His Ala Phe Asp Leu Glu Leu Arg Asp Ser Gly Leu
                   645                 650                 655
   Thr Val Arg Val Glu Pro Thr Gln Thr Val Leu Asp Ala Leu Arg Ala
               660                 665                 670
   Asn Asn Ile Asp Val Pro Ser Asp Cys Glu Glu Gly Leu Cys Gly Ser
           675                 680                 685
   Cys Glu Val Ala Val Leu Asp Gly Glu Val Asp His Arg Asp Thr Val
       690                 695                 700
   Leu Thr Lys Ala Glu Arg Ala Ala Asn Arg Gln Met Met Thr Cys Cys
   705                 710                 715                 720
   Ser Arg Ala Cys Gly Asp Arg Leu Ala Leu Arg Leu
                   725                 730
<210> 42
   <211> 732
   <212> PRT
   <213> Artificial
<220>
   <223> Synthetic protein
<400> 42
   Met Arg Val Asp Ser Glu Asn Met Asn Glu Pro Val Thr Leu Pro Thr
   1               5                   10                  15
   Gly Arg Ala Val Gly Tyr Pro Leu Asp Pro Pro Pro Asp Leu Val Lys
               20                  25                  30
   Leu Pro Pro Val Ser Pro Met Arg Phe Pro Asp Gly His Ile Gly Trp
           35                  40                  45
   Leu Val Thr Ser His Ala Ala Ala Arg Thr Val Met Ile Asp Pro Arg
       50                  55                  60
   Phe Ser Asn Arg Pro Glu His Lys His Pro Val Phe Ser Val Ile Pro
   65                  70                  75                  80
   Arg Pro Gly Gly Ala Thr Lys Ala Pro Ala Pro Gly Trp Phe Ile Asn
                   85                  90                  95
   Met Asp Ala Pro Glu His Thr Arg Tyr Arg Arg Met Leu Ile Ser Gln
               100                 105                 110
   Phe Thr Val Arg Arg Ile Lys Glu Leu Glu Pro Arg Ile Val Gln Ile
           115                 120                 125
   Thr Glu Asp His Leu Asp Ala Met Ala Lys Ala Gly Pro Pro Val Asp
       130                 135                 140
   Leu Val Gln Ala Phe Ala Leu Pro Val Pro Ser Leu Val Ile Cys Glu
   145                 150                 155                 160
   Leu Leu Gly Val Ser Tyr Ala Asp His Ala Phe Phe Gln Glu Gln Thr
                   165                 170                 175
   Thr Ile Met Leu Ser Val Asp Lys Thr Gln Asp Glu Val Thr Thr Ala
               180                 185                 190
   Leu Gly Lys Leu Thr Arg Tyr Ile Ala Glu Leu Val Ala Thr Lys Arg
   195 200 205
   Leu Ser Pro Lys Asp Asp Leu Leu Gly Ser Leu Ile Thr Asp Thr Asp
   210 215 220
   Leu Thr Asp Glu Glu Leu Thr Asn Ile Ala Leu Ile Leu Leu Val Ala
   225 230 235 240
   Gly His Glu Thr Thr Ala Asn Met Leu Gly Leu Gly Thr Phe Ala Leu
   245 250 255
   Leu Gln His Pro Glu Gln Ile Ala Cys Leu Asp Ser Pro Asp Ala Val
   260 265 270
   Glu Glu Leu Leu Arg Tyr Leu Ser Ile Val His Leu Gly Thr Pro Asn
   275 280 285
   Arg Ala Ala Leu Glu Asp Val Glu Leu Glu Gly Gln Met Ile Arg Lys
   290 295 300
   Gly Asp Thr Val Ala Ile Gly Leu Pro Ala Val Asn Arg Asp Pro Lys
   305 310 315 320
   Val Phe Asp Glu Pro Asp Ile Leu Gln Leu Asp Arg Val Asp Ala Arg
   325 330 335
   Lys His Ala Ala Phe Gly Gly Gly Ile His Gln Cys Leu Gly Gln Gln
   340 345 350
   Leu Ala Arg Val Glu Met Arg Ile Gly Phe Thr Arg Leu Phe Ala Arg
   355 360 365
   Phe Pro Ser Leu Arg Leu Ala Val Pro Ala Glu Glu Ile Lys Leu Arg
   370 375 380
   Glu Lys Ser Ala Ala Tyr Gly Val Trp Ala Leu Pro Val Ala Trp Asp
   385 390 395 400
   Ala Ser Ser Val Leu His Arg His Gln Pro Val Thr Ile Gly Glu Pro
   405 410 415
   Ala Ala Arg Ala Val Ser Arg Thr Val Thr Val Glu Arg Leu Asp Arg
               420                 425                 430
   Ile Ala Asp Asp Val Leu Arg Leu Val Leu Arg Asp Ala Gly Gly Lys
           435                 440                 445
   Thr Leu Pro Thr Trp Thr Pro Gly Ala His Ile Asp Leu Asp Leu Gly
       450                 455                 460
   Ala Leu Ser Arg Gln Tyr Ser Leu Cys Gly Ala Pro Asp Ala Pro Ser
   465                 470                 475                 480
   Tyr Glu Ile Ala Val His Leu Asp Pro Glu Ser Arg Gly Gly Ser Arg
                   485                 490                 495
   Tyr Ile His Glu Gln Leu Glu Val Gly Ser Pro Leu Arg Met Arg Gly
               500                 505                 510
   Pro Arg Asn His Phe Ala Leu Asp Pro Gly Ala Glu His Tyr Val Phe
           515                 520                 525
   Val Ala Gly Gly Ile Gly Ile Thr Pro Val Leu Ala Met Ala Asp His
       530                 535                 540
   Ala Arg Ala Arg Gly Trp Ser Tyr Glu Leu His Tyr Cys Gly Arg Asn
   545                 550                 555                 560
   Arg Ser Gly Met Ala Tyr Leu Glu Arg Val Ala Gly His Gly Asp Arg
                   565                 570                 575
   Ala Ala Leu His Val Ser Glu Glu Gly Thr Arg Ile Asp Leu Ala Ala
               580                 585                 590
   Leu Leu Ala Glu Pro Ala Pro Gly Val Gln Ile Tyr Ala Cys Gly Ala
           595                 600                 605
   Gly Arg Leu Leu Ala Gly Leu Glu Asp Ala Ser Arg Asn Trp Pro Asp
       610                 615                 620
   Gly Ala Leu His Val Glu His Phe Thr Ser Ser Leu Ala Ala Leu Asp
   625                 630                 635                 640
   Pro Asp Val Glu His Ala Phe Asp Leu Glu Leu Arg Asp Ser Gly Leu
                   645                 650                 655
   Thr Val Arg Val Glu Pro Thr Gln Thr Val Leu Asp Ala Leu Arg Ala
               660                 665                 670
   Asn Asn Ile Asp Val Pro Ser Asp Cys Glu Glu Gly Leu Cys Gly Ser
           675                 680                 685
   Cys Glu Val Ala Val Leu Asp Gly Glu Val Asp His Arg Asp Thr Val
       690                 695                 700
   Leu Thr Lys Ala Glu Arg Ala Ala Asn Arg Gln Met Met Thr Cys Cys
   705                 710                 715                 720
   Ser Arg Ala Cys Gly Asp Arg Leu Ala Leu Arg Leu
                   725                 730
<210> 43
   <211> 16
   <212> PRT
   <213> Artificial
<220>
   <223> Synthetic protein
<400> 43
   Lys Ala Pro Ala Pro Gly Trp Phe Ile Asn Met Asp Ala Pro Glu His
   1               5                   10                  15
<210> 44
   <211> 18
   <212> PRT
   <213> Artificial
<220>
   <223> Synthetic protein
<400> 44
   Leu Thr Asn Ile Ala Leu Leu Leu Leu Val Ala Gly His Glu Thr Thr
   1               5                   10                  15
   Ala Asn
<210> 45
   <211> 13
   <212> PRT
   <213> Artificial
<220>
   <223> Synthetic protein
<400> 45
   Arg Tyr Leu Ser Ile Val His Leu Gly Thr Pro Asn Arg
   1 5 10
<210> 46
   <211> 13
   <212> PRT
   <213> Artificial
<220>
   <223> Synthetic protein
<400> 46
   Glu Glu Ile Lys Leu Arg Glu Lys Ser Ala Ala Tyr Gly
   1 5 10
<210> 47
   <211> 12
   <212> PRT
   <213> Artificial
<220>
   <223> Synthetic protein
<400> 47
   Phe Gln Glu Gln Thr Thr Ile Met Ala Ser Val Asp
   1 5 10
<210> 48
   <211> 16
   <212> PRT
   <213> Artificial
<220>
   <223> Synthetic protein
<400> 48
   Lys Ala Pro Ala Pro Gly Trp Phe Ala Asn Met Asp Ala Pro Glu His
   1 5 10 15
<210> 49
   <211> 16
   <212> PRT
   <213> Artificial
<220>
   <223> Synthetic protein
<400> 49
   Lys Ala Pro Ala Pro Gly Trp Phe Thr Asn Met Asp Ala Pro Glu His
   1               5                   10                  15
<210> 50
   <211> 16
   <212> PRT
   <213> Artificial
<220>
   <223> Synthetic protein
<400> 50
   Lys Ala Pro Ala Pro Gly Trp Phe Phe Asn Met Asp Ala Pro Glu His
   1               5                   10                  15
<210> 51
   <211> 18
   <212> PRT
   <213> Artificial
<220>
   <223> Synthetic protein
<400> 51
   Leu Thr Asn Thr Ala Leu Leu Leu Leu Val Ala Gly His Glu Thr Thr
   1               5                   10                  15
   Ala Asn
<210> 52
   <211> 18
   <212> PRT
   <213> Artificial
<220>
   <223> Synthetic protein
<400> 52
   Leu Thr Asn Ile Ala Leu Ile Leu Leu Val Ala Gly His Glu Thr Thr
   1               5                   10                  15
   Ala Asn
<210> 53
   <211> 18
   <212> PRT
   <213> Artificial
<220>
   <223> Synthetic protein
<400> 53
   Leu Thr Asn Ile Ala Leu Pro Leu Leu Val Ala Gly His Glu Thr Thr
   1               5                   10                  15
   Ala Asn
<210> 54
   <211> 13
   <212> PRT
   <213> Artificial
<220>
   <223> Synthetic protein
<400> 54
   Arg Tyr Leu Ser Ile Val His Leu Gly Ala Pro Asn Arg
   1               5                   10
<210> 55
   <211> 13
   <212> PRT
   <213> Artificial
<220>
   <223> Synthetic protein
<400> 55
   Glu Glu Ile Lys Leu Arg Glu Lys Ser Thr Ala Tyr Gly
   1               5                   10
<210> 56
   <211> 12
   <212> PRT
   <213> Artificial
<220>
   <223> Synthetic protein
<400> 56
   Phe Gln Glu Gln Thr Thr Ile Met Thr Ser Val Asp
   1               5                   10
<210> 57
   <211> 12
   <212> PRT
   <213> Artificial
<220>
   <223> Synthetic protein
<400> 57
   Phe Gln Glu Gln Thr Thr Ile Met Val Ser Val Asp
   1               5                   10
<210> 58
   <211> 12
   <212> PRT
   <213> Artificial
<220>
   <223> Synthetic protein
<400> 58
   Phe Gln Glu Gln Thr Thr Ile Met Leu Ser Val Asp
   1               5                   10
<210> 59
   <211> 12
   <212> PRT
   <213> Artificial
<220>
   <223> Synthetic protein
<400> 59
   Phe Gln Glu Gln Thr Thr Ile Met Phe Ser Val Asp
   1               5                   10

## Description

### Field of the invention

The present invention relates to a method for the production of pravastatin.

### Background of the invention

Statins are known inhibitors of 3-hydroxy-3-methylbutyryl coenzyme A reductase, the rate-limiting enzyme in cholesterol biosynthesis. As such, statins are able to reduce plasma cholesterol levels in various mammalian species, including man, and the compounds are therefore effective in the treatment of hypercholesterolemia. There are several types of statins on the market, amongst which atorvastatin, pravastatin, compactin, lovastatin and simvastatin. While the former is made via chemical synthesis, the latter four are produced either via direct fermentation or via precursor fermentation. These (precursor) fermentations are carried out by fungi of the genera *Penicillium, Aspergillus* and *Monascus.*

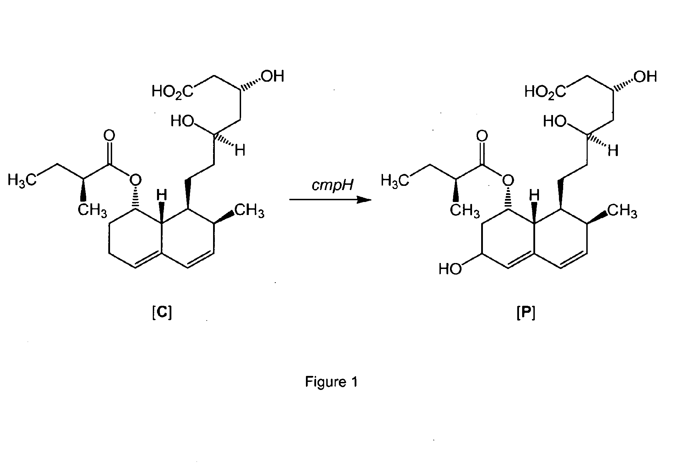

Pravastatin is produced in two sequential fermentations. First *Penicillium citrinum* produces compactin, of which the lactone ring is chemically hydrolyzed and the resulting product is subsequently fed to a cultivation of *Streptomyces carbophilus* which hydroxylates it to pravastatin. Biocatalytic hydroxylation processes are also known in *Actinomadura sp.* ATCC 55678 from WO 96/40863 and Peng and Demain (2000, J. Mol. Cat. B, Enz. 10: 151-156) and in *Amycolatopsis* from Chen Chao-Hsien et al. (2006, Curr. Micribiol. 53: 108-112), CN 1584016 and US 5,372,816. In the context of the present invention the term "hydrolyzed compactin" refers to the non-lactone form of compactin, i.e. in which the lactone ring was opened by reaction with water (Figure 1); likewise, the term 'hydrolysis of compactin' refers to opening of the lactone ring. The industrial species and processes for the production of these metabolites are optimized using different methods. Thus, compactin production by *Penicillium citrinum* was increased from the original 40 mg/l to 5 g/l. For the biocatalytic conversion a *Streptomyces* mutant strain with resistance to 3 g/l of mevastatin with an 80% conversion yield was obtained by Metkinen (Metkinen News March 2000, Metkinen Oy, Finland; reviewed by Manzoni and Rollini, 2002, Appl. Microbiol. Biotechnol. 58:555-564). Although commercially viable, this process is far from optimal as compactin titers are low as compared to, for example, industrial amino acid or penicillin G production; moreover, the compactin must be diluted to prevent toxic effects for the *Streptomyces* strains used in the bioconversion (Hosobuchi et al., 1983, J. Antibiot. 36:887-891) and 20% of the compactin fed is not converted by the *Streptomyces* strains.

The conversion from compactin into pravastatin is catalyzed by a p450 enzyme of *Streptomyces carbophilus* (see Matsuoka et al., 1989, Eur. J Biochem. 184:707-713). There is a common problem with *Streptomyces* bacteria as they grow in filaments, which results in cultivations with high viscosity leading to low oxygen transfer rates and therefore lower fermentation outputs. Various other sources for p450 enzymes have been reported (see Geneseq database XP002425141 and XP02425142; JPO Proteins database XP002425148 and XP002425149; UniProt database XP002425145, XP002425146, XP002425147, XP002425267, XP002425268, XP002472886, XP002472887, XP002472888 and XP002472889) but their use in converting compactin into pravastatin has neither been disclosed nor suggested. Optimally, industrially well equipped species like *Escherichia coli,* a host widely used in large scale biocatalysis would be useful, but this species does neither have p450 enzymes nor p450 redox regenerating systems. So far, the use of species suitable for fermentation and enzyme production, like *Escherichia coli,* in the conversion of compactin into pravastatin has not been reported.

Another problem is requirement for co-factor regeneration for the p450 enzyme, which is typically realized by a specific pair of proteins that are present in the host-cell. If this system is not optimal the overall conversion will be substantially lower than 100%, as in the compactin example. Various attempts have been made to isolate alternative species, none of which have a 100% conversion rate (see US 6,905,851, US 6,365,382, US 2005/0153422, US 2004/0253692 and US 2004/0209335). Moreover, none of these show a real improvement over *Streptomyces carbophilus.* Also reported are species with extreme high resistance towards compactin, but with an inefficient conversion (US 6,306,629, US 6,750,366). Others suggest using family shuffling as a method of improving the conversion rate of known p450 enzymes, but do not show any data (US 6,605,430) as indeed this will be very difficult since p450 enzymes can be very substrate specific, do not have much sequence homology and need a set of specific enzymes for co-factor regeneration. It has been tried to solve this latter problem by isolating species that use different enzymes for the conversion. One particular example in this field is an *Actinomadura* species capable of converting compactin into pravastatin with a maximum conversion rate of 78% (Peng and Demain (1998, J. Ind. Microbiol. Biotechnol. 20:373-375; US 6,274,360)). So despite all efforts, *Streptomyces carbophilus,* with only 80% conversion rate, is still used as the industrial species of choice for the formation of pravastatin and improvements are highly desirable.

### Description of the invention

In the context of the present invention, the term "conservative substitution" is intended to mean that a substitution in which the amino acid residue is replaced with an amino acid residue having a similar side chain. These families are known in the art and include amino acids with basic side chains (e.g. lysine, arginine and histidine), acidic side chains (e.g. aspartic acid, glutamic acid), uncharged polar side chains (*e.g.,* glycine, asparagines, glutamine, serine, threonine, tyrosine, cysteine), non-polar side chains (e.g., alanine, valine, leucine, isoleucine, proline, phenylalanine, methionine, tryptophan), β-branched side chains (e.g., threonine, valine, isoleucine) and aromatic side chains (e.g., tyrosine, phenylalanine tryptophan, histidine).

The term "isolated polynucleotide or nucleic acid sequence" as used herein refers to a polynucleotide or nucleic acid sequence which is essentially free of other nucleic acid sequences, *e.g*., at least 20% pure, preferably at least 40% pure, more preferably at least 60% pure, even more preferably at least 80% pure, most preferably at least 90% pure as determined by agarose electrophoresis. For example, an isolated nucleic acid sequence can be obtained by standard cloning procedures used in genetic engineering to relocate the nucleic acid sequence from its natural location to a different site where it will be reproduced.

The term "pravastatin" is defined as the 6'-hydroxyl variant of compactin with an α or β-configuration, or a mixture of both α and β-configurations. It is important to mention here that in the scientific literature, the term pravastatin is solely used for the β-configuration of the 6'-hydroxyl variant of compactin, while the α variant is named epi-pravastatin. However, this invention described a general efficient method to generate 6-hydroxyl variants of compactin. Therefore, the term pravastatin applies for both the α and β forms.

It is an object of the present invention to provide for an effective and industrial applicable method for converting compactin into pravastatin. It is another object of the invention to use a novel p450 enzyme from *Amycolatopsis orientalis* to convert compactin into pravastatin. The present invention solves the problems encountered in prior art processes, by providing a process in which compactin hydroxylation is performed efficiently in *Escherichia coli.* Also provided is a process in which compactin hydroxylation is performed with 100% conversion. More specifically a process is provided wherein compactin is contacted with the *Amycolatopsis orientalis* compactin hydroxylase enzyme (encoded by the *cmpH* gene), either by contacting whole cells or a cell-free extract of *Amycolatopsis orientalis* with compactin. Preferably, a process is provided wherein the compactin hydroxylase *(cmpH)* is obtained from *Amycolatopsis orientalis* and transferred to another host species. Preferably, this host is resistant to high levels of compactin and capable of compactin production.

In a first aspect, provided is a polypeptide selected from the group consisting of a polypeptide having an amino acid sequence according to SEQ ID NO 3 and a polypeptide having an amino acid that is substantially homologous to the sequence of SEQ ID NO 3, the polypeptide displaying compactin hydroxylase activity.

In a first embodiment, said polypeptide hydroxylates compactin with an efficiency of at least 50%, preferably at least 70%, more preferably at least 80%, still more preferably at least 90%, most preferably 99%. Preferably the product of said hydroxylation is pravastatin.

As part of the present invention it is demonstrated that the industrial application of the currently available compactin hydroxylases is limited to species from the class of Actinomycetes; i.e. they cannot be transferred to species more amenable to industrial scale fermentations like *Escherichia coli* or filamentous fungi such as Aspergillus or Penicillium species. The compactin hydroxylase genes described by the present invention do not have this problem. The activity of the novel polypeptides encoded by these genes can therefore be characterized as follows: they enable application in other species than Actinomycetes, for example in *Escherichia coli,* and/or they enable an efficient hydroxylation of compactin to pravastatin with a conversion efficiency of at least 80%. In the context of the invention, an efficiency of at least 80% means that at least 80% of the compactin is converted into pravastatin.

A polypeptide with an amino acid sequence that is substantially homologous to SEQ ID NO 3 is defined as a polypeptide with an amino acid sequence with a degree of identity to the specified amino acid sequence of at least 90%, most preferably at least 95%, still most preferably at least 97%, ultimately at least 98%, still more ultimately at least 99%, the substantially homologous peptide displaying compactin hydroxylase activity. A substantially homologous polypeptide encompasses polymorphisms that may exist in cells from different populations or within a population due to natural allelic or intra-strain variation. A substantially homologous polypeptide may further be derived from a species other than the species where the specified amino acid and/or DNA sequence originates from, or may be encoded by an artificially designed and synthesized DNA sequence. DNA sequences related to the specified DNA sequences and obtained by degeneration of the genetic code are also part of the invention. Homologues also encompass biologically active fragments of the full-length sequence, still displaying compactin hydroxylase activity.

The degree of identity between two amino acid sequences refers to the percentage of amino acids that are identical between the two sequences. The degree of identity is determined using the BLAST algorithm, which is described in Latched et al. (1990, J. Mol. Biol. 215:403-410). BLAST analysis software is available through the National Center for Biotechnology Information (http://www.ncbi.nim.nih.gov/). The BLAST algorithm parameters W, T, and X determine the sensitivity and speed of the alignment. The BLAST program uses as defaults a word length (W) of 11, the BLOSUM62 scoring matrix (see Henikoff and Henikoff, 1989, Proc Natl. Acad. Sci. USA 89:10915) alignments (B) of 50, expectation (E) of 10, M=5 and N=-4.

Substantially homologous polypeptides may contain only conservative substitutions of one or more amino acids of the specified amino acid sequences or substitutions, insertions or deletions of non-essential amino acids. Accordingly, a non-essential amino acid is a residue that can be altered in one of these sequences without substantially altering the biological function. For example, guidance concerning how to make phenotypically silent amino acid substitutions is provided in Bowie et al. (1990, Science 247:1306-1310) indicating that there are two main approaches for studying the tolerance of an amino acid sequence to change. The first method relies on the process of evolution, in which mutations are either accepted or rejected by natural selection. The second approach uses genetic engineering to introduce amino acid changes at specific positions of a cloned gene and selects or screens to identify sequences that maintain functionality. These studies have revealed that proteins are surprisingly tolerant to amino acid substitutions and reveal which changes are likely to be permissive at a certain position of the protein. For example, most buried amino acid residues require non-polar side chains, whereas few features of surface side chains are generally conserved. Other such phenotypically silent substitutions are described in Bowie et al, and the references cited therein.

In a second embodiment, variants leading to an improved catalytic function (i.e. conversion of compactin into pravastatin) may be obtained by modifying the polynucleotide sequences encoding compactin hydroxylase. Among such modifications are:
- Improving the codon usage in such a way that the codons are adapted to the host species used for expressing the compactin hydroxylase
- Improving the codon pair usage in such a way that the codons are adapted to the host species used for expressing the compactin hydroxylase
- Addition of stabilizing sequences to the genomic information encoding compactin hydroxylase resulting in mRNA molecules with an increased half life
- Error prone PCR to introduce random mutations, followed by a screening of obtained variants (essentially as described in example 4) and isolating of variants with improved kinetic properties
- Family shuffling of related variants of compactin hydroxylase, followed by a screening of obtained variants (essentially as described in example 4) and isolating of variants with improved kinetic properties

Preferred methods to isolate variants with improved kinetic properties are described in WO03010183 and WO0301311.

An improved catalytic function is obtained when an improved polynucleotide is obtained that encodes compactin hydroxylase with improved functionality. As part of the present invention, it has surprisingly been found that the ratio between the β-configuration of the 6-hydroxyl variant of compactin (*i.e*. the pharmaceutically active pravastatin isomer) and the α-configuration of the 6-hydroxyl variant of compactin could be improved significantly using the improved polypeptide sequences of SEQ ID NO 19, 20, 21, 22, 23, 24, 25 or 26 or sequences substantially homologous thereto.

In addition, it was established that certain stretches within the sequence of the polypeptides of the first aspect of the present invention are directly involved in the catalytic machinery of the hydroxylation of compactin. These are SEQ ID NO 43, 44, 45, 46 and 47. An improved catalytic function could be obtained by introducing modifications in any or in all of SEQ ID NO 43-47. Preferably, any or all of SEQ ID NO 43-47 are modified by replacing a single amino acid, two amino acids, three amino acids or at most four amino acids. It has been established that the following modifications lead to an improved catalytic function. For SEQ ID NO 43 the preferred modifications are SEQ ID NO 48, 49 and 50, for SEQ ID NO 44 the preferred modifications are SEQ ID NO 51, 52 and 53, for SEQ ID NO 45 the preferred modification is SEQ ID NO 54, for SEQ ID NO 46 the preferred modification is SEQ ID NO 55 and for SEQ ID NO 47 the preferred modifications are SEQ ID NO 56, 57, 58 and 59. Stretches suitable for contributing to the hydroxylation of compactin are also SEQ ID NO 43-59 wherein one, two or three amino acids are replaced with alternate amino acids.

In a third embodiment, there is provided a polynucleotide or nucleic acid sequence comprising a DNA sequence encoding the polypeptides mentioned above. This may be an isolated polynucleotide of genomic, cDNA, RNA, semi-synthetic, synthetic origin, or any combinations thereof. In particular, a specific DNA sequence is provided encoding the polypeptide of SEQ ID NO 3, i.e. SEQ ID NO 1 or 2. More preferably, the specific DNA sequence is provided encoding the polypeptides of SEQ ID 19-26, i.e. SEQ ID 11-18. Unless otherwise indicated, all nucleotide sequences determined by sequencing a DNA molecule herein are determined using an automated DNA sequencer and all amino acid sequences of polypeptides encoded by DNA molecules determined herein were predicted by translation of a DNA sequence determined as above. Therefore, for any DNA sequence determined by this automated approach, any nucleotide sequence determined may contain some errors. Nucleotide sequences determined by automation are typically at least about 90% identical, more typically at least about 95% to at least about 99.9% identical to the actual nucleotide sequence of the sequenced DNA molecule. The actual sequence can be more precisely determined by other approaches including manual DNA sequencing methods. As is also known in the art, a single insertion or deletion in a determined nucleotide sequence compared to the actual sequence will cause a frame shift in translation of the nucleotide sequence such that the predicted amino acid sequence encoded by a determined nucleotide sequence will be completely different from the amino acid sequence actually encoded by the sequenced DNA molecule, beginning at the point of such an insertion or deletion. The person skilled in the art is capable of identifying such erroneously identified bases and knows how to correct for such errors.

The polypeptides and the encoding nucleic acid sequences of the first aspect of the invention may be obtained from any prokaryotic cell, preferably from Actinomycetes. Preferred actinomycetes species include, but are not limited to, strains of *Streptomyces, Amycolatopsis, Pseudonocardia, Micromonospora, Nocardia* and *Actinokineospora.* In a preferred embodiment, the nucleic acid sequence encoding a polypeptide of the present invention is obtained from a strain of *Amycolatopsis orientalis.*

DNA sequences of the invention may be identified by hybridization. Nucleic acid molecules corresponding to variants (e.g. natural allelic variants) and homologues of the DNA of the invention can be isolated based on their homology to the nucleic acids disclosed herein using these nucleic acids or a suitable fragment thereof, as a hybridization probe according to standard hybridization techniques, preferably under highly stringent hybridization conditions. Alternatively, one could apply *in silico* screening through the available genome databases. "Stringency" of hybridization reactions is readily determinable by one of ordinary skill in the art. For additional details and explanation of stringency of hybridization reactions, see Ausubel et al. (1995, Current Protocols in Molecular Biology, Wiley Interscience Publishers).

The nucleic acid sequence may be isolated by e.g. screening a genomic or cDNA library of the microorganism in question. Once a nucleic acid sequence encoding a polypeptide having an activity according to the invention has been detected with e.g. a probe derived from SEQ ID NO 2, the sequence may be isolated or cloned by utilizing techniques which are known to those of ordinary skill in the art (see Sambrook et al., 1989, Molecular Cloning, A Laboratory Manual, 2d edition, Cold Spring Harbor, New York). The cloning of the nucleic acid sequences of the present invention from such (genomic) DNA can also be effected, *e.g*. by using methods based on polymerase chain reaction (PCR) or antibody screening of expression libraries to detect cloned DNA fragments with shared structural features (See, *e.g*., Innis et al., 1990, PCR: A Guide to Methods and Application, Academic Press, New York.).

The sequence information as provided herein should not be so narrowly construed as to require inclusion of erroneously identified bases. The specific sequences disclosed herein can be readily used to isolate the complete gene from Actinomycetes, in particular *Amycolatopsis orientalis,* which in turn can easily be subjected to further sequence analyses thereby identifying sequencing errors.

Unless otherwise indicated, all nucleotide sequences determined by sequencing a DNA molecule herein where determined using an automated DNA sequencer and all amino acid sequences of polypeptides encoded by DNA molecules determined herein were predicted by translation of a DNA sequence determined as above. Therefore, as is known in the art for any DNA sequence determined by this approach, any nucleotide sequence determined herein may contain errors. Nucleotide sequences determined by automation are typically at least about 90% identical, more typically at least about 95% to at least about 99.9% identical to the actual nucleotide sequence of the sequenced DNA molecule. The actual sequence can be more precisely determined by other approaches including manual DNA sequencing methods well known in the art. As is also known in the art, a single insertion or deletion in a determined nucleotide sequence compared to the actual sequence will cause a frame shift in translation of the nucleotide sequence such that the predicted amino acid sequence encoded by a determined nucleotide sequence will be completely different from the amino acid sequence actually encoded by the sequenced DNA molecule, beginning at the point of such an insertion or deletion. The person skilled in the art is capable of identifying such erroneously identified bases and knows how to correct for such errors.

In a fourth embodiment the invention provides for an improved compactin hydroxylase enzyme by fusing the polypeptide of SEQ ID NO 3 to a so-called reductase domain to form the polypeptide of SEQ ID NO 6 and displaying compactin hydroxylase activity. The scope of this invention is not limited to this specific amino acid sequence, but includes polypeptides having an amino acid sequence that is "substantially homologous" to the sequence of SEQ ID NO 6, which is defined as a polypeptide having an amino acid sequence possessing a degree of identity to the specified amino acid sequence of at least 90%, still more preferably at least 95%, still more preferably at least 98%, most preferably at least 99%, the substantially homologous peptide displaying compactin hydroxylase activity. A substantially homologous polypeptide may encompass polymorphisms that may exist in cells from different populations or within a population due to natural allelic or intra-strain variation. A substantially homologous polypeptide may further be derived from a species other than the species where the specified amino acid and/or DNA sequence originates from, or may be encoded by an artificially designed and synthesized DNA sequence. DNA sequences related to the specified DNA sequences and obtained by degeneration of the genetic code are also part of the invention. Homologues may also encompass biologically active fragments of the full-length sequence, still displaying compactin hydroxylase activity. The person skilled in the art will understand that the hydroxylase part of this fusion protein may be exchanged for non-homologous, but still functional equivalent sequences, like other p450 enzymes capable of hydroxylating compactin, such as the *Streptomyces carbophilus* p450sca-2 gene, provided that the fused protein displays compactin hydroxylation pravastatin. Also, the reductase domain can be exchanged for non-homologous, but still functionally equivalent sequences, for example ferredoxins and ferredoxin reductases, provided that the fused protein displays compactin hydroxylation towards pravastatin. Alternative reductase domains that can be used are for example the reductase domains of the self-sufficient P450 enzymes from *Bacillus megaterium,* P450 BM3, NCBI Genbank accession number gi142797. Preferred fused polypeptides are the congeners of the improved polypeptides of SEQ ID NO 19-26, namely SEQ ID NO 35, 36, 37, 38, 39, 40, 41 or 42 or sequences substantially homologous thereto. In addition, also the specific DNA sequences encoding the polypeptides of SEQ ID NO 34-42, i.e. SEQ ID NO 27-34 are part of the present invention. Alternatively, improvements in the catalytic function as described in the second embodiment may also be performed on the reductase region.

In a second aspect, the present invention discloses the use of a polynucleotide of the first aspect in recombinant host strains. More particularly, disclosed is a method for producing pravastatin comprising the steps of:
(i) transforming a host cell of interest with a polynucleotide comprising the gene of interest encoding compactin hydroxylase,
(ii) selecting clones of transformed cells,
(iii) cultivating said selected cells,
(iv) optionally processing said cultivated cells (i.e. immobilizing),
(v) feeding compactin to said cultivated cells,
(vi) isolating pravastatin from said cultivations.

The choice of a host cell in the method of the present invention will to a large extent depend upon the source of the nucleic acid sequence (gene) of interest encoding a polypeptide. Preferably, the host cell is a prokaryotic cell. In a preferred embodiment, the prokaryotic host cell is a cell of a species cited as species from which the polynucleotide of the first or second aspect may be obtained, examples of which are, but are not limited to, *Streptomyces* species (*i.e. Streptomyces carbophilus, Streptomyces flavidovirens, Streptomyces coelicolor, Streptomyces lividans, Streptomyces exfoliatus*) or *Amycolatopsis* species *(i.e. Amycolatopsis orientalis).* In the most preferred situation, the host cell is a host cell suitable for large scale fermentation, examples of which are, but are not limited to, *Streptomyces* species *(i.e. Streptomyces avermitilis, Streptomyces lividans, Streptomyces clavuligerus)* or *Bacillus* species (*i.e*. *Bacillus subtilus, Bacillus amyloliquefaciens, Bacillus licheniformis)* or *Corynebacterium* species *(i.e. Corynebacterium glutamicum)* or *Escherichia* species (*i.e*. *Escherichia* coli). Even more preferably, the host cell is a eukaryotic cell, such as Saccharomyces, *Aspergillus* or *Penicillium* species, suitable examples of which are the yeast *Saccharomyces cerevisiae* or the filamentous fungi *Aspergillus niger, Penicillium chrysogenum* or *Penicillium citrinum.*

Nucleic acid constructs, *e.g*. expression constructs, may contain a selection marker gene and the polynucleotide of the invention (compactin hydroxylase), each operably linked to one or more control sequences, which direct the expression of the encoded polypeptide in a suitable expression host. The nucleic acid constructs may be on separate fragments or, preferably, on one DNA fragment. Expression will be understood to include any step involved in the production of the polypeptide and may include transcription, post-transcriptional modification, translation, post-translational modification and secretion. The term "nucleic acid construct" is synonymous with the term "expression vector" or "cassette" when the nucleic acid construct contains all the control sequences required for expression of a coding sequence in a particular host organism. The term "control sequences" is defined herein to include all components, which are necessary or advantageous for the expression of a polypeptide. Each control sequence may be native or foreign to the nucleic acid sequence encoding the polypeptide. Such control sequences may include, but are not limited to, a promoter, a leader, optimal translation initiation sequences (as described in Kozak, 1991, J. Biol. Chem. 266:19867-19870), a secretion signal sequence, a pro-peptide sequence, a polyadenylation sequence, a transcription terminator. At a minimum, the control sequences include a promoter, and transcriptional and translational stop signals. The term "operably linked" is defined herein as a configuration in which a control sequence is appropriately placed at a position relative to the coding sequence of the DNA sequence such that the control sequence directs the production of a polypeptide.

The control sequence may include an appropriate promoter sequence containing transcriptional control sequences. The promoter may be any nucleic acid sequence, which shows transcription regulatory activity in the cell including mutant, truncated, and hybrid promoters, and may be obtained from genes encoding extra cellular or intracellular polypeptides. The promoter may be either homologous or heterologous to the cell or to the polypeptide. Preferred promoters for prokaryotic cells are known in the art and can be, for example, strong promoters ensuring high level messenger RNA. The promotor used in the expression cassette according to the invention may be selected from the well-known set of inducible promoters for highly expressed operons/genes like the lactose operon *(lac, lacUV5),* the arabinose operon *(ara),* the tryptophan operon (*trp*), and the operon encoding enzymes common to the biosynthesis of all aromatic amino acids (*aro*), or functional hybrids of these, e.g. the *tac* promoter, which is a fusion of the *trp* and the *lac* promoter (Amann et al., 1983, Gene 25:161-178). Alternatively, constitutive promoters can be used providing for a constant supply of messenger RNA throughout the cell's life. Any other useful promoters can be found among others at the NCBI website (http://www.ncbi.nlm.nih.gov/entrez/).

In a preferred embodiment, the promoter may be derived from a gene, which is highly expressed (defined herein as the mRNA concentration with at least 0.5% (w/w) of the total cellular mRNA). In another preferred embodiment, the promoter may be derived from a gene, which is medium expressed (defined herein as the mRNA concentration with at least 0.01% until 0.5% (w/w) of the total cellular mRNA). In another preferred embodiment, the promoter may be derived from a gene, which is low expressed (defined herein as the mRNA concentration lower than 0.01% (w/w) of the total cellular mRNA).

In an even more preferred embodiment, Micro Array data is used to select genes, and thus promoters of those genes, that have a certain transcriptional level and regulation. In this way one can adapt the gene expression cassettes optimally to the conditions it should function in.

Alternatively, one could clone random DNA fragments in front of the polynucleotides of this invention. These can be isolated via a so-called direct selection approach. Using a promoter-less selectable marker gene (i.e. kanamycin resistance) one can clone random DNA fragments in front of this gene and easily screen for active promoters, as these should facilitate growth on media containing kanamycin. These DNA fragments can be derived from many sources, i.e. different species, PCR amplified, synthetically and the like. Subsequently, the sequences can be isolated and cloned in front of the polynucleotides of this invention. Similar strategies can be used to improve translation of the messenger RNA pool by introducing, via recDNA methodology, 5'-untranslated leader regions from efficiently translated messenger RNA's like those obtainable from the *tuf* gene encoding the highly expressed Elongation Factor Tu protein, or modified or synthetic variants of the tryptophan operon.

The control sequence may also include a suitable transcription terminator sequence, a sequence recognized by a prokaryotic cell to terminate transcription. The terminator sequence is operably linked to the 3' terminus of the nucleic acid sequence encoding the polypeptide. Any terminator, which is functional in the cell, may be used in the present invention. Preferred terminators for prokaryotic cells are obtained either from the native gene to be expressed or from sources like rRNA genes or viral operons, e.g. the ribosomal RNA terminator, or the *fd* terminator (Sambrook et al., 1989. Molecular Cloning 2nd edition; CSH Press).

For secretion of a polypeptide, the control sequence may include a signal peptide-encoding region, coding for an amino acid sequence linked to the amino terminus of the polypeptide, which can direct the encoded polypeptide into the cell's secretory pathway. The 5'-end of the coding sequence may inherently contain a signal peptide-coding region naturally linked in translation reading frame with the segment of the coding region, encoding the secreted polypeptide. Alternatively, the 5'-end of the coding sequence may contain a signal peptide-coding region, foreign to the coding sequence. The foreign signal peptide-coding region may be required where the coding sequence does not normally contain a signal peptide-coding region. Alternatively, the foreign signal peptide-coding region may simply replace the natural signal peptide-coding region in order to obtain enhanced secretion of the polypeptide.

The nucleic acid construct may be an expression vector. The expression vector may be any vector (e.g. a plasmid or virus), which can be conveniently subjected to recombinant DNA procedures and can bring about the expression of the nucleic acid sequence encoding the polypeptide. The choice of the vector will typically depend on the compatibility of the vector with the cell into which the vector is to be introduced. The vectors may be linear or closed circular plasmids.

In another embodiment, the expression cassette last mentioned is additionally modified by replacement of the original promoter by the *trp* promoter or the *aro* promoter. In order to fully exploit the basic improvement of expression efficiency, additional modifications relating to the increased gene expression, messenger RNA translation and plasmid stability may be applied to the recDNA constructs used to create the actual production strain e.g. addition of the Transcription terminator of phage *fd*, or the introduction of the partitioning function par from plasmid pSC101 (Churchward et al., 1983. Nucl. Acid. Res. 11:5645-5659).

To increase production of the desired protein one can insert the expression cassette on extrachromosomal elements, such as plasmids ColE1, ColD, R1162, RK2 or derivatives that are present in predetermined low copy numbers or, often dynamic, high copy numbers and that are capable of propagation or autonomous replication in *e.g. Escherichia coli* strains HB101, B7, RV308, DH1, HMS174, W3110, BL21.

The vector may be an autonomously replicating vector, *i.e*. a vector, which exists as an extrachromosomal entity, the replication of which is independent of chromosomal replication, *e.g*. a plasmid, an extrachromosomal element, a minichromosome, or an artificial chromosome. Alternatively, the vector may be one which, when introduced into the cell, is integrated into the genome and replicated together with the chromosome(s) into which it has been integrated: The integrative cloning vector may integrate at random or at a predetermined target locus in the chromosomes of the host cell. In a preferred embodiment of the invention, the integrative cloning vector comprises a DNA fragment, which is homologous to a DNA sequence in a predetermined target locus in the genome of host cell for targeting the integration of the cloning vector to this predetermined locus. In order to promote targeted integration, the cloning vector is preferably linearized prior to transformation of the host cell. Linearization is preferably performed such that at least one but preferably either end of the cloning vector is flanked by sequences homologous to the target locus. The length of the homologous sequences flanking the target locus is preferably at least at least 0.1 kb, even preferably at least 0.2 kb, more preferably at least 0.5 kb, even more preferably at least 1 kb, most preferably at least 2 kb. The vector system may be a single vector or plasmid or two or more vectors or plasmids, which together contain the total DNA to be introduced into the genome of the host cell.

The DNA constructs may be used on an episomal vector. Preferably, the constructs are integrated in the genome of the host strain.

In another embodiment, the application of the polypeptides of the present invention can be improved by deleting one or more of the endogenous genes from the genome of the host strain encoding enzymes limiting the pravastatin yields. Examples of such enzymes are, but are not limited to, enzymes that hydrolyze the side chains of compactin or pravastatin.

In a preferred embodiment the *cmpH* gene (SEQ ID NO 1), all homologous sequences, the *cmpH* fusion to a reductase domain (SEQ ID NO 4) and all functional equivalents encoding compactin hydroxylase can be expressed in a compactin producing host cell to produce pravastatin. In case of a prokaryotic host one can apply all aspects of functional expression in such a host as described above. In case of a eukaryotic host cell one preferably adapts the expression constructs towards efficient expression in such hosts. Preferably, the host cell is a fungus, more preferably a filamentous fungus, most preferably, the fungal host cell is a cell which produces statins, preferably compactin. Examples of which are, but are not limited to, *Aspergillus* species (*i.e. Aspergillus terreus*), or *Penicillium* species (*i.e. Penicillium citrinum* or *chrysogenum*), or *Monascus* species (*i.e. Monascus ruber* or *paxii*).

Preferred promoters for filamentous fungal cells are known in the art and can be, for example, the glucose-6-phosphate dehydrogenase *gpdA* promoters, protease promoters such as *pep*A, *pep*B, *pep*C, the glucoamylase *gla*A promoters, amylase *amy*A, *amy*B promoters, the catalase *cat*R or catA promoters, glucose oxidase *gox*C promoter, beta-galactosidase *lac*A promoter, alpha-glucosidase *aglA* promoter, translation elongation factor *tef*A promoter, xylanase promoters such as *xln*A, *xln*B, *xln*C, *xln*D, cellulase promoters such as *egl*A, *egl*B, *cbh*A, promoters of transcriptional regulators such as *are*A, *cre*A, *xln*R, *pac*C, *prt*T, etc or any other, and can be found among others at the NCBI website (http://www.ncbi.nlm.nlm.gov/entrez/).

In a preferred embodiment, the promoter may be derived from a gene, which is highly expressed (defined herein as the mRNA concentration with at least 0.5% (w/w) of the total cellular mRNA). In another preferred embodiment, the promoter may be derived from a gene, which is medium expressed (defined herein as the mRNA concentration with at least 0.01% until 0.5% (w/w) of the total cellular mRNA). In another preferred embodiment, the promoter may be derived from a gene, which is low expressed (defined herein as the mRNA concentration lower than 0.01% (w/w) of the total cellular mRNA).

In an even more preferred embodiment, Micro Array data is used to select genes, and thus promoters of those genes, that have a certain transcriptional level and regulation. In this way one can adapt the gene expression cassettes optimally to the conditions it should function in.

The control sequence may also include a suitable transcription terminator sequence, a sequence recognized by a filamentous fungal cell to terminate transcription. The terminator sequence is operably linked to the 3' terminus of the nucleic acid sequence encoding the polypeptide. Any terminator, which is functional in the cell, may be used in the present invention. Preferred terminators for filamentous fungal cells are obtained from the genes encoding *Aspergillus oryzae* TAKA amylase, *Aspergillus niger* glucoamylase, *Aspergillus nidulans* anthranilate synthase, *Aspergillus niger* alpha-glucosidase, trpC gene and *Fusarium oxysporum* trypsin-like protease.

The control sequence may also include a suitable leader sequence, a non-translated region of an mRNA, which is important for translation by the filamentous fungal cell. The leader sequence is operably linked to the 5'-terminus of the nucleic acid sequence encoding the polypeptide. Any leader sequence, which is functional in the cell, may be used in the present invention. Preferred leaders for filamentous fungal cells are obtained from the genes encoding *Aspergillus oryzae* TAKA amylase and *Aspergillus nidulans* triose phosphate isomerase and *Aspergillus niger* glaA.

The control sequence may also include a polyadenylation sequence, operably linked to the 3'-terminus of the nucleic acid sequence and which, when transcribed, is recognized by the filamentous fungal cell as a signal to add polyadenosine residues to transcribed mRNA. Any polyadenylation sequence, functional in the cell, may be used in the present invention. Preferred polyadenylation sequences for filamentous fungal cells are obtained from the genes encoding *Aspergillus oryzae* TAKA amylase, *Aspergillus niger* glucoamylase, *Aspergillus nidulans* anthranilate synthase, *Fusarium oxysporum* trypsin-like protease and *Aspergillus niger* α-glucosidase.

The nucleic acid construct may be an expression vector. The expression vector may be any vector (*e.g*. a plasmid or virus), which can be conveniently subjected to recombinant DNA procedures and can bring about the expression of the nucleic acid sequence encoding the polypeptide. The choice of the vector will typically depend on the compatibility of the vector with the cell into which the vector is to be introduced. The vectors may be linear or closed circular plasmids.

The vector may be an autonomously replicating vector, *i.e*. a vector, existing as an extrachromosomal entity, the replication of which is independent of chromosomal replication, *e.g*. a plasmid, an extrachromosomal element, a minichromosome, or an artificial chromosome. An autonomously maintained cloning vector for a filamentous fungus may comprise the AMA1-sequence (see *e.g*. Aleksenko and Clutterbuck (1997), Fungal Genet. Biol. 21: 373-397). Alternatively, the vector may be one which, when introduced into the cell, is integrated into the genome and replicated together with the chromosome(s) into which it has been integrated. The integrative cloning vector may integrate at random or at a predetermined target locus in the chromosomes of the host cell. Preferably, the integrative cloning vector comprises a DNA fragment, which is homologous to a DNA sequence in a predetermined target locus in the genome of host cell for targeting the integration of the cloning vector to this predetermined locus. In order to promote targeted integration, the cloning vector is preferably linearized prior to transformation of the host cell. Linearization is preferably performed such that at least one but preferably either end of the cloning vector is flanked by sequences homologous to the target locus. The length of the homologous sequences flanking the target locus is preferably at least at least 0.1 kb, even preferably at least 0.2 kb, more preferably at least 0.5 kb, even more preferably at least 1 kb, most preferably at least 2 kb. The vector system may be a single vector or plasmid or two or more vectors or plasmids, which together contain the total DNA to be introduced into the genome of the host cell.

The DNA constructs may be used on an episomal vector. Preferably, the constructs are integrated in the genome of the host strain.

Fungal cells are transformed using co-transformation, *i.e*. along with gene(s) of interest also a selectable marker gene is transformed. This can be either physically linked to the gene of interest (*i.e*. on a plasmid) or on a separate fragment. Following transfection transformants are screened for the presence of this selection marker gene and subsequently analyzed for the presence of the gene(s) of interest. A selectable marker is a product, which provides resistance against a biocide or virus, resistance to heavy metals, prototrophy to auxotrophs and the like. Useful selectable markers include *amdS* (acetamidase), *argB* (omithinecarbamoyltransferase), *bar* (phosphinothricinacetyltransferase), *hygB* (hygromycin phosphotransferase), *niaD* (nitrate reductase), *pyrG* (orotidine-5'-phosphate decarboxylase), *sC* or *sutB* (sulfate adenyltransferase), *trpC* (anthranilate synthase), ble (phleomycin resistance protein), or equivalents thereof.

The obtained host cell may be used for producing pravastatin.

### Legends to the figures

Figure 1 shows the conversion catalyzed by the product of the *cmpH* gene, compactin hydroxylase. Legend: [C] = compactin; [P] = pravastatin.
Figure 2 shows the plasmid pZERO-Ao-11H9. Legend: ORF-1 = first Open Reading Frame, ORF-2 = second Open Reading Frame, zeo = gene encoding resistance to zeocin, kan = gene encoding resistance to kanamycin.
Figure 3 shows the plasmid pZERO-Ao-11H9d. Legend: ORF-1 = first Open Reading Frame, zeo = gene encoding resistance to zeocin, kan = gene encoding resistance to kanamycin.
Figure 4 shows the plasmid pACYC-taqScp450. Legend: Sc-p450 = *Streptomyces carbophilus* gene encoding compactin hydroxylase p450, cat = gene encoding resistance to chloramphenicol.
Figure 5 shows the plasmid pACYC-taqAop450. Legend: A0-cmpH = *Amycolatopsis orientalis* gene encoding compactin hydroxylase p450, cat = gene encoding resistance to chloramphenicol.

### EXAMPLES

### General Methods

Standard DNA procedures and prokaryotic cultivations were carried out as described elsewhere (Sambrook, J. et al., 1989, Molecular cloning: a laboratory manual, 2nd Ed., Cold Spring Harbor Laboratory Press, Cold Spring Harbor, New York). DNA was amplified using the proofreading enzyme Phusion polymerase (Finnzymes). Restriction enzymes were from Invitrogen or New England Biolabs. Hydrolysis of compactin was done by dissolving compactin in ethanol to a final concentration of 20 mg/ml. NaOH was added from a 4M stock, to a final concentration of 0.1M. The solution was heated at 50°C for 1 to 2 hrs and subsequently cooled to rT. This solution can be stored at rT for 3 months. Pravastatin and non-hydrolyzed compactin stock solutions were made by dissolving both compounds in ethanol at 20 mg/ml.

### Example 1

### Screening for efficient whole-cell compactin to pravastatin bioconversion

Diverse prokaryotic and fungal species (Table 1) were tested to isolate a species with improved conversion from hydrolyzed compactin. All species were pre-cultured for 1-3 days (depending on the growth rate of the species) in 25 ml 2xYT medium, washed and suspended in 25 ml fresh 2xYT medium. After an adaptation period of several hours while shaking at 280 rpm and 30°C, hydrolyzed compactin was added at final concentrations of 0.1, 0.2, 0.5 and 1 mg/ml. After 24 h incubation the broths were collected by transferring the content of the shake flasks into a 50 ml Greiner tube.

Samples were frozen at -20°C, followed by freeze drying. The statins were extracted by addition of 1-2 ml methanol to the freeze-dried samples, followed by repeated vortexing. The solids were separated from the liquid phase by centrifugation. 200 µl of the methanol-extract was transferred into an HPLC vial followed by HPLC analysis as follows:

| | | | |
|---|---|---|---|
| Eluens: | A: 33% acetonitrile, 0.025% trifluoroacetic acid in milliQ water | | |
| | B: 80% acetonitrile in MilliQ water | | |

| Gradient: | Time (min) | % eluent A | % eluent B |
|---|---|---|---|
| | 0 - 8 | 100 | 0 |
| | 8 - 8.1 | 100→0 | 0→100 |
| | 8.1 - 12 | 0 | 100 |
| | 12 - 13 | 0→100 | 100→0 |
| | 13 - 14 | 100 | 0 |
| Column: | Waters XTerra RP18 (Column Temp. =Room temperature) | | |
| Flow: | 1 ml/min | | |
| Injection vol.: | 10 µl; (Tray Temp. = Room temperature) | | |
| Instrument: | Waters Alliance 2695 | | |
| Detector: | Waters 996 Photo Diode Array | | |
| Wavelength: | 238 nm | | |
| Retention | time: Pravastatin 4 min, hydr. compactin 10.4 min, compactin 10.9 min | | |

**Table 1 Prokaryotic species tested for hydroxylation of hydrolyzed compactin**

| Species | Strain | Hydrolyzed compactin→pravastatin |
|---|---|---|
| *Escherichia coli* | DH10b | - |
| *Choanephora circinans* | CBS153.58 | - |
| *Helicostylum piriforme* | VKM-F1068 | - |
| *Rhizopus microsporus chinensis* | VKM-F1218 | - |
| *Rhizopus stolonifer stolonifer* | CBS 382.52 | - |
| *Actinokineospora riparia* | VKM-Ac1980 | +/- |
| *Pseudonocardia alni* | VKM-Ac916 | +/- |
| *Streptomyces carbophilus* | FERM-BP1145 | ++ |
| *Amycolatopsis orientalis* | NRRL18098 | ++++ |

As can be seen in Table 1 pravastatin was synthesized by four species of the tested set: *Actinokineospora riparia, Pseudonocardia alni, Streptomyces carbophilus* and *Amycolatopsis orientalis.*

### Example 2

### Biological hydrolysis of compactin

To establish if the species described in Example 1 could also hydrolyze and/or hydroxylate compactin in lactone-form, four selected species were pre-cultured for 1-3 days (depending on the growth rate of the species) in 25 ml 2xYT medium, washed and resuspended in 25 ml fresh 2xYT medium. After adaptation for several hours while shaking at 280 rpm and 30°C, non-hydrolyzed compactin was added at 0.2 mg/ml. After 24 h incubation the broths were collected by transferring the content of the shake flasks into a 50 ml Greiner tube. Samples were frozen at -20°C, followed by freeze drying. The statins were extracted by addition of 1-2 ml methanol, followed by repeated vortexing. The solids were separated from the liquid phase by centrifugation. 200 µl of the methanol-extract was transferred into an HPLC vial followed by HPLC analysis as described in Example 1. All four species (*Actinokineospora riparia, Escherichia coli, Streptomyces carbophilus* and *Amycolatopsis orientalis*) hydrolyze compactin, but this is not a prerequisite for pravastatin formation. *Amycolatopsis orientalis* was the most efficient species in synthesizing pravastatin.

**Table 2 Prokaryotic species tested for hydrolysis and/or hydroxylation of compactin**

| | Compactin added | Compactin | Hydrolyzed compactin | Pravastatin | Unknown product |
|---|---|---|---|---|---|
| *Amycolatopsis orientalis* | 0.2 mg/ml | 4% | 1% | 16% | 81% |
| *Actinokineospora riparia* | 0.2 mg/ml | 75% | 25% | <1% | |
| *Escherichia coli* | 0.2 mg/ml | 49% | 51% | | |
| *Streptomyces carbophilus* | 0.2 mg/ml | 14% | 78% | 8% | |

### Example 3

### Amycolatopsis orientalis has very efficient compactin hydroxylation

From Example 1 it was concluded that *Amycolatopsis orientalis* was superior for compactin hydroxylation to *Streptomyces carbophilus.* For further study both species were pre-cultured for 24 h in 25 ml 2xYT medium, washed and resuspended in 25 ml fresh 2xYT medium. After several hours while shaking at 280 rpm and 30°C, hydrolyzed compactin was added at 0.1 and 0.2 mg/ml. After 24 h incubation the broths were collected by transferring the content of the shake flasks into a 50 ml Greiner tube. Samples were frozen at -20°C, followed by freeze drying. The statins were extracted by addition of 1-2 ml methanol to the dried samples, followed by repeated vortexing. The solids were separated from the liquid phase by centrifugation. 200 µl of the methanol-extract was transferred into an HPLC vial followed by HPLC analysis as described in Example 1. As can be seen from Table 3, *Amycolatopsis orientalis* is capable of converting compactin into pravastatin with 100% efficiency where Streptomyces carbophilus is not.

**Table 3 Comparison in compactin hydroxylation by Amycolatopsis orientalis and Streptomyces carbophilus.**

| | Hydrolyzed compactin added (mg/ml) | Hydrolyzed compactin | Pravastatin | Unknown Product |
|---|---|---|---|---|
| *Amycolatopsis orientalis* | 0.1 | | 100% | |
| | 0.2 | 6% | 88% | 6% |
| *Streptomyces carbophilus* | 0.1 | 14% | 86% | |
| | 0.2 | 16% | 84% | |

### Example 4

### Isolating gene fragment encoding the biocatalyst converting compactin to pravastatin

### Gene library Amycolatopsis orientalis

A colony of *Amycolatopsis orientalis* in liquid medium (10 g/l glucose, 5 g/l yeast extract, 20 g/l starch, 1 g/l CaCO₃ and 0.5 g/l casaminoacids, baffled flasks) was grown at 28°C until OD=2.0. Part was used to prepare glycerol stocks and part was used to inoculate (ratio 1/50) a flask with 50 ml of the liquid medium for the preparation of cells for genomic DNA isolation. After 16 h at 28°C the culture was used for isolating genomic DNA. Thereto, during the last hour of the incubation ampicillin was added to a final concentration of 200 µg/ml. Cells were harvested by centrifugation (15 min at 8000 rpm) and the pellet was resuspended in 5 ml of 50 mM Tris-HCl with 50 mM EDTA adjusted to pH 8.0. After adding 100 µl of lysozyme (100 mg/ml) and 40 µl proteinase K (20 mg/ml), the suspension was incubated for 30 min at 37°C. Nuclei Lysis Solution (6 ml) of Promega was added. Incubation for 15 min at 80°C and 30 min at 65°C led to almost complete cell lysis. After RNase treatment (10 µl of 100 mg/ml RNaseA solution), 2 ml of Protein Precipitation Solution of Promega was added, the mixture was vortexed (20 s) and incubated on ice (15 min). After centrifugation (15 min at 5000 rpm) the supernatant was mixed with 0.1 volumes of NaAc (3 M, pH 5) and 2 volumes of EtOH (96%). The visible complexes of precipitated genomic DNA were transferred with a Pasteur pipette and dissolved in 500 µl of 10 mM Tris (pH 8.0). A second proteinase K treatment (10 µl of the 20 mg/ml stock solution was used per 200 µl sample, followed by 30 min incubation at 37°C) was applied to remove remaining proteins. After the proteinase K step, 500 µl of phenol/chloroform/isoamylalcohol (PCI, 25:24:1) was added and the mixture was centrifuged for 5 min at 14,000 rpm. The upper phase was transferred to a new vial and 500 µl of PCI (24:1) was added for removing traces of phenol. The phases were separated by centrifugation and the upper phase was mixed with 0.1 volumes of NaAc (3 M, pH 5) and 2 volumes of EtOH (96%) for precipitation of the DNA. The genomic DNA was taken out with a pipette, washed with 70% cold EtOH and dissolved in 500 µl of Tris-EDTA buffer. This resulted in 134 µg of purified genomic DNA with an A260nm/A280nm of 1.85. *Sau*3Al (0.067 units/µg DNA) was used to partially digest isolated *Amycolatopsis orientalis* DNA to obtain smaller fragments ranging from 4 to 10 kb. Genomic DNA (50 µg) was digested and the fragments between 4 and 10 kb were isolated from a preparative 0.6% agarose gel using the Qiagen QlAquick extraction kit and finally dissolved in 20 µl 10 mM Tris, pH 8.0. These fragments were ligated to *Bam*HI digested pZErO-2 (Invitrogen) and transformed into *Escherichia coli* DH10B, resulting in about 39,000 colonies. Twenty individual colonies were used to inoculate 10 ml 2xYT medium to check the diversity of the library and to determine the average insert size. 19 plasmids contained inserts of different sizes, whereas one colony had no insert (self-ligated vector 5%). The average insert size of the gDNA fragments in pZErO-2 was about 3.8 kb. All obtained transformants were collected from the plates and resuspended in liquid 2xYT medium with kanamycin and glycerol was added to a final concentration of 8% (v/v) and stored at -80°C.

### Screening the Amycolatopsis orientalis gene library

The *Amycolatopsis orientalis* gene library was plated on 2xYT agar + kanamycin (50 mg/L) and incubated for 72 h at rT. Almost 12,000 colonies were used to inoculate 120 96-wells microtiterplates (MTPs), containing 0.2 ml 2xYT medium + 35 mg/L kanamycin. The MTPs were incubated at 25°C with 500 rpm for 48 h. From each well 140 µl cell suspension was centrifuged for 10 min at 3,000 rpm and the supernatant was discarded by tapping the plates on tissue paper (the remainder of the culture was added to 50 µl 20% glycerol and stored at -80°C). Per well 250 µl of substrate solution (2xYT medium with hydrolyzed compactin, 200 mg/L; glucose, 2 g/l; phosphate buffer, 50 mM; pH 6,8). Cell pellets in the wells were resuspended and incubated for 48 h at 30°C, 280 rpm. The statins were extracted after addition of 0.35 ml methanol per well and one hour mixing at 280 rpm. Cell debris was removed by centrifugation for 15 minutes at 2750 rpm. Samples of 100 µl were analyzed by LC-MS.

### LC-MS analysis of the Amycolatopsis orientalis gene library

The compactin standard was prepared by hydrolysis of mevastatin (A.G. Scientific, lot no. A7413, purity 99.36%) with 1.5 M NaOH in MeOH (1:2) during the night under stirring in helium atmosphere 2. The pH is reduced by addition of HCl (4 M) and the standard is further diluted with water. The samples are analyzed on a Waters LC/MS system with a short (20 mm) CN column of ACT (Advanced Chromatography Technologies) with water and acetonitrile (both with 0.1% formic acid) as mobile phases. The details of the LC-part are:

| | | | | | |
|---|---|---|---|---|---|
| Apparatus: | Waters Alliance 2795 LC | | | | |
| Mobile phases: | Solvent A: | | Water with 0.1% formic acid | | |
| | Solvent B: | | Acetonitrile with 0.1% formic acid | | |
| Needle wash: | 50% MilliQ water + 50% acetonitrile | | | | |

| Gradient timetable:Time | (min) | A% | B% | flow (ml/min) | curve |
|---|---|---|---|---|---|
| | 0.00 | 80.0 | 20.0 | 1.00 | 1 |
| | 0.35 | 80.0 | 20.0 | 1.00 | 6 |
| | 1.00 | 20.0 | 80.0 | 1.00 | 6 |
| | 1.40 | 20.0 | 80.0 | 1.00 | 6 |
| | 1.50 | 80.0 | 20.0 | 1.00 | 6 |
| | 2.00 | end | | | |
| Column: | ACT, ACE 3 CN, 20 x 2.1 mm, particle size 3 µm | | | | |
| Column temp.: | 25°C | | | | |
| Injection volume: | 5 µl | | | | |

In the MS electrospray ionization in the positive mode (ES+) is used and the compounds are analyzed as the sodium adducts ([M + Na]+) with selected ion (SIR) of the three compounds. The details of the MS-part are:

| | | | |
|---|---|---|---|
| Apparatus: | Waters ZQ 2000 | Source: | ES+ |
| Capillary: | 3.50 kV | Cone: | 30 V |
| Desolation temp.: | 360°C | Source temp.: | 140°C |
| Extractor: | 2 V | RF lens: | 0.3 V |
| Cone Gas flow: | 130 l/hour | Desolvation flow: | 610 l/hour |
| LM 1 Resolution: | 15.0 | HM 2 Resolution: | 15.0 |
| Ion Energy 1: | 0.1 | Multiplier: | 650 V |

Scan mass range (m/z) 200 - 600 amu, scan duration 0.20 s, interscan delay 0.05 s; SIR of 3 channels: 413.30, 431.3, 447.3, dwell 0.07 s, interscan delay 0.05 s.

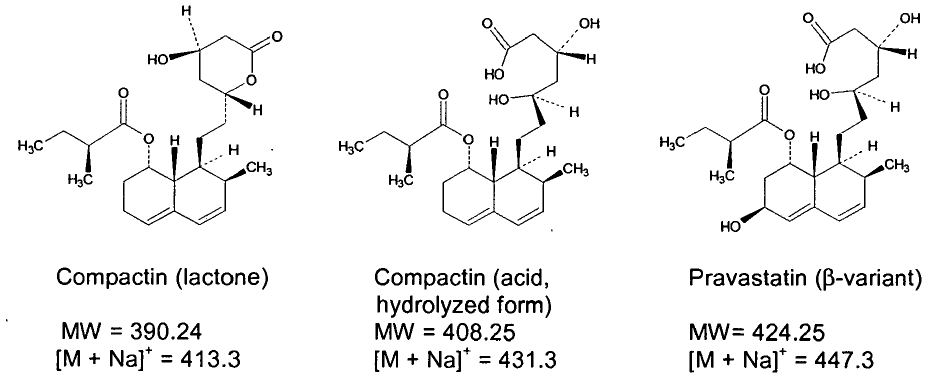

With this set-up it is possible to distinguish the four most important molecules, compactin, hydrolyzed compactin and the two stereoisomers of 6-hydroxy-compactin, i.e. the β-variant pravastatin (structure see above) and the α-variant epi-pravastatin.

**Table 4 Example of results from MTP screening for compactin hydroxylase**

| Nr. | Well position | Area pravastatin | Area compactin | Nr. | well position | Area pravastatin | Area compactin |
|---|---|---|---|---|---|---|---|
| 1 | B,1 | 18076 | 3664332 | 12 | B,23 | <10000 | 3710229 |
| 2 | B,3 | <10000 | 3409885 | 13 | D,1 | <10000 | 3615275 |
| 3 | B,5 | 10338 | 3176999 | 14 | D,3 | <10000 | 3396888 |
| 4 | B,7 | <10000 | 3421702 | 15 | D,5 | <10000 | 3487926 |
| 5 | B,9 | <10000 | 3719904 | 16 | D,7 | <10000 | 3443088 |
| 6 | B,11 | <10000 | 3202990 | 17 | D,9 | <10000 | 3483672 |
| 7 | B,13 | <10000 | 3237775 | 18 | D,11 | <10000 | 3683947 |
| 8 | B,15 | <10000 | 3473592 | 19 | D,13 | <10000 | 3565824 |
| 9 | B,17 | <10000 | 3638848 | 20 | D,15 | <10000 | 3610494 |
| 10 | B,19 | <10000 | 3694601 | 21 | D,17 | <10000 | 3719262 |
| 11 | B,21 | <10000 | 3606196 | 22 | D,19 | <10000 | 3856010 |

Several clones were identified as candidates as they showed a small, but significant peak at the position of pravastatin. In Table 4 as example a subset of the results is shown in which one clone gives a signal above background (clone in well position B1).

### Example 5

### Identification of gene encoding compactin to pravastatin biocatalyst

### Retesting the putative compactin hydroxylases from Amycolatopsis orientalis

The analysis of Example 4 was repeated with four clones which were identified in the first round as putative clones. However, now the clones were grown in shake flasks in stead of MTPs and some cultivation conditions were varied. The clones were precultivated in 10 ml 2xYT which was inoculated with the *Escherichia coli* cells containing putative compactin hydroxylases and grown for 24 hours at 30°C, 280 rpm. Subsequently, 0.1-0.5 mM IPTG and 0.5 mM δ-aminolevulinate was added and the cultures were incubated at 22°C, 280 rpm for 12h. Cells were harvested, washed and resuspended by vortexing in fresh 2xYT medium (supplemented with hydrolyzed compactin, 200 mg/L; glucose, 2 g/l; phosphate buffer, 50 mM; pH 6,8). Cell suspensions were incubated either at 30 or 37°C, for 24 or 48 hours, at 280 rpm. The statins were extracted and analyzed as described in Example 4.

**Table 5 Results of retesting putative compactin hydroxylases from A. orientalis.**

| Clone | Condition | Pravastatin (peak area) |
|---|---|---|
| 11H9 | 24 hr, 37 C | 39964580 |
| 12H9 | 24 hr, 37 C | 1914055 |
| 15H2 | 24 hr, 37 C | 1951158 |
| 16H2 | 24 hr, 37 C | 2120422 |
| control | | 1894831 |
| 11H9 | 48 hr, 30 C | 26749341 |
| 12H9 | 48 hr, 30 C | 2542003 |
| 15H2 | 48 hr, 30 C | 2388226 |
| 16H2 | 48 hr, 30 C | 1565684 |
| 11H9 | 48 hr, 37 C | 48316813 |
| 12H9 | 48 hr, 37 C | 2613137 |
| 15H2 | 48 hr, 37 C | 2408859 |
| 16H2 | 48 hr, 37 C | 2934999 |

As can be seen in Table 5 only clone 11 H9 has a real significant conversion of compactin to pravastatin. This one was therefore selected for further analyses.

### Sequencing and sequence analysis

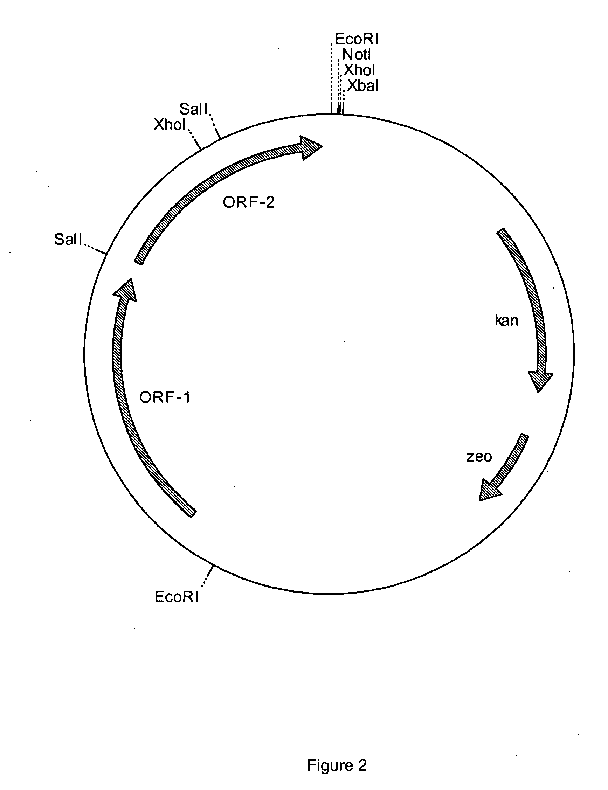

*Escherichia coli* clone 11H9 was cultivated in 2xYT with kanamycin and plasmid DNA was isolated using the Qiagen QIAprepep kit and the sequence of the *Amycolatopsis orientalis* genome insert in the pZERO-2 plasmid was determined. The sequence of the insert is 2545 nucleotides long (see SEQ ID NO. 1). Analyses of the DNA sequence were performed and two Open Reading Frames (ORFs) could be identified (Figure 2). The first ORF encodes a putative protein of 401 amino acids (SEQ ID no. 2 and 3) which has some homology to known p450 enzymes (i.e. the best being Cytochrome p450 monooxygenase CYP105S2 from *Streptomyces tubercidicus*). The second ORF has some membrane spanning regions and might encode an ATP-type Binding Cassette (ABC) protein.

### Identification of the structural gene, cmpH

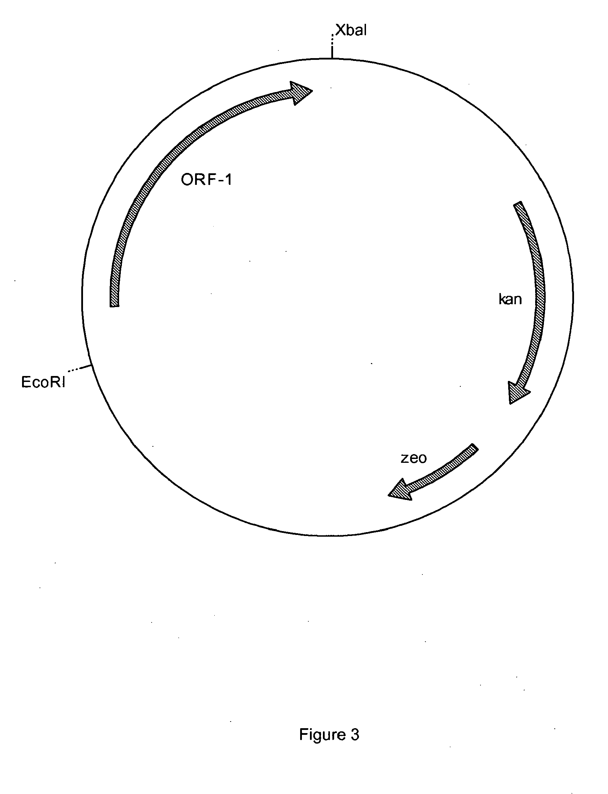

To identify the structural gene capable of hydroxylating compactin, ORF-2 was deleted from pZERO-Ao-11H9 via double digestion with *Sal*I and *Xho*I. Subsequently, the 4.9 kb fragment was isolated and self-ligated. The resulting plasmid pZERO-Ao-11 H9 contains only ORF-1 as a full ORF (Figure 3). This clone has the same conversion rate as clone pZERO-Ao-11H9, indicating that ORF-1 encodes a functional compactin hydroxylase, named *cmpH.*

### Comparative Example 6

### Activity of Streptomyces carbophilus compactin hydroxylase in Escherichia coli

### Construction of p450-SCA E. coli expression clone

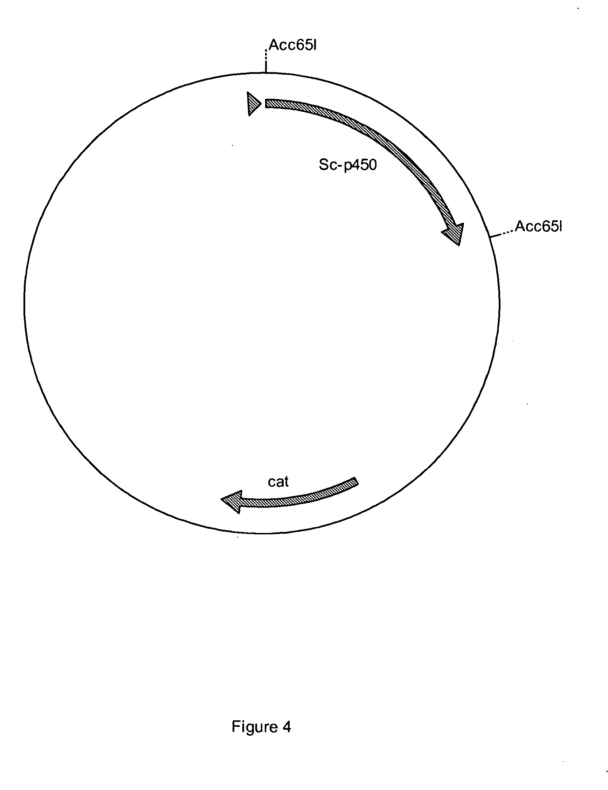

The gene encoding the *Streptomyces carbophilus* p450 was PCR amplified from genomic DNA isolated from strain FERM-BP1145 using the primers of SEQ ID NO. 7 and SEQ ID NO. 8. The PCR fragment was clone in the pCR2.1TOPO/TA vector according to the supplier's instructions (Invitrogen). The expression clone was constructed by digesting pACYC-taq (Krämer, M., 2000. Untersuchungen zum Einfluss erhöhter Bereitstellung von Erythrose-4-Phosphat und Phosphoenolpyruvat auf den Kohlenstofffluss in den Aromatenbiosyntheseweg von Escherichia coli. Berichte des Forschungszentrums Jülich 3824, ISSN 0944-2952, PhD Thesis, University of Düsseldorf) with Acc651 and ligating the *Acc*65I fragment isolated from the pCR2.1TOPO/TA vector, resulting in pACYC-taqScp450 (Figure 4).

### Activity determination in Escherichia coli extracts

*Escherichia coli* cells containing pACYC-taqScp450 were cultivated in 10 ml 2xYT with chloramphenicol. Cell suspensions were basically processed and incubated with compactin as described in example 5. In this case cultivation temperature was 37 degrees Celsius and chloramphenicol and IPTG (0.1 mM) were added to the reaction mixture. The reactions were incubated at 30°C and 220 rpm. Samples were taken at various time points and analyzed using the HPLC protocol as described in Example 1. After 24 h no pravastatin could be determined.

### Example 7

### Activity of Amycolatopsis orientalis compactin hydroxylase in Escherichia coli

### Construction of Ao-cmpH Escherichia coli expression clone

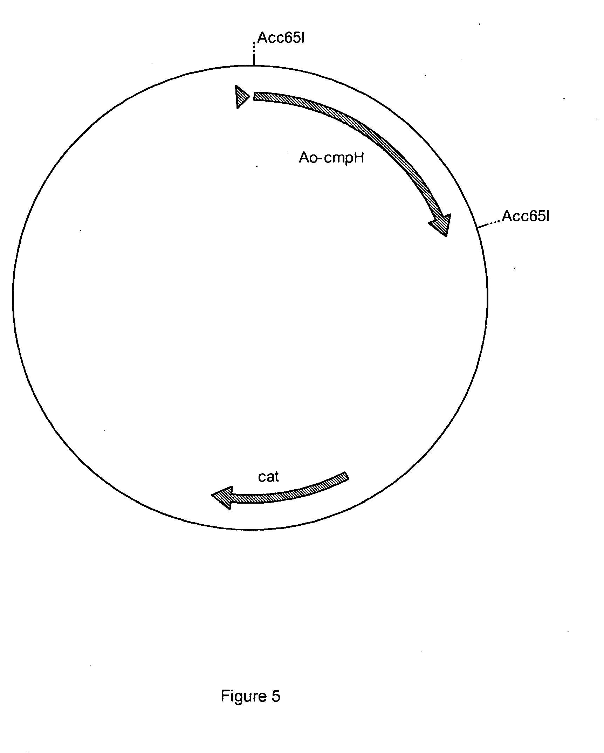

The gene encoding the *Amycolatopsis orientalis* p450 was PCR amplified from genomic DNA isolated using the primers of SEQ ID NO 9 and SEQ ID NO 10. The PCR fragment was cloned in the pCR2.1TOPO/TA vector according to the supplier's instructions (Invitrogen). The expression clone was constructed by digesting pACYC-taq (Krämer, 2000) with *Acc*65I and ligating the *Acc*65I fragment isolated from the pCR2.1TOPO/TA vector, resulting in pACYC-taqAop450 (Figure 5).

### Activity determination in Escherichia coli extracts

*Escherichia coli* cells containing pACYC-taqAop450 (Figure 5) were cultivated in 2xYT (10 ml) with chloramphenicol. Cell suspensions were incubated with compactin as described in Example 5. Cultivation temperature was 37°C and chloramphenicol and IPTG (0.1 mM) were added. The reactions were incubated at 30°C and 220 rpm. Samples were taken at various time points and analyzed using the HPLC protocol as described in Example 1. After 24 h a large peak of pravastatin was detected. This result clearly demonstrates that the *Amycolatopsis orientalis* p450 enzyme is much better suitable for hydroxylating compactin in *Escherichia coli* than other p450's.

**Table 6 Compactin conversion of Escherichia coli strains harboring the P450 genes. The data summarize average conversion ratios of 50 tested clones, each giving a compactin to pravastatin conversion of at least 90%. Percentages refer to converted compactin.**

| **Strain tested** | **Pravastatin β-variant Ratio in %** | **Pravastatin α-variant Ratio in %** |
|---|---|---|
| E. coli Top10 (Invitrogen) pACYC-taqAop450 | 5-10% | 90-95% |

### Example 8

### Activity of the derivatives of the Amycolatopsis orientalis compactin hydroxylase in Escherichia coli

The genes SEQ ID NO 11-18, 27-34 were produced synthetically and used as template for PCR reactions with oligonucleotides SEQ ID NO 9 and 10 with added attB1 (for SEQ ID NO 9) and attB2 (for SEQ ID NO 10) recombination sites. The PCR fragments were cloned in the pDONR221 vector (Invitrogen Corporation, The Netherlands) by performing the Gateway BP reaction (Invitrogen Corporation); the sequences were verified by DNA sequencing to exclude PCR related errors. Using the Gateway LR reaction, the genes were transferred from the pDONR221 vector to the pET-DEST42 vector, resulting in the final expression vectors pET-DEST42-P450. *Escherichia coli* BL21 DE3 harboring pET-DEST42-P450 (SEQ ID NO 11-18, 27-34 as inserts) was cultivated in 10 ml 2xYT with kanamycin at 30°C until OD₆₀₀=0.5-1.0 Subsequently the culture was supplemented with 0.1-0.3 mM IPTG and 0.5 mM δ-aminolevulinate and incubated at 22°C at 280 rpm for 12h. Cells were harvested, washed and resuspended in fresh 2xYT medium (supplemented with hydrolyzed compactin, 200 mg/L; glucose, 2 g/l; phosphate buffer, 50 mM; pH 6.8). Cell suspensions were incubated either at 30 or 37°C, for 24 or 48 h, at 280 rpm. The statins were extracted and analyzed as described in Example 4. After 24 h, a very large pravastatin peak could be identified. In contrast to the experiment described in example 7, the majority of produced pravastatin is the β-variant, giving proof for the significantly changed stereospecificity of the enzymes of SEQ ID NO 19-26 and SEQ ID NO 35-42, respectively, if compared to the compactin hydroxylase enzyme encoded by SEQ ID NO 3 or SEQ ID NO 6.

**Table 7 Compactin conversion of Escherichia coli strains harboring the P450 genes. The data summarize average conversion ratios of 50 tested clones each giving a compactin to pravastatin conversion of at least 90%. All DNA fragments encoded by the SEQ ID NO 11-18, 27-34 catalyze very similar compactin conversion features as shown below. Percentages refer to converted compactin.**

| **Strain tested** | **Pravastatin β-variant Ratio in %** | **Pravastatin α-variant Ratio in %** |
|---|---|---|
| E. coli BL21 DE3 (Invitrogen) pET-DEST42-P450 (SEQ ID NO 11-18, 27-34) | 90-95% | 5-10% |

## Claims

1. A polypeptide suitable for hydroxylation of compactin selected from the group consisting of a polypeptide having an amino acid sequence according to SEQ ID NO 3, SEQ ID NO 6, SEQ ID NO 19-26, SEQ ID NO 35-59 and a polypeptide having an amino acid sequence that has a degree of identity to SEQ ID NO 3, SEQ ID NO 6, SEQ ID NO 19-26 or SEQ ID NO 35-59 of at least 90%.

2. An isolated polynucleotide comprising a DNA sequence encoding the polypeptide of claim 1.

3. The polynucleotide of claim 2 that is SEQ ID NO 1, 2, 4 or 5.

4. A method for producing pravastatin comprising the steps of:
(i) transforming and expressing a polynucleotide of any of claims 2 to 3 in a production host;
(ii) growing the production host obtained in step (i);
(iii) isolating pravastatin from the mixture obtained in step (ii)
wherein compactin is added during growth of said production host.

5. Method according to claim 4 wherein said production host is a fungal cell or a bacterial cell.

6. Method according to claim 5 wherein said fungal cell is yeast or a filamentous fungal cell.

7. Method according to claim 4 wherein said production host is *Aspergillus niger, Bacillus amyloliquefaciens, Bacillus licheniformis, Bacillus subtilis, Corynebacterium glutamicum, Escherichia coli, Penicillium chrysogenum, Penicillium citrinum, Saccharomyces cerevisiae, Streptomyces avermitilis, Streptomyces clavuligerus* or *Streptomyces lividans.*

## Patentansprüche

1. Polypeptid mit Eignung zur Hydroxylierung von Compactin, ausgewählt aus der Gruppe bestehend aus einem Polypeptid mit einer Aminosäuresequenz gemäß SEQ ID NO 3, SEQ ID NO 6, SEQ ID NO 19-26, SEQ ID NO 35-59 und einem Polypeptid mit einer Aminosäuresequenz, die einen Identitätsgrad von wenigstens 90% zu SEQ ID NO 3, SEQ ID NO 6, SEQ ID NO 19-26 oder SEQ ID NO 35-59 aufweist.

2. Isoliertes Polynukleotid, umfassend eine für das Polypeptid nach Anspruch 1 codierende DNA-Sequenz.

3. Polynukleotid nach Anspruch 2, bei dem es sich um SEQ ID NO 1, 2, 4 oder 5 handelt.

4. Verfahren zur Herstellung von Pravastatin, mit den folgenden Schritten:
(i) Transformieren und Exprimieren eines Polynukleotids nach einem der Ansprüche 2 bis 3 in einem Produktionswirt;
(ii) Kultivieren des in Schritt (i) erhaltenen Produktionswirts;
(iii) Isolieren von Pravastatin aus dem in Schritt (ii) erhaltenen Gemisch,
wobei während der Kultivierung des Produktionswirts Compactin zugegeben wird.

5. Verfahren gemäß Anspruch 4, wobei es sich bei dem Produktionswirt um eine Pilzzelle oder eine Bakterienzelle handelt.

6. Verfahren gemäß Anspruch 5, wobei es sich bei der Pilzzelle um Hefe oder eine Fadenpilzzelle handelt.

7. Verfahren gemäß Anspruch 4, wobei es sich bei dem Produktionswirt um *Aspergillus niger, Bacillus amyloliquefaciens, Bacillus licheniformis, Bacillus* subtilis, *Corynebacterium glutamicum, Escherichia coli*, *Penicillium chrysogenum, Penicillium citrinum, Saccharomyces cerevisiae, Streptomyces avermitilis*, *Streptomyces clavuligerus* oder *Streptomyces lividans* handelt.

## Revendications

1. Polypeptide approprié pour hydroxyler la compactine, choisi dans le groupe constitué par un polypeptide ayant une séquence d'acides aminés selon l'identification de séquence n° 3, l'identification de séquence n° 6, les identifications de séquences n° 19 à 26, les identifications de séquences n° 35 à 59, et un polypeptide ayant une séquence d'acides aminés qui a un degré d'identité d'au moins 90% avec l'identification de séquence n° 3, l'identification de séquence n° 6, les identifications de séquences n° 19 à 26 ou les identifications de séquences n° 35 à 59.

2. Polynucléotide isolé comprenant une séquence d'ADN qui code pour le polypeptide selon la revendication 1.

3. Polynucléotide, selon la revendication 2, qui est l'identification de séquence n° 1, 2, 4 ou 5.

4. Procédé de production de pravastatine comprenant les étapes consistant à :
(i) transformer et exprimer un polynucléotide, selon l'une quelconque des revendications 2 à 3, dans un hôte de production ;
(ii) cultiver l'hôte de production obtenu à l'étape (i) ;
(iii) isoler de la pravastatine à partir du mélange obtenu à l'étape (ii)
dans lequel de la compactine est ajoutée lors de la culture dudit hôte de production.

5. Procédé selon la revendication 4, dans lequel ledit hôte de production est une cellule fongique ou une cellule bactérienne.

6. Procédé selon la revendication 5, dans lequel ladite cellule fongique est une levure ou une cellule de champignon filamenteux.

7. Procédé selon la revendication 4, dans lequel ledit hôte de production est *Aspergillus niger*, *Bacillus amyloliguefaciens, Bacillus licheniformis, Bacillus subtilis, Corynebacterium glutamicum, Escherichia coli, Penicillium chrysogenum, Penicillium citrinum, Saccharomyces cerevisiae, Streptomyces avermitilis, Streptomyces clavuligerus* ou *Streptomyces lividans.*
